(19) Europäisches Patentamt
European Patent Office
Office européen des brevets

(11) Veröffentlichungsnummer: **0 548 664 B1**

# (12) EUROPÄISCHE PATENTSCHRIFT

(45) Veröffentlichungstag der Patentschrift: **31.05.95**

(51) Int. Cl.⁶: **C07D 209/60**, A61K 31/40

(21) Anmeldenummer: **92120925.0**

(22) Anmeldetag: **09.12.92**

## (54) 2,3,3a,4,5,9b-Hexahydro-1H-benzo[e]indol-Derivate.

(30) Priorität: **20.12.91 CH 3806/91**
**19.10.92 CH 3254/92**

(43) Veröffentlichungstag der Anmeldung:
**30.06.93 Patentblatt 93/26**

(45) Bekanntmachung des Hinweises auf die Patenterteilung:
**31.05.95 Patentblatt 95/22**

(84) Benannte Vertragsstaaten:
**AT BE CH DE DK ES FR GB GR IE IT LI LU MC NL PT SE**

(56) Entgegenhaltungen:
**EP-A- 0 041 488**
**EP-A- 0 127 597**
**WO-A-90/15047**
**WO-A-91/00856**
**DE-A- 2 044 172**
**DE-A- 2 235 667**

**CHIM. THER. Bd. 7, Nr. 6, 1972, Seiten 450 - 458 M. LANGLOIS ET AL. 'Recherches dans la série des aryl-3 pyrrolidines III. Préparation de dérivés du benzo(e)indole et de l'indano(1,2b)pyrrole'**

**J. PHARM. PHARMACOL. Bd. 43, 1991, Seiten 11 - 16 I. DEN DAAS ET AL. 'Orally Active Carbamate Prodrugs of the Selective Dopamine Agonist N-0437: In-vivo Activities in the 6-OHDA Turning Model and In-vitro Activities'**

(73) Patentinhaber: **F. HOFFMANN-LA ROCHE AG**
**Postfach 3255**
**CH-4002 Basel (CH)**

(72) Erfinder: **Bruderer, Hans**
**4 Schulgasse**
**CH-4105 Biel-Benken (CH)**
Erfinder: **Godel, Thierry**
**1 Lenweg**
**CH-4153 Reinach (CH)**
Erfinder: **Imhof, René**
**3 Bleumatthöhe**
**CH-5264 Gipf-Oberfrick (CH)**
Erfinder: **Jakob-Roetne, Roland**
**26 Oberer Baselblick**
**W-7854 Inzlingen (DE)**

(74) Vertreter: **Bertschinger, Christoph, Dr. et al**
**Grenzacherstrasse 124**
**P.O. Box 3255**
**CH-4002 Basel (CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 548 664 B1

## Beschreibung

Die vorliegende Erfindung betrifft Indolderivate. Im speziellen betrifft sie cis-2,3,3a,4,5,9b-Hexahydro-1H-benzo[e]indol-Derivate der allgemeinen Formel

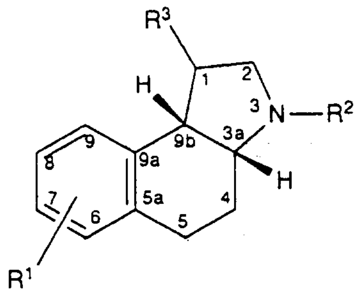

I

worin $R^1$ einen Rest der Formel $-O-CO-NR^4R^5$, $R^2$ niederes Alkyl, niederes Cycloalkyl oder durch niederes Cycloalkyl, Aryl, Aroyl, Aroylamino, Amino oder durch eine cyclische Amino-, Amid- oder Imidgruppe substituiertes niederes Alkyl, $R^3$ Wasserstoff oder niederes Alkyl, $R^4$ niederes Alkyl und $R^5$ Wasserstoff oder niederes Alkyl bedeuten,
entsprechende trans-Isomere oder cis-trans-Isomerengemische und pharmazeutisch anwendbare Säureadditionssalze davon.

Diese Verbindungen und Salze sind neu und zeichnen sich durch wertvolle therapeutische Eigenschaften aus. Sie eignen sich insbesondere zur Behandlung oder Prophylaxe kognitiver Störungen und seniler Demenz (speziell Alzheimer-Krankheit) und zur Verbesserung der Gedächtnisleistung.

WO-A-9100856 beschreibt Hexahydro-1H-benzo[e]indole und Octahydrobenzo[f]chinoline mit einer Hydroxy- oder Alkoxygruppe am aromatischen Ring, welche u.a. zur Behandlung von seniler Demenz geeignet sind. EP-A- 0 127 597 beschreibt Octahydrobenzo[f]chinoline, welche am Benzolring eine Carbamoyloxygruppe aufweisen können und welche eine Wirkung auf das zentrale Nervensystem entfalten.
J. Pharm. Pharmacol. 43 (1991) 11 beschreibt Carbamat-Prodrugs des selektiven Dopamin-Agonisten 5-Hydroxy-2-(N-n-propyl-N-2-ehtylthienylamino)tetralin. DE-A-20 44 172 und Chem. Ther. (1972) 450 beschreiben Hexahydro-1H-benzo[e]indole und entsprechende Indano(b)pyrrole mit einer Hydroxy- oder Alkoxygruppe am aromatischen Ring, welche als Analgetica, Entzündungshemmer und z.T. auch als Sedativa verwendet werden können.

Gegenstand der vorliegenden Erfindung sind die Verbindungen der Formel I sowie entsprechende trans-Isomere oder cis-trans-Isomerengemische und pharmazeutisch anwendbare Salze davon als solche und als therapeutische Wirkstoffe, ihre Herstellung, diese enthaltende Arzneimittel und deren Herstellung sowie ihre Verwendung zur Behandlung oder Prophylaxe kognitiver Störungen und seniler Demenz (speziell Alzheimer-Krankheit) und zur Verbesserung der Gedächtnisleistung bzw. zur Herstellung von Arzneimitteln für die genannten Indikationen.

Der Ausdruck "niederes Alkyl" umfasst geradkettige oder verzweigte gesättigte Kohlenwasserstoffreste mit bis zu 7 Kohlenstoffatomen, wie Methyl, Ethyl, n-Propyl, Isopropyl, n-Butyl, sek.Butyl, Isobutyl, n-Pentyl, n-Hexyl u.dgl.

Der Ausdruck "niederes Cycloalkyl" umfasst cyclische gesättigte Kohlenwasserstoffreste mit bis zu 7 Kohlenstoffatomen, wie Cyclopentyl, Cyclohexyl u.dgl.

Die Ausdrücke "Aryl" und "Aroyl" umfassen gegebenenfalls substituierte Phenyl- bzw. Benzoylreste, wobei als Substituenten in erster Linie niedere Alkoxygruppen (wie Methoxy), niedere Alkylsulfonylgruppen (wie Methansulfonyl), Halogene (wie Fluor) u.dgl. in Frage kommen und wobei 1 bis 3 derartige Substituenten vorhanden sein können; Beispiele für das Substitutionsmuster sind 4-Methoxy-, 3,4-Dimethoxy-, 4-Fluor-, 4-Methansulfonyl- u.dgl.

Die Ausdrücke "cyclische Aminogruppe", "cyclische Amidgruppe" und "cyclische Imidgruppe" umfassen über das Stickstoffatom gebundene 5- oder 6-gliedrige heterocyclische Reste, welche ein zusätzliches Heteroatom enthalten und/oder substituiert und/oder annelliert sein können. Als zusätzliches Heteroatom kommen dabei insbesondere Sauerstoff und als Substituenten insbesondere eine oder zwei niedere Alkylgruppen, wie Methyl, in Frage; annellierte Reste enthalten zusätzlich zum heterocyclischen Rest insbesondere einen Benzolring, welcher hydriert und/oder substituiert sein kann, insbesondere durch Halogen (vorzugsweise ein oder zwei Chloratome), Nitro, Hydroxy oder auch Pyridyl.

Beispiele für cyclische Amino-, Amid- und Imidgruppen sind Piperidin-1-yl, Morpholin-4-yl, 1-Oxoisoindolin-2-yl, 1,3-Dioxoisoindolin-2-yl, 1,3-Dioxo-octahydro-cis-isoindolin-2-yl, 4,4-Dimethyl-2,6-dioxopiperidin-1-yl u.dgl.

Falls in den Verbindungen der Formel I bzw. in den entsprechenden trans-Isomeren $R^3$ Wasserstoff bedeutet und weder $R^1$ noch $R^2$ ein Asymmetriezentrum aufweist, können die erfindungsgemässen Verbindungen als Enantiomere vorliegen, andernfalls sind verschiedene diastereomere Enantiomerenpaare möglich. Die Erfindung umfasst sämtliche möglichen Stereoisomeren sowie auch Gemische davon, insbesondere cis-trans-Gemische und/oder Racemate.

$R^1$ sitzt beispielsweise in 6- oder 9-Stellung des tricyclischen Ringgerüsts. In dem durch die Formel -O-CO-$NR^4R^5$ dargestellten Rest $R^1$ können beispielsweise $R^4$ Methyl, Ethyl, Propyl oder Butyl und $R^5$ Wasserstoff oder $R^4$ Methyl und $R^5$ Ethyl oder $R^4$ und $R^5$ beide Methyl bedeuten.

$R^2$ kann beispielsweise n-Propyl, 3-Methylbutyl, Cyclopentyl, Cyclohexylmethyl, 2-Cyclohexylethyl, 3-Cyclohexylpropyl, 4-Cyclohexylbutyl, 2-Cyclopentylethyl, Benzyl, 2-Phenylethyl, 3-Phenylpropyl, 3-Benzoylpropyl, 4-Benzoylbutyl, 3-(4-Methoxybenzoyl)propyl, 4-(3,4-Dimethoxybenzoyl)butyl, 5-(3,4-Dimethoxybenzoyl)pentyl, 3-(4-Fluorbenzoyl)propyl, 4-Benzoylaminobutyl, 5-Benzoylaminopentyl, 6-Benzoylaminohexyl, 4-(4-Methansulfonylbenzoylamino)butyl, 5-(4-Methansulfonylbenzoylamino)pentyl, 6-(4-Methansulfonylbenzoylamino)hexyl, 4-Aminobutyl, 5-Aminopentyl, 6-Aminohexyl, 2-Morpholin-4-ylethyl, 3-Morpholin-4-ylpropyl, 2-Piperidin-1-ylethyl, 3-Piperidin-1-ylpropyl, 4-(1,3-Dioxoisoindolin-1-yl)butyl, 5-(1,3-Dioxoisoindolin-1-yl)pentyl, 6-(1,3-Dioxoisoindolin-1-yl)hexyl, 5-(1,3-Dioxo-octahydro-cis-isoindolin-2-yl)pentyl, 4-(4,4-Dimethyl-2,6-dioxopiperidin-1-yl)butyl, 5-(4,4-Dimethyl-2,6-dioxopiperidin-1-yl)pentyl, 6-(4,4-Dimethyl-2,6-dioxopiperidin-1-yl)-hexyl, 4-(1-Oxoisoindolin-2-yl)butyl, 5-(1-Oxoisoindolin-2-yl)pentyl oder 6-(1-Oxoisoindolin-2-yl)pentyl bedeuten.

$R^3$ kann beispielsweise Wasserstoff oder Methyl bedeuten.

Bevorzugte Verbindungen der Formel I sind:

(+)-cis-3-(2-Cyclohexylethyl)-2,3,3a,4,5,9b-hexahydro-1H-benzo[e]indol-6-yl dimethylcarbamat,
(-)-cis-3-(2-Cyclohexylethyl)-2,3,3a,4,5,9b-hexahydro-1H-benzo[e]indol-6-yl dimethylcarbamat,
rac-cis-3-(2-Cyclohexylethyl)-2,3,3a,4,5,9b-hexahydro-1H-benzo[e]indol-6-yl dimethylcarbamat,
rac-cis-2,3,3a,4,5,9b-Hexahydro-3-(4-phthalimidobutyl)-1H-benzo[e]indol-6-yl dimethylcarbamat,
rac-cis-2,3,3a,4,5,9b-Hexahydro-3-(6-phthalimidohexyl)-1H-benzo[e]indol-6-yl dimethylcarbamat,
rac-cis-2,3,3a,4,5,9b-Hexahydro-3-(2-morpholinoethyl)-1H-benzo[e]indol-6-yl dimethylcarbamat,
rac-2,3,3a$\alpha$,4,5,9b$\alpha$-Hexahydro-1$\alpha$-methyl-3-propyl-1H-benzo[e]indol-6-yl dimethylcarbamat ,
rac-cis-2,3,3a,4,5,9b-Hexahydro-3-propyl-1H-benzo[e]indol-9-yl dimethylcarbamat,
(+)-3-(2-Cyclohexylethyl)-cis-2,3,3a,4,5,9b-hexahydro-1H-benzo[e]indol-6-yl-methylcarbamat,
(+)-3-(2-Cyclohexylethyl)-cis-2,3,3a,4,5,9b-hexahydro-1H-benzo[e]indol-6-yl-propylcarbamat,
(+)-3-(2-Cyclohexylethyl)-cis-2,3,3a,4,5,9b-hexahydro-1H-benzo[e]indol-6-yl-ethylmethylcarbamat,
rac-3-Cyclohexylmethyl-cis-2,3,3a,4,5,9b-hexahydro-1H-benzo[e]indol-6-yl-dimethylcarbamat,
rac-3-(3-Cyclohexylpropyl)-cis-2,3,3a,4,5,9b-hexahydro-1H-benzo[e]indol-6-yl-dimethylcarbamat,
rac-3-(4-Cyclohexylbutyl)-cis-2,3,3a,4,5,9b-hexahydro-1H-benzo[e]indol-6-yl-dimethylcarbamat und
rac-3-(2-Cyclopentylethyl)-cis-2,3,3a,4,5,9b-hexahydro-1H-benzo[e]indol-6-yl-dimethylcarbamat.

Weitere Beispiele für Verbindungen der Formel I sind:

(+)-3-(2-Cyclohexylethyl)-cis-2,3,3a,4,5,9b-hexahydro-1H-benzo[e]indol-6-yl-ethylcarbamat,
rac-3-(2-Cyclohexylethyl)-cis-2,3,3a,4,5,9b-hexahydro-1H-benzo[e]indol-6-yl-butylcarbamat,
rac-3-(3-Methylbutyl)-cis-2,3,3a,4,5,9b-hexahydro-1H-benzo[e]indol-6-yl-dimethylcarbamat,
rac-3-Phenethyl-cis-2,3,3a,4,5,9b-hexahydro-1H-benzo[e]indol-6-yl-dimethylcarbamat,
rac-3-Propyl-cis-2,3,3a,4,5,9b-hexahydro-1H-benzo[e]indol-6-yl-dimethylcarbamat,
rac-3-(3-Phenylpropyl)-cis-2,3,3a,4,5,9b-hexahydro-1H-benzo[e]indol-6-yl-dimethylcarbamat,
rac-3-(3-Morpholin-4-yl-propyl)-cis-2,3,3a,4,5,9b-hexahydro-1H-benzo[e]indol-6-yl-dimethylcarbamat,
rac-3-(2-Piperidin-1-yl-ethyl)-cis-2,3,3a,4,5,9b-hexahydro-1H-benzo[e]indol-6-yl-dimethylcarbamat,
rac-3-(3-Piperidin-1-yl-propyl)-cis-2,3,3a,4,5,9b-hexahydro-1H-benzo[e]indol-6-yl-dimethylcarbamat,
rac-3-[5-(1,3-Dioxo-isoindolin-2-yl)-pentyl]-cis-2,3,3a,4,5,9b-hexahydro-1H-benzo[e]indol-6-yl-dimethylcarbamat,
rac-3-[4-(1-Oxo-isoindolin-2-yl)-butyl]-cis-2,3,3a,4,5,9b-hexahydro-1H-benzo[e]indol-6-yl-dimethylcarbamat,
rac-3-[5-(1-Oxo-isoindolin-2-yl)-pentyl]-cis-2,3,3a,4,5,9b-hexahydro-1H-benzo[e]indol-6-yl-dimethylcarbamat,
rac-3-[6-(1-Oxo-isoindolin-2-yl)-hexyl]-cis-2,3,3a,4,5,9b-hexahydro-1H-benzo[e]indol-6-yl-dimethylcarbamat,
rac-3-(3-Benzoyl-propyl)-cis-2,3,3a,4,5,9b-hexahydro-1H-benzo[e]indol-6-yl-dimethylcarbamat,

rac-3-(4-Benzoyl-butyl)-cis-2,3,3a,4,5,9b-hexahydro-1H-benzo[e]indol-6-yl-dimethylcarbamat,
rac-3-[3-(4-Methoxy-benzoyl)-propyl]-cis-2,3,3a,4,5,9b-hexahydro-1H-benzo[e]indol-6-yl-dimethylcarbamat,
rac-3-[4-(3,4-Dimethoxy-benzoyl)-butyl]-cis-2,3,3a,4,5,9b-hexahydro-1H-benzo[e]indol-6-yl-dimethylcarbamat,
rac-3-[5-(3,4-Dimethoxy-benzoyl)-pentyl]-cis-2,3,3a,4,5,9b-hexahydro-1H-benzo[e]indol-6-yl-dimethylcarbamat,
rac-3-[4-(4,4-Dimethyl-2,6-dioxo-piperidin-1-yl)-butyl]-cis-2,3,3a,4,5,9b-hexahydro-1H-benzo[e]indol-6-yl-dimethylcarbamat,
rac-3-[5-(4,4-Dimethyl-2,6-dioxo-piperidin-1-yl)-pentyl]-cis-2,3,3a,4,5,9b-hexahydro-1H-benzo[e]indol-6-yl-dimethylcarbamat,
rac-3-[6-(4,4-Dimethyl-2,6-dioxo-piperidin-1-yl)-hexyl]-cis-2,3,3a,4,5,9b-hexahydro-1H-benzo[e]indol-6-yl-dimethylcarbamat,
rac-3-[3-(4-Fluorbenzoyl)-propyl]-cis-2,3,3a,4,5,9b-hexahydro-1H-benzo[e]indol-6-yl-dimethylcarbamat,
rac-N-[4-(6-Dimethylcarbamoyloxy)-cis-2,3,3a,4,5,9b-hexahydro-1H-benzo[e]indol-3-yl)-butyl]-4-methansulfonyl-benzamid,
rac-N-[5-(6-Dimethylcarbamoyloxy)-cis-2,3,3a,4,5,9b-hexahydro-1H-benzo[e]indol-3-yl)-pentyl]-4-methansulfonyl-benzamid,
rac-N-[6-(6-Dimethylcarbamoyloxy)-cis-2,3,3a,4,5,9b-hexahydro-1H-benzo[e]indol-3-yl)-hexyl]-4-methansulfonyl-benzamid,
rac-N-[4-(6-Dimethylcarbamoyloxy)-cis-2,3,3a,4,5,9b-hexahydro-1H-benzo[e]indol-3-yl)-butyl]-benzamid,
rac-N-[5-(6-Dimethylcarbamoyloxy)-cis-2,3,3a,4,5,9b-hexahydro-1H-benzo[e]indol-3-yl)-pentyl]-benzamid,
rac-N-[6-(6-Dimethylcarbamoyloxy)-cis-2,3,3a,4,5,9b-hexahydro-1H-benzo[e]indol-3-yl)-hexyl]-benzamid,
rac-cis-(3-Cyclopentylpropyl)-2,3,3a,4,5,9b-hexahydro-1H-benzo[e]indol-6-yl-dimethylcarbamat,
rac-cis-3-Cyclopentyl-2,3,3a,4,5,9b-hexahydro-1H-benzo[e]indol-6-yl-dimethylcarbamat,
rac-3-(4-Aminobutyl)-2,3,3a,4,5,9b-hexahydro-1H-cis-benzo[e]indol-6-yl-dimethylcarbamat,
rac-3-(5-Aminopentyl)-2,3,3a,4,5,9b-hexahydro-1H-cis-benzo[e]indol-6-yl-dimethylcarbamat,
rac-3-(6-Aminohexyl)-2,3,3a,4,5,9b-hexahydro-1H-cis-benzo[e]indol-6-yl-dimethylcarbamat,
rac-3-Benzyl-2,3,3a,4,5,9b-hexahydro-1H-cis-benzo[e]indol-6-yl-dimethylcarbamat,
rac-3-[5-(1,3-Dioxo-octahydro-cis-isoindol-2-yl)pentyl]-2,3,3a,4,5,9b-hexahydro-1H-cis-benzo[e]indol-6-yl-dimethylcarbamat,
rac-3-[5-(1,3-Dioxoisoindolin-2-yl)pentyl]-2,3,3a,4,5,9b-hexahydro-1H-cis-benzo[e]indol-6-yl-ethylcarbamat, und
(+)-trans-3-(2-Cyclohexylethyl)-2,3,3a,4,5,9b-hexahydro-1H-benzo[e]indol-6-yl-dimethylcarbamat.

Die Verbindungen der Formel I oder die entsprechenden trans-Isomeren oder cis-trans-Isomerengemische und ihre pharmazeutisch annehmbaren Säureadditionssalze können erfindungsgemäss dadurch hergestellt werden, dass man

a) eine Verbindung der allgemeinen Formel

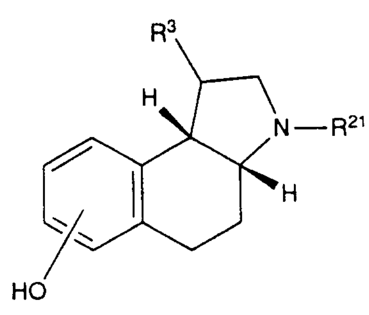

II

worin $R^3$ die oben angegebene Bedeutung besitzt und $R^{21}$ im Prinzip die oben für $R^2$ angegebene Bedeutung besitzt aber nicht durch Amino substituiertes niederes Alkyl bedeuten kann,
oder das entsprechende trans-Isomere oder cis-trans-Isomerengemisch mit einem einen Rest der Formel $-CO-NR^4R^5$ liefernden Mittel acyliert, wobei $R^4$ und $R^5$ die oben angegebene Bedeutung besitzen,
oder

b) eine Verbindung der allgemeinen Formel

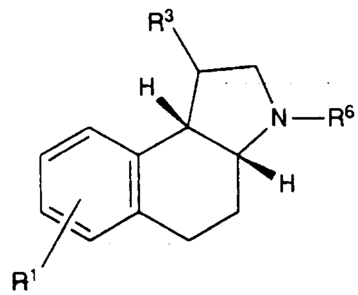

III

worin $R^1$ und $R^3$ die oben angegebene Bedeutung besitzen und $R^6$ durch einen in eine Aminogruppe überführbaren Rest substituiertes niederes Alkyl bedeutet,
oder das entsprechende trans-Isomere oder cis-trans-Isomerengemisch in eine entsprechende Verbindung der Formel I oder das entsprechende trans-Isomere oder cis-trans-Isomerengemisch überführt, worin $R^2$ durch Amino substituiertes niederes Alkyl bedeutet; oder

c) in einer Verbindung der Formel I, worin $R^2$ durch Amino substituiertes niederes Alkyl bedeutet, oder in einem entsprechenden trans-Isomeren oder cis-trans-Isomerengemisch die Aminogruppe entsprechend acyliert;

d) erwünschtenfalls ein erhaltenes cis-trans-Isomerengemisch und/oder Diastereomerengemisch auftrennt; und/oder

e) erwünschtenfalls ein erhaltenes Racemat aufspaltet; und/oder

f) erwünschtenfalls eine erhaltene Verbindung der Formel I oder ein entsprechendes trans-Isomeres oder cis-trans-Isomerengemisch in ein pharmazeutisch anwendbares Säureadditionssalz überführt.

Zur Acylierung der phenolischen Hydroxylgruppe in den Verbindungen der Formel II oder in den entsprechenden trans-Isomeren oder cis-trans-Isomerengemischen verwendet man zweckmässigerweise entsprechende Carbamoylchloride, wie Dimethylcarbamoylchlorid, Ethylmethylcarbamoylchlorid o. dgl. bzw. entsprechende Isocyanate, wie Methylisocyanat, Ethylisocyanat, Propylisocyanat, Butylisocyanat o.dgl. Bei Verwendung derartiger Acylierungsmittel erfolgt die Acylierung zweckmässigerweise in Gegenwart eines unter den Reaktionsbedingungen inerten organischen Lösungsmittels, beispielsweise in einem aromatischen Kohlenwasserstoff, wie Toluol, in einem halogenisierten Kohlenwasserstoff, wie 1,1,2-Trichlorethan usw.

Weiterhin erfolgt die Acylierung zweckmässigerweise in Gegenwart von 4-Dimethylaminopyridin und von Base(n), wobei als Basen in erster Linie organische Basen, wie Trialkylamine, Pyridin usw, in Frage kommen. Reaktionstemperatur und Reaktionszeit richten sich nach den übrigen Reaktionsparametern; im allgemeinen dürfte etwa 1- bis 30-stündiges Kochen am Rückfluss angezeigt sein.

Ausser Carbamoylchloriden bzw. Isocyanaten eignen sich als Acylierungsmittel auch Mischungen aus Bis-(trichlormethyl)-carbonat und entsprechenden Mono- bzw. Dialkylaminen.

Der in Formel III durch das Symbol $R^6$ bezeichnete Substituent enthält einen Rest, welcher in eine Aminogruppe übergeführt werden kann. Als derartige Reste kommen in erster Linie geschützte Aminogruppen in Betracht, beispielsweise acylierte Aminogruppen, wie Phthalimido, o.dgl.

Methoden, durch welche Gruppen der vorstehend beschriebenen Art in eine Aminogruppe übergeführt werden können, sind jedem Fachmann geläufig. Weiterhin ist es auch jedem Fachmann klar, dass nur solche Gruppen in Frage kommen, welche einerseits durch Methoden in eine Aminogruppe übergeführt werden können, die andere Gruppierungen im Molekül nicht in Mitleidenschaft ziehen, und welche anderseits unter der bei der Synthese der Verbindungen der Formel III oder der entsprechenden trans-Isomeren oder cis-trans-Isomerengemischen verwendeten Reaktionsbedingungen unversehrt bleiben.

Die Phthalimidogruppe wird zweckmässigerweise durch Umsetzen mit Hydrazin in Ethanol in eine Aminogruppe übergeführt.

Die Acylierung einer im Substituenten $R^2$ vorhandenen Aminogruppe erfolgt nach an sich bekannten und jedem Fachmann geläufigen Methoden, beispielsweise durch Umsetzung mit einem reaktionsfähigen funktionellen Derivat der dem einzuführenden Acylrest entsprechenden Carbonsäure, wie Benzoylchlorid, in Gegenwart einer Base, wie Triethylamin o.dgl., oder mit der entsprechenden freien Carbonsäure, wie 4-(Methylsulfonyl)benzoesäure, in Gegenwart eines geeigneten Kondensationsmittels, wie 2-(1H-Benzotriazol-1-yl)-1,1,3,3-tetramethyluronium-hexafluorophosphat o.dgl., und einer Base, wie Triethylamin o.dgl.

Die Auftrennung von cis-trans-Gemischen und von Diastereomerengemischen sowie die Aufspaltung von Racematen kann nach allgemein üblichen Methoden erfolgen, wobei es zweckmässig sein kann, dies

nicht erst bei Verbindungen der Formel I bzw. von entsprechenden trans-Isomeren oder cis-trans-Isomerengemischen durchzuführen, sondern bereits auf einer früheren Synthesestufe, z.B. auf der Stufe der Verbindungen der Formel II bzw. von entsprechenden trans-Isomeren oder cis-trans-Isomerengemischen.

Auch die Herstellung von pharmazeutisch anwendbaren Säureadditionssalzen von Verbindungen der Formel I bzw. von entsprechenden trans-Isomeren oder cis-trans-Isomerengemischen kann nach allgemein üblichen und jedem Fachmann geläufigen Methoden erfolgen. Es kommen sowohl Salze mit anorganischen als auch Salze mit organischen Säuren in Betracht, beispielsweise Hydrochloride, Hydrobromide, Sulfate, Methansulfonate, p-Toluolsulfonate, Oxalate, Tartrate, Citrate, Maleinate, Ascorbate, Acetate und dergleichen.

Die Herstellung der Ausgangsprodukte der Formel II bzw. von entsprechenden trans-Isomeren oder cis-trans-Isomerengemischen (welche einer aus WO 91/00856 im Prinzip bekannten Verbindungsklasse angehören) wird nachstehend anhand eines Reaktionsschemas generell erläutert; ausserdem enthalten zahlreiche der weiter hinten dargelegten Beispiele ausführliche Angaben über die Herstellung spezifischer Verbindungen der Formel II bzw. von entsprechenden trans-Isomeren oder cis-trans-Isomerengemischen. Dabei kommen durchwegs an sich bekannte und jedem Fachmann geläufigen Methoden zur Anwendung.

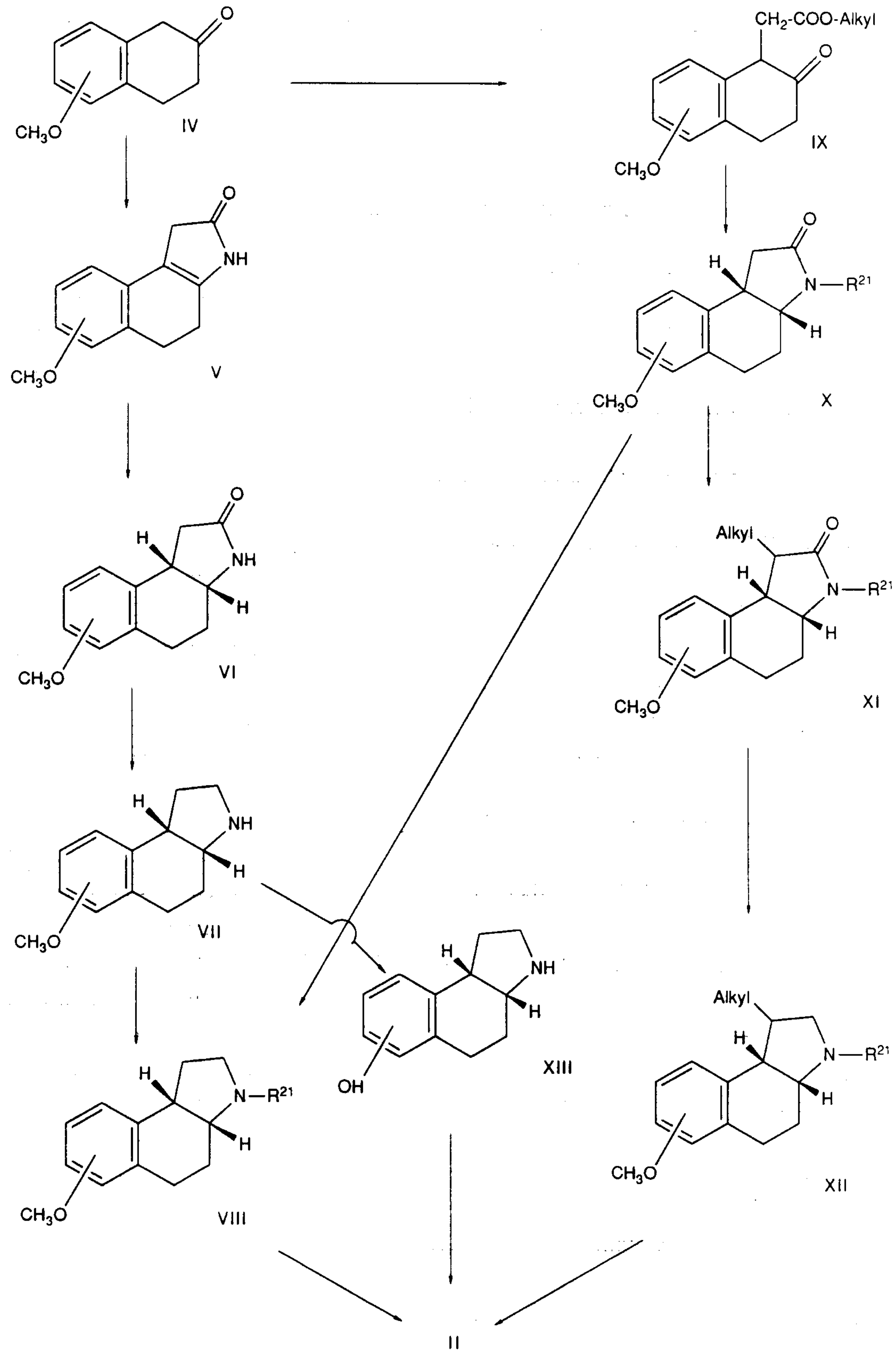

IV IX V X VI XI VII XIII VIII XII II

IV → V

Das Methoxytetralon der Formel IV wird zunächst mittels Pyrrolidin, Piperidin, Morpholin oder einem anderen sekundären Amin, wie Dimethylamin oder Diäthylamin in ein Enamin übergeführt, und dieses wird anschliessend mit Jodacetamid umgesetzt.

V → VI

Die Reduktion der Doppelbindung erfolgt zweckmässigerweise mittels Triethylsilan und Trifluoressigsäure in einem unter den Reaktionsbedingungen inerten organischen Lösungsmittel, beispielsweise in einem chlorierten Kohlenwasserstoff, wie Methylenchlorid; dabei kann neben der Verbindung der Formel VI auch das entsprechende trans-Isomere entstehen, allerdings nur in einem relativ geringfügigen Ausmass (ca. 5-10%). Die Reduktion der Doppelbindung kann aber auch durch katalytische Hydrierung erfolgen, z.B. in Gegenwart von Raney-Nickel; dabei entsteht ausschliesslich die cis-Verbindung der Formel VI.

VI → VII

Die Reduktion der Oxo- zur Methylengruppe erfolgt zweckmässigerweise mit einem komplexen Metallhydrid in einem unter den Reaktionsbedingungen inerten organischen Lösungsmittel, z.B. mit Lithiumaluminiumhydrid in Tetrahydrofuran.

VII → VIII

Die N-Substitution erfolgt zweckmässigerweise mit einem Chlorid oder Bromid der Formel $R^{21}$-Cl bzw. $R^{21}$-Br in Gegenwart einer Base, wie Kaliumcarbonat und eines Alkalimetalljodids, wie Natriumjodid, in einem unter den Reaktionsbedingungen inerten organischen Lösungsmittel, z.B. in einem Keton, wie Aceton, Methylethylketon o.dgl. Die Umsetzung kann beispielsweise auch in Toluol o.dgl. in Gegenwart einer organischen Base, wie Triethylamin o.dgl. erfolgen.

Die N-Substitution kann auch dadurch erfolgen, dass man das Stickstoffatom zunächst acyliert, z.B. mittels Cyclopentylessigsäurechlorid, Cyclopentylpropionsäurechlorid, Propionsäurechlorid o.dgl., und dann das Acylierungsprodukt mittels Lithiumaluminiumhydrid o.dgl. reduziert.

Enthält der eingeführte Substituent $R^{21}$ eine Phthalimidgruppe, so kann man diese spalten und anschliessend die freie Aminogruppe acylieren, analog wie oben im Zusammenhang mit Verfahrensvarianten b) und c) beschrieben. Man kann eine im eingeführten Rest $R^{21}$ vorhandene Phthalimidgruppe auch reduzieren. Reduktion mit Zink in Essigsäure liefert eine 1-Oxoisoindol-2-ylverbindung, und katalytische Hydrierung, z.B. über Pd/C in Eisessig, liefert ein Gemisch einer 1-Oxoisoindolin-2-ylverbindung und der entsprechenden 1,3-Dioxo-octahydro-cis-isoindol-2-ylverbindung.

Soll im Endprodukt $R^2$ einen durch Methoxy mono- oder disubstituierten Phenylrest enthalten, wie dies bei 4-(3,4-Dimethoxybenzoyl)butyl oder 5-(3,4-Dimethoxybenzoyl)pentyl der Fall ist, so erfolgt die entsprechende N-Substitution erst nach der Aetherspaltung, d.h. an Stelle der Sequenz VII→VIII→II tritt die Sequenz VII→XIII→II.

VII → XIII

Die Aetherspaltung erfolgt zweckmässigerweise mittels Bromwasserstoffsäure in Wasser oder mittels Bortribromid in Methylenchlorid oder mittels Pyridinhydrochlorid.

XIII → II

Die N-Substitution erfolgt zweckmässigerweise mit einem entsprechenden Bromid, wie 5-Brom-3',4'-dimethoxyvalerophenon, 6-Brom-(3,4-dimethoxyphenyl)hexan-1-on o.dgl., in Gegenwart einer Base, wie Triethylamin o.dgl., in einem inerten organischen Lösungsmittel, wie Toluol o.dgl.

IV → IX

Das Methoxytetralon der Formel IV wird zunächst - analog wie bei Stufe IV → V beschrieben - in ein Enamin übergeführt, und dieses wird dann mit einem Bromessigsäurealkylester umgesetzt.

IX → X

Die Verbindung der Formel X wird mit einem Amin der Formel $R^{21}$-$NH_2$ umgesetzt, worauf das erhaltene Produkt reduziert wird, zweckmässigerweise durch Hydrierung in Gegenwart von Raney-Nickel. Die Reduktion kann aber auch mittels Triethylsilan und Trifluoressigsäure erfolgen, wobei neben der Verbindung der Formel X in geringem Ausmass auch das entsprechende trans-Isomere entstehen kann.

X → VIII

Die Reduktion der Oxo- zur Methylengruppe erfolgt analog wie bei Stufe VI → VII beschrieben.

X → XI

Die Alkylierung in 1-Stellung erfolgt zweckmässigerweise mit einem entsprechenden Alkylhalogenid, wie Methyljodid, nach Deprotonieren mit einer starken Base, wie Lithium-Diisopropylamid.

XI → XII

Die Reduktion der Oxo- zur Methylengruppe erfolgt analog wie bei Stufe VI → VII beschrieben.

VIII bzw. XII → II

Die Aetherspaltung erfolgt zweckmässigerweise mit Bromwasserstoffsäure in Wasser oder mittels Bromtribromid in Methylenchlorid oder mit Pyridinhydrochlorid.

Die Herstellung der Verbindungen der Formel III oder der entsprechenden trans-Isomeren oder cis-trans-Isomerengemische erfolgt analog zu derjenigen der Verbindungen der Formel I oder der entsprechenden trans-Isomeren oder cis-trans-Isomerengemische, d.h. in Analogie zum vorstehenden Reaktionsschema und zur vorstehend beschriebenen Verfahrensvariante a).

Wie eingangs erwähnt besitzen die Verbindungen der Formel I sowie entsprechende trans-Isomere oder cis-trans-Isomerengemische und ihre pharmazeutisch anwendbaren Säureadditionssalze wertvolle pharmakodynamische Eigenschaften. Sie sind in der Lage, das Enzym Acetylcholin-Esterase zu hemmen und dadurch den Acetylcholinspiegel im Gehirn zu erhöhen. Sie eignen sich daher zur Behandlung oder Prophylaxe kognitiver Störungen und seniler Demenz (speziell Alzheimer-Krankheit) und zur Verbesserung der Gedächtnisleistung.

Die Bestimmung der Acetylcholin-Esterase-Hemmung erfolgte nach der Methode von Ellmann et al., Biochem. Pharmacol. 7 (1961) 88, und/oder nach der Methode von Johnson et al., Analytical Biochemistry 64 (1975), 229. Die nachfolgende Tabelle enthält für repräsentative Verbindungen der Formel I sowie für das Standardpräparat Physostigmin Angaben über die IC 50-Werte für die Acetylcholin-Esterase-Hemmung in nM/l sowie Angaben über ihre Toxizität (LLD, d.h. "lowest lethal dose" [niedrigste letale Dosis], in mg/kg, wobei auch Verabreichungsweg und Tierart angegeben sind).

| Verbindung | IC 50 | LLD |
|---|---|---|
| Physostigmin | 18 | 1 sc Ratte |
| A | 22 | 30 sc Ratte |
| B | 30 | 30 sc Ratte |
| C | 16 | 30 ip Ratte |
| D | 1,4 | 3 ip Ratte |
| E | 0,4 | 10 sc Ratte |
| F | 108 | 30 sc Ratte |
| G | 350 | 125 po Maus |
| H | 488 | 100 sc Ratte |

A = (+)-cis-3-(-Cyclohexylethyl)-2,3,3a,4,5,9b-hexahydro-1H-benzo[e]indol-6-yl dimethylcarbamat hydrochlorid

B = (-)-cis-3-(-Cyclohexylethyl)-2,3,3a,4,5,9b-hexahydro-1H-benzo[e]indol-6-yl dimethylcarbamat hydrochlorid

C = rac-cis-3-(-Cyclohexylethyl)-2,3,3a,4,5,9b-hexahydro-1H-benzo[e]indol-6-yl dimethylcarbamat hydrochlorid

D = rac-cis-2,3,3a,4,5,9b-Hexahydro-3-(4-phthalimidobutyl)-1H-benzo[e]indol-6-yl dimethylcarbamat hydrochlorid

E = rac-cis-2,3,3a,4,5,9b-Hexahydro-3-(6-phthalimidohexyl)-1H-benzo[e]indol-6-yl dimethylcarbamat hydrochlorid

F = rac-cis-2,3,3a,4,5,9b-Hexahydro-3-(2-morpholinoethyl)-1H-benzo[e]indol-6-yl dimethylcarbamat dihydrochlorid

G = rac-cis-2,3,3a,4,5,9b-Hexahydro-3-propyl-1H-benzo[e]indol-9-yl dimethylcarbamat hydrochlorid

H = 2,3,3aα,4,5,9bα-Hexahydro-1α-methyl-3-propyl-1H-benzo[e]indol-6-yl dimethylcarbamat hydrochlorid

Für weitere erfindungsgemässe Verbindungen sind die IC 50-Werte:
(+)-3-(2-Cyclohexylethyl)-cis-2,3,3a,4,5,9b-hexahydro-1H-benzo[e]indol-6-yl-methylcarbamat: 18;
(+)-3-(2-Cyclohexylethyl)-cis-2,3,3a,4,5,9b-hexahydro-1H-benzo[e]indol-6-yl-ethylcarbamat: 2460;
(+)-3-(2-Cyclohexylethyl)-cis-2,3,3a,4,5,9b-hexahydro-1H-benzo[e]indol-6-yl-propylcarbamat: 2,6;
rac-3-(2-Cyclohexylethyl)-cis-2,3,3a,4,5,9b-hexahydro-1H-benzo[e]indol-6-yl-butylcarbamat: 793;
(+)-3-(2-Cyclohexylethyl)-cis-2,3,3a,4,5,9b-hexahydro-1H-benzo[e]indol-6-yl-ethylmethylcarbamat: 3,5;
rac-3-Cyclohexylmethyl-cis-2,3,3a,4,5,9b-hexahydro-1H-benzo[e]indol-6-yl-dimethylcarbamat: 36;
rac-3-(3-Cyclohexylpropyl)-cis-2,3,3a,4,5,9b-hexahydro-1H-benzo[e]indol-6-yl-dimethylcarbamat: 19;
rac-3-(4-Cyclohexylbutyl)-cis-2,3,3a,4,5,9b-hexahydro-1H-benzo[e]indol-6-yl-dimethylcarbamat: 35;
rac-3-(2-Cyclopentylethyl)-cis-2,3,3a,4,5,9b-hexahydro-1H-benzo[e]indol-6-yl-dimethylcarbamat: 30;
rac-3-(3-Methylbutyl)-cis-2,3,3a,4,5,9b-hexahydro-1H-benzo[e]indol-6-yl-dimethylcarbamat: 67;
rac-3-Phenethyl-cis-2,3,3a,4,5,9b-hexahydro-1H-benzo[e]indol-6-yl-dimethylcarbamat: 47;
rac-3-Propyl-cis-2,3,3a,4,5,9b-hexahydro-1H-benzo[e]indol-6-yl-dimethylcarbamat: 40;
rac-3-(3-Phenylpropyl)-cis-2,3,3a,4,5,9b-hexahydro-1H-benzo[e]indol-6-yl-dimethylcarbamat: 36;
rac-3-(3-Morpholin-4-yl-propyl)-cis-2,3,3a,4,5,9b-hexahydro-1H-benzo[e]indol-6-yl-dimethylcarbamat: 63;
rac-3-(2-Piperidin-1-yl-ethyl)-cis-2,3,3a,4,5,9b-hexahydro-1H-benzo[e]indol-6-yl-dimethylcarbamat: 31;
rac-3-[5-(1,3-Dioxo-isoindolin-2-yl)-pentyl]-cis-2,3,3a,4,5,9b-hexahydro-1H-benzo[e]indol-6-yl-dimethylcarbamat: 2,7;
rac-3-[4-(1-Oxo-isoindolin-2-yl)-butyl]-cis-2,3,3a,4,5,9b-hexahydro-1H-benzo[e]indol-6-yl-dimethylcarbamat: 8,6;
rac-3-[5-(1-Oxo-isoindolin-2-yl)-pentyl]-cis-2,3,3a,4,5,9b-hexahydro-1H-benzo[e]indol-6-yl-dimethylcarbamat: 4,5;
rac-3-[6-(1-Oxo-isoindolin-2-yl)-hexyl]-cis-2,3,3a,4,5,9b-hexahydro-1H-benzo[e]indol-6-yl-dimethylcarbamat: 2,9;
rac-3-(3-Benzoyl-propyl)-cis-2,3,3a,4,5,9b-hexahydro-1H-benzo[e]indol-6-yl-dimethylcarbamat: 8,3;
rac-3-(4-Benzoyl-butyl)-cis-2,3,3a,4,5,9b-hexahydro-1H-benzo[e]indol-6-yl-dimethylcarbamat: 612;
rac-3-[4-(3,4-Dimethoxy-benzoyl)-butyl]-cis-2,3,3a,4,5,9b-hexahydro-1H-benzo[e]indol-6-yl-dimethylcarbamat: 1,4;

rac-3-[5-(3,4-Dimethoxy-benzoyl)-pentyl]-cis-2,3,3a,4,5,9b-hexahydro-1H-benzo[e]indol-6-yl-dimethylcarbamat: 1,2;

rac-3-[4-(4,4-Dimethyl-2,6-dioxo-piperidin-1-yl)-butyl]-cis-2,3,3a,4,5,9b-hexahydro-1H-benzo[e]indol-6-yl-dimethylcarbamat: 13;

rac-3-[5-(4,4-Dimethyl-2,6-dioxo-piperidin-1-yl)-pentyl]-cis-2,3,3a,4,5,9b-hexahydro-1H-benzo[e]indol-6-yl-dimethylcarbamat: 11;

rac-3-[6-(4,4-Dimethyl-2,6-dioxo-piperidin-1-yl)-hexyl]-cis-2,3,3a,4,5,9b-hexahydro-1H-benzo[e]indol-6-yl-dimethylcarbamat: 8;

rac-3-[3-(4-Fluor-benzoyl)-propyl]-cis-2,3,3a,4,5,9b-hexahydro-1H-benzo[e]indol-6-yl-dimethylcarbamat: 6,8;

rac-N-[4-(6-Dimethylcarbamoyloxy)-cis-2,3,3a,4,5,9b-hexahydro-1H-benzo[e]indol-3-yl)-butyl]-4-methansulfonyl-benzamid: 6,7;

rac-N-[5-(6-Dimethylcarbamoyloxy)-cis-2,3,3a,4,5,9b-hexahydro-1H-benzo[e]indol-3-yl)-pentyl]-4-methansulfonyl-benzamid: 5,1;

rac-N-[6-(6-Dimethylcarbamoyloxy)-cis-2,3,3a,4,5,9b-hexahydro-1H-benzo[e]indol-3-yl)-hexyl]-4-methansulfonyl-benzamid: 5,6;

rac-N-[4-(6-Dimethylcarbamoyloxy)-cis-2,3,3a,4,5,9b-hexahydro-1H-benzo[e]indol-3-yl)-butyl]-benzamid: 16;

rac-N-[5-(6-Dimethylcarbamoyloxy)-cis-2,3,3a,4,5,9b-hexahydro-1H-benzo[e]indol-3-yl)-pentyl]-benzamid: 15,2;

rac-N-[6-(6-Dimethylcarbamoyloxy)-cis-2,3,3a,4,5,9b-hexahydro-1H-benzo[e]indol-3-yl)-hexyl]-benzamid: 11;

rac-cis-(3-Cyclopentylpropyl)-2,3,3a,4,5,9b-hexahydro-1H-benzo[e]indol-6-yl-dimethylcarbamat: 14;

rac-cis-3-Cyclopentyl-2,3,3a,4,5,9b-hexahydro-1H-benzo[e]indol-6-yl-dimethylcarbamat: 95;

rac-3-(4-Aminobutyl)-2,3,3a,4,5,9b-hexahydro-1H-cis-benzo[e]indol-6-yl-dimethylcarbamat: 267;

rac-3-(5-Aminopentyl)-2,3,3a,4,5,9b-hexahydro-1H-cis-benzo[e]indol-6-yl-dimethylcarbamat: 111;

rac-3-(6-Aminohexyl)-2,3,3a,4,5,9b-hexahydro-1H-cis-benzo[e]indol-6-yl-dimethylcarbamat: 8;

rac-3-Benzyl-2,3,3a,4,5,9b-hexahydro-1H-cis-benzo[e]indol-6-yl-dimethylcarbamat: 49;

rac-3-[5-(1,3-Dioxo-octahydro-cis-isoindol-2-yl)pentyl]-2,3,3a,4,5,9b-hexahydro-1H-cis-benzo[e]indol-6-yl-dimethylcarbamat: 7,8; und

(+)-trans-3-(2-Cyclohexylethyl)-2,3,3a,4,5,9b-hexahydro-1H-benzo[e]indol-6-yl-dimethylcarbamat: 22.

Weiterhin besitzen die erfindungsgemässen Verbindungen Affinitäten für Sigma-Rezeptoren (bestimmt nach Weber et al., Proc. Natl. Acad. Sci. USA 83 (1986), 8784) und zum Teil auch für muskarinische Rezeptoren (bestimmt nach Watson et al., Pharmacol. Exp. Ther. 237 (1986), 419 und nach Eberlein et al., Trends Pharmacol. Science 10 (1989), 50). Aus dem nachfolgenden Bindungsprofil geht hervor, dass Verbindung A einerseits Affinität zu Sigma-Rezeptoren und anderseits Affinität praeferentiell für muskarinische Rezeptoren vom M2-Typ zeigt:

| Rezeptor | Radioligand | Prozentuale Hemmung bei Konzentration von | |
|---|---|---|---|
| | | $10^{-7}$ M | $10^{-5}$ M |
| Sigma | [3H]-DTG | 62,1 | 100,7 |
| M1 | [3H]-Pirenzepin | 2,2 | 87,9 |
| M2 | [3H]-AFDX 384 | 31,3 | 94,8 |

Dabei besitzt die Verbindung A am muskarinischen Rezeptor antagonistische Eigenschaften, da sie in mikromolaren Konzentrationen eine durch muskarinische Agonisten induzierte Kontraktion einer Präparation des Plexus Myentericus des Meerschweinchens antagonisiert (nach Kilbinger et al., Eur. J. Pharmacol. 103 (1984) 313).

Die erfindungsgemässen Verbindungen und deren pharmazeutisch anwendbare Säureadditionssalze können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Wie weiter vorne erwähnt, sind Arzneimittel, enthaltend eine erfinddungsgemässe Verbindung oder ein pharmazeutisch anwendbares Säureadditionssalz davon, ebenfalls Gegenstand der vorliegenden Erfindung, weiterhin auch ein Verfahren zur Herstellung derartiger Arzneimittel, welches dadurch gekennzeichnet ist,

dass man eine oder mehrere erfindungsgemässe Verbindungen und/oder pharmazeutisch anwendbare Säureadditionssalze davon und erwünschtenfalls einen oder mehrere andere therapeutisch wirksame Stoffe zusammen mit einem oder mehreren therapeutisch inerten Exzipientien in eine galenische Darreichungsform bringt.

Zur Herstellung von Tabletten, Lacktabletten, Dragées und Hartgelatinekapseln kann man als Excipientien z.B. Lactose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser, Polyole, Saccharose, Invertzucker, Glukose und dergleichen.

Für Injektionslösungen eignen sich als Excipientien z.B. Wasser, Alkohole, Polyole, Glyzerin, vegetabile Oele etc.

Für Suppositorien eignen sich als Excipientien z.B. natürliche oder gehärtete Oele, Wachse, Fette, halbflüssige oder flüssige Polyole und dergleichen.

Die pharmazeutischen Präparate können daneben noch Konservierungsmittel, Lösevermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man die erfindungsgemässen Verbindungen und pharmazeutisch anwendbare Säureadditionssalze davon zur Behandlung oder Prophylaxe kognitiver Störungen und seniler Demenz (speziell Alzheimer-Krankheit) und zur Verbesserung der Gedächtnisleistung verwenden. Die Dosierung kann innerhalb weiter Grenzen variieren und ist natürlich in jedem einzelnen Fall den individuellen Gegebenheiten anzupassen. Im Allgemeinen dürfte bei oraler Verabreichung eine Tagesdosis von etwa 1 bis 200 mg angemessen sein, wobei aber die soeben angegebene obere Grenze auch überschritten werden kann, wenn sich dies als angezeigt erweisen sollte.

Gegenstand der Erfindung ist schliesslich auch die Verwendung der erfindungsgemässen Verbindungen und von pharmazeutisch anwendbaren Säureadditionssalzen davon zur Herstellung von Arzneimitteln zur Behandlung oder Prophylaxe kognitiver Störungen und seniler Demenz (speziell Alzheimer-Krankheit) und zur Verbesserung der Gedächtnisleistung.

In den nachfolgenden Beispielen, welche die vorliegende Erfindung illustrieren, deren Umfang jedoch in keiner Weise einschränken sollen, sind sämtliche Temperaturen in Celsiusgraden angegeben.

Beispiel 1

a) 439 g (2.49 Mol) 5-Methoxy-2-tetralon und 227 ml (3.67 Mol) Pyrrolidin wurden in 3 l Toluol gelöst. Nach Zugabe von 16.4 g (0.09 Mol) p-Toluolsulfonsäuremonohydrat wurde das Gemisch 4 Stunden gekocht, wobei das entstandene Wasser mittels eines Wasserabscheiders kontinuierlich entfernt wurde. Hierauf wurde das Toluol im Vakuum abdestilliert. Der Rückstand wurde in 1 l Tetrahydrofuran gelöst, worauf bei Raumtemperatur tropfenweise eine Lösung von 500 g (2.70 Mol) Jodacetamid in 2.5 l Tetrahydrofuran zugegeben wurde. Das Gemisch wurde 30 Minuten am Rückfluss gekocht, dann auf Raumtemperatur abgekühlt und noch 12 Stunden nachgerührt. Der Festkörper wurde abfiltriert und mit Tetrahydrofuran gewaschen. Man erhielt 475 g (89%) 1,3,4,5-Tetrahydro-6-methoxy-2H-benzo[e]indol-2-on als weisse Kristalle mit Smp. 220-222°.

b) 313 g (1.45 Mol) 1,3,4,5-Tetrahydro-6-methoxy-2H-benzo[e]indol-2-on wurden in 3 l Methylenchlorid suspendiert. Nach Zugabe von 690 ml (4.33 Mol) Triethylsilan wurden bei 0° langsam 500 ml (6.53 Mol) Trifluoressigsäure zugetropft, worauf das Gemisch 3 Stunden bei Raumtemperatur gerührt, dann mit einem Eis-Ethanolbad gekühlt, mit ca. 830 ml 28-prozentiger Natronlauge alkalisch gestellt, auf Wasser geleert und mit Methylenchlorid extrahiert wurde. Die organische Phase wurde mit gesättigter Kochsalzlösung gewaschen und mit Natriumsulfat getrocknet. Nach Abdestillieren des Lösungsmittels im Vakuum wurde der Rückstand in eiskaltem Ether suspendiert. Der Festkörper wurde abfiltriert, mit Ether nachgewaschen und getrocknet. Man erhielt 210 g (67%) rac-cis-1,3,3a,4,5,9b-Hexahydro-6-methoxy-2H-benzo[e]indol-2-on als weisse Kristalle mit Smp. 192-194°.

c) Eine Suspension von 25 g (0.66 Mol) Lithiumaluminiumhydrid in 2 l Tetrahydrofuran wurde portionenweise mit 71.5 g (0.33 Mol) rac-cis-1,3,3a,4,5,9b-Hexahydro-6-methoxy-2H-benzo[e]indol-2-on versetzt. Das Gemisch wurde über Nacht am Rückfluss gekocht, auf ca. -10° abgekühlt und dann hydrolysiert, indem nacheinander 25 ml Wasser, 25 ml 15-prozentige Natronlauge und 75 ml Wasser zugetropft wurden. Der feste Rückstand wurde abfiltriert und mit Methylenchlorid nachgewaschen. Die organischen Phasen wurden eingeengt. Der Rückstand wurde in Methylenchlorid gelöst, und die Lösung wurde mit

Natriumsulfat getrocknet. Nach Abdestillieren des Lösungsmittels im Vakuum und Kristallisieren im Kühlschrank erhielt man 64.0 g (96%) rac-cis-2,3,3a,4,5,9b-Hexahydro-6-methoxy-1H-benzo[e]indol als graue Kristalle.

Eine kleine Probe wurde in Ethanol gelöst und mit ethanolischer HCl versetzt. Nach Einengen und Umkristallisieren aus Ethanol erhielt man rac-cis-2,3,3a,4,5,9b-Hexahydro-6-methoxy-1H-benzo[e]indol-hydrochlorid mit einem Smp. von 226-231°.

d) Eine Suspension von 24.1 g (0.12 Mol) rac-cis-2,3,3a,4,5,9b-Hexahydro-6-methoxy-1H-benzo[e]indol, 28.3 g (0.15 Mol) 2-Cyclohexylethylbromid, 26.3 g (0.19 Mol) Kaliumcarbonat und 6.22 g (0.04 Mol) Natriumiodid in 550 ml Ethylmethylketon wurde 3 Tage am Rückfluss gekocht. Der Festkörper wurde abfiltriert, und das Lösungsmittel wurde im Vakuum abdestilliert. Der Rückstand wurde in Essigester gelöst, worauf die Lösung mit Wasser und Kochsalzlösung gewaschen und eingeengt wurde. Nach Chromatographieren über Kieselgel (Essigester-Methylenchlorid 1:2) erhielt man 16.1 g (43%) rac-cis-3-(2-Cyclohexylethyl)-2,3,3a,4,5,9b-hexahydro-6-methoxy-1H-benzo[e]indol als beiges Oel.

MS: m/e (% Basispeak): 313 ($M^+$,11), 217(21), 216(100), 108(13), 44(13).

e) 16.1 g (0.05 Mol) rac-cis-3-(2-Cyclohexylethyl)-2,3,3a,4,5,9b-hexahydro-6-methoxy-1H-benzo[e]indol wurde mit 270 ml 48-prozentiger wässriger Bromwasserstoffsäure 3 Stunden bei 120° gekocht. Nach Abdestillieren der Bromwasserstoffsäure im Vakuum wurde der Rückstand in Methylenchlorid/Wasser gelöst. Nach Extraktion mit Methylenchlorid bei pH 8 wurde das Lösungsmittel im Vakuum abdestilliert. Nach Chromatographieren über Kieselgel (Methylenchlorid-Methanol 10:1) erhielt man 10.3 g (67%) rac-cis-3-(2-Cyclohexylethyl)-2,3,3a,4,5,9b-hexahydro-1H-benzo[e]indol-6-ol als beiges Oel.

Eine kleine Probe wurde in Ethanol gelöst und mit ethanolischer HCl versetzt. Nach Einengen und Umkristallisieren aus Ethanol erhielt man rac-cis-3-(2-Cyclohexylethyl)-2,3,3a,4,5,9b-hexahydro-1H-benzo[e]indol-6-ol-hydrochlorid mit Smp. 223-224°.

f) Eine Suspension von 2.00 g (0.006 Mol) rac-cis-3-(2-Cyclohexylethyl)-2,3,3a,4,5,9b-hexahydro-1H-benzo[e]indol-6-ol in 160 ml Toluol wurde mit 2 ml (0.014 Mol) Triethylamin, 0.88 g (0.007 Mol) 4-Dimethylaminopyridin und 0.7 ml (0.007 Mol) Dimethylcarbamoylchlorid versetzt und dann 4 Stunden unter Rückfluss gekocht. Nach Abkühlen wurde das Gemisch in eine gesättigte wässrige Natriumhydrogencarbonatlösung gegossen. Nach Extraktion mit Methylenchlorid und Chromatographie über Kieselgel mit Essigester/Hexan 1:2 wurden 2.40 g rac-cis-3-(2-Cyclohexylethyl)-2,3,3a,4,5,9b-hexahydro-1H-benzo-[e]indol-6-yl-dimethylcarbamat als Oel erhalten.

MS: m/e (% Basispeak): 370 ($M^+$,7), 274(18), 273(100), 72(60).

Das erhaltene Oel wurde in Ethanol gelöst und mit ethanolischer HCl versetzt. Nach Abdestillieren erhielt man 2.34 g (94%) rac-cis-3-(2-Cyclohexylethyl)-2,3,3a,4,5,9b-hexahydro-1H-benzo[e]indol-6yl-dimethylcarbamathydrochlorid als beigen Schaum.

## Beispiel 2

a) Eine Lösung von 6.98 g (0.023 Mol) rac-cis-3-(2-Cyclohexylethyl)2,3,3a,4,5,9b-hexahydro-1H-benzo[e]-indol-6-ol in 70 ml Methylenchlorid wurde mit einer Lösung aus 5.80 g (0.017 Mol) (-)-2,2'-(1,1'-Binaphthyl)phosphorsäure in 70 ml Ethanol und 70 ml Methylenchlorid vermischt. Nach Abdestillieren der Lösungsmittel im Vakuum wurde der Rückstand fünfmal aus Ethanol umkristallisiert und dann in wässriger Ammoniaklösung suspendiert. Extraktion mit Ether ergab 2.53 g (36%) (+)- cis-3-(2-Cyclohexylethyl)-2,3,3a,4,5,9b-hexahydro-1H-benzo[e]indol-6-ol als weisse Kristalle mit Smp. 130-133°, $[\alpha]_D^{20}$ = +65.9° (MeOH, C=1%).

Eine kleine Probe wurde in Ethanol gelöst und mit ethanolischer HCl versetzt. Nach Einengen und Umkristallisieren aus Ethanol erhielt man (+)- cis-3-(2-Cyclohexylethyl)-2,3,3a,4,5,9b-hexahydro-1H-benzo[e]indol-6-ol-hydrochlorid als weisse Kristalle mit Smp. 199-200°, $[\alpha]_D^{20}$ = +35.7 (MeOH, C=1%).

b) Eine Suspension von 2.53 g (0.008 Mol) (+)-cis-3-(2-Cyclohexylethyl)-2,3,3a,4,5,9b-hexahydro-1H-benzo[e]indol-6-ol in 100 ml Toluol wurde mit 1.4 ml (0.01 Mol) Triethylamin, 1.24 g (0.01 Mol) 4-Dimethylaminopyridin und 0.93 ml (0.01 Mol) Dimethylcarbamoylchlorid versetzt. Das Gemisch wurde 4 Stunden unter Rückfluss gekocht. Nach Abdestillieren des Lösungsmittels im Vakuum wurde der Rückstand in Methylenchlorid gelöst. Die Lösung wurde mit Wasser und gesättigter wässriger Kochsalzlösung extrahiert. Die Extrakte wurden mit Natriumsulfat getrocknet und eingeengt. Chromatographie über Kieselgel mit Essigester/n-Hexan/Methylenchlorid 1:1:1 ergab 2.49 g eines Oels, das in Ethanol gelöst und mit 2.3 ml 8.8M ethanolischer HCl versetzt wurde. Nach Einengen wurde der Rückstand in Methylenchlorid gelöst. Nach Filtration wurde das Lösungsmittel im Vakuum abdestilliert. Der Rückstand wurde in Essigester suspendiert. Nach Abfiltrieren und Trocknen bei 60° im Vakuum erhielt man 2.37 g (69%) (+)- cis-3-(2-Cyclohexylethyl)-2,3,3a,4,5,9b-hexahydro-1H-benzo[e]indol-6-yl-dimethylcarbamat-hydrochlorid als farblose Kristalle mit Smp. 207°.

Beispiel 3

a) Eine Lösung von 14.1 g (0.047 Mol) rac-cis-3-(2-Cyclohexylethyl)-2,3,3a,4,5,9b-hexahydro-1H-benzo-[e]indol-6-ol in 150 ml Methylenchlorid wurde mit einer Lösung aus 9.81 g (0.028 Mol) (+)-2,2'-(1,1'-Binaphthyl)phosphorsäure in 150 ml Ethanol und 150 ml Methylenchlorid vermischt. Nach Abdestillieren der Lösungsmittel im Vakuum wurde der Rückstand fünfmal aus Ethanol umkristallisiert und dann in wässriger Ammoniaklösung suspendiert. Extraktion mit Ether ergab 2.09 g (14%) (-)- cis-3-(2-Cyclohexylethyl)-2,3,3a,4,5,9b-hexahydro-1H-benzo[e]indol-6-ol als weisse Kristalle mit Smp. 130-133°, $[\alpha]_D^{20}$ = -63.4° (MeOH, C=1%).

Eine kleine Probe wurde in Ethanol gelöst und mit ethanolischer HCl versetzt. Nach Einengen der Lösung und Umkristallisieren des Rückstands aus Ethanol erhielt man (-)-cis-(2-Cyclohexylethyl)-2,3,3a,4,5,9b-hexahydro-1H-benzo[e]indol-6-ol-hydrochlorid als weisse Kristalle mit Smp. 199-200°C, $[\alpha]_D^{20}$ = -33.7° (MeOH, C=1%).

b) Eine Suspension von 1.78 g (0.006 Mol) (-)-cis-3-(2-Cyclohexylethyl)-2,3,3a,4,5,9b-hexahydro-1H-benzo[e]indol-6-ol in 100 ml Toluol wurde mit 1 ml (0.007 Mol) Triethylamin, 0.87 g (0.007 Mol) 4-Dimethylaminopyridin und 0.66 ml (0.007 Mol) Dimethylcarbamoylchlorid versetzt und 24 Stunden unter Rückfluss gekocht. Nach Abdestillieren des Lösungsmittels im Vakuum wurde der Rückstand in Methylenchlorid gelöst, worauf die Lösung mit Wasser und Kochsalzlösung extrahiert wurde. Die Extrakte wurden mit Natriumsulfat getrocknet und eingeengt. Chromatographie über Kieselgel mit Methylenchlorid/Methanol 20:1 ergab 1.12 g eines Oels, das in Ethanol gelöst und mit ethanolischer HCl versetzt wurde. Nach Einengen und Umkristallisieren aus Essigester erhielt man 0.70 g (29%) (-)-cis-3-(2-Cyclohexylethyl)-2,3,3a,4,5,9b-hexahydro-1H-benzo[e]indol-6-yl-dimethylcarbamat-hydrochlorid als farblose Kristalle mit Smp. 206°.

Beispiel 4

a) Eine Lösung von 1.00 g (0.005 Mol) rac- cis-2,3,3a,4,5,9b-Hexahydro-6-methoxy-1H-benzo[e]indol in 60 ml Ethylmethylketon wurde mit 0.86 g (0.006 Mol) Kaliumcarbonat, 0.29 g (0.002 Mol) Natriumiodid und 1.40 g (0.005 Mol) N-(4-Brombutyl)phthalimid versetzt. Das Gemisch wurde 24 Stunden unter Rückfluss gekocht und nach Abkühlen mit Wasser versetzt. Man extrahierte mit Essigester, trocknete die Extrakte mit Natriumsulfat und destillierte das Lösungsmittel im Vakuum ab. Chromatographie über Kieselgel mit Methylenchlorid/Methanol 20:1 ergab 2.10 g (quant.) rohes rac- cis-N-[4-(1,2,3a,4,5,9b-Hexahydro-6-methoxy-3H-benzo[e]indol-3-yl)butyl]phthalimid als hellgelbe Kristalle. Eine kleine Probe wurde aus Essigester/Ether umkristallisiert und zeigte dann einen Smp. von 103-105°.

b) Eine Lösung von 2.00 g (0.005 Mol) rac-cis-N-[4-(1,2,3a,4,5,9b-Hexahydro-6-methoxy-3H-benzo[e]-indol-3-yl)butyl]-phthalimid in 100 ml Methylenchlorid wurde tropfenweise mit einer Lösung von 2.4 ml (0.025 Mol) Bortribromid in 10 ml Methylenchlorid versetzt, worauf 1.5 Stunden bei Raumtemperatur gerührt wurde. Unter Kühlen mit einem Eisbad wurden 32 ml (0.065 Mol) 2N Natronlauge zugetropft. Das Gemisch wurde auf Wasser gegossen, worauf eine wässrige Natriumhydrogencarbonatlösung zugegeben wurde, bis ein pH von 8 erreicht war. Man extrahierte mit Methylenchlorid, trocknete die Extrakte über Natriumsulfat und destillierte das Lösungsmittel im Vakuum ab. Chromatographie über Kieselgel mit Methylenchlorid/Methanol 20:1 lieferte ein Oel, welches in Ethanol gelöst und mit ethanolischr HCl versetzt wurde. Abdestillieren des Lösungsmittels und Umkristallisieren aus Methanol ergab 0.3 g (14%) rac-N-[4-(cis-1,2,3a,4,5,9b-Hexahydro-6-hydroxy-3H-benzo[e]indol-3-yl)butyl]phthalimid-hydrochlorid als weisse Kristalle mit Smp. 258-260° (Zers.).

c) Ein Gemisch von 0.12 g (0.28 mMol) rac-cis-N-[4-(1,2,3a,4,5,9b-Hexahydro-6-hydroxy-3H-benzo[e]-indol-3-yl)butyl]phthalimid-hydrochlorid,0.03 ml (0.34 mMol) Dimethylcarbamoylchlorid, 0.05 ml (034 mMol) Triethylamin und 0.042 g (0.34 mMol) 4-Dimethylaminopyridin in 10 ml Toluol wurde 4 Stunden unter Rückfluss gekocht und dann auf Wasser gegossen. Man extrahierte mit Toluol, trocknete die Extrakte mit Natriumsulfat und destillierte das Lösungsmittel im Vakuum ab. Der Rückstand wurde über Kieselgel mit Methylenchlorid/Methanol (20:1) chromatographiert und dann in Ethanol gelöst. Die Lösung wurde mit ethanolischer HCl versetzt und eingeengt. Man löste den Rückstand in Methylenchlorid, gab Ether zu, destillierte die Lösungsmittel ab und erhielt 0.11 g (79%) rac-cis-2,3,3a,4,5,9b-Hexahydro-3-(4-phthalimidobutyl)-1H-benzo[e]indol-6-yl-dimethylcarbamat-hydrochlorid als Schaum.

MS: m/e (% Basispeak): 461 ($M^+$,8), 457(2), 274(19), 273(100), 216(4), 200(3), 160(10), 130(2), 72(57).

Beispiel 5

a) Eine Lösung von 1.00 g (0.005 Mol) rac-cis-2,3,3a,4,5,9b-Hexahydro-6-methoxy-1H-benzo[e]indol in 60 ml Ethylmethylketon wurde mit 0.85 g (0.006 Mol) Kaliumcarbonat, 0.28 g (0.002 Mol) Natriumiodid und 1.60 g (0.005 Mol) N-(6-Bromhexyl)phthalimid versetzt. Das Gemisch wurde 24 Stunden unter Rückfluss gekocht, abgekühlt, mit Wasser versetzt und mit Essigester extrahiert. Die Extrakte wurden mit Natriumsulfat getrocknet, worauf das Lösungsmittel im Vakuum abdestilliert wurde. Chromatographieren des Rückstandes über Kieselgel mit Methylenchlorid/Methanol (20:1) ergab 1.90 g eines Oels, das in Methylenchlorid gelöst wurde. Nach Zugabe von ethanolischer HCl und Abdestillieren der Lösungsmittel erhielt man 2.00 g (87%) rac-cis-N-[6-(1,2,3a,4,5,9b-Hexahydro-6-methoxy-3H-benzo[e]indol-3-yl)hexyl]-phthalimid-hydrochlorid.
MS: m/e (% Basispeak): 432 ($M^+$, 6), 217(15), 216(100), 185(4), 160(7).
b) Eine Lösung von 1.80 g (0.004 Mol) rac- cis-N-[6-(1,2,3a,4,5,9b-Hexahydro-6-methoxy-3H-benzo[e]-indol-3-yl)hexyl]phthalimid-hydrochlorid in 100 ml Methylenchlorid wurde tropfenweise mit einer Lösung von 1.8 ml (0.019 Mol) Bortribromid in 10 ml Methylenchlorid versetzt. Das Gemisch wurde 1.5 Stunden bei Raumtemperatur gerührt, anschliessend unter Kühlen mit einem Eisbad tropfenweise mit 25 ml (0.05 Mol) 2N Natronlauge versetzt und dann auf Wasser gegossen, worauf wässrige Natriumhydrogencarbonatlösung zugegeben wurde, bis ein pH von 8 erreicht war. Man extrahierte mit Chloroform, trocknete die Extrakte über Natriumsulfat und destillierte das Lösungsmittel im Vakuum ab. Der Rückstand wurde über Kieselgel mit Methylenchlorid/Methanol (20:1) chromatographiert. Das erhaltene Oel wurde in Ethanol gelöst und mit ethanolischer HCl versetzt. Abdestillieren des Lösungsmittels und Umkristallisieren aus Methanol ergab 0.93 g (53%) rac-cis-N-[6-(1,2,3a,4,5,9b-Hexahydro-6-hydroxy-3H-benzo[e]indol-3-yl)-hexyl]phthalimid-hydrochlorid als weisse Kristalle mit Smp. 218°.
c) Ein Gemisch von 0.30 g (0.66 mMol) rac-cis-N-[6-(1,2,3a,4,5,9b-Hexahydro-6-hydroxy-3H-benzo[e]-indol-3-yl)hexyl]phthalimid-hydrochlorid,0.06 ml (0.66 mMol) Dimethylcarbamoylchlorid, 0.19 ml (1.32 mMol) Triethylamin und 0.08 g (0.66 mMol) 4-Dimethylaminopyridin in 10 ml Toluol wurde 24 Stunden unter Rückfluss gekocht und dann in gesättigte wässrige Natriumhydrogencarbonatlösung gegossen. Man extrahierte mit Methylenchlorid, trocknete die Extrakte mit Natriumsulfat und destillierte das Lösungsmittel im Vakuum ab. Der Rückstand wurde über Kieselgel mit Methylenchlorid/Methanol (20:1) chromatographiert und in Methylenchlorid gelöst. Die Lösung wurde mit ethanolischer HCl versetzt und eingeengt. Man löste den Rückstand in Methylenchlorid, gab Ether zu und destillierte die Lösungsmittel ab. Es wurden 0.12 g (32%) rac-cis-2,3,3a, 4,5,9b-Hexahydro-3-(6-phthalimidohexyl)-1H-benzo[e]indol-6-yl-dimethylcarbamat-hydrochlorid als Schaum erhalten.
MS: m/e (% Basispeak): 489 ($M^+$,7), 396(2), 274(19), 273(100), 201(2), 160(8), 72(64).

Beispiel 6

a) 28 g (0.16 Mol) 5-Methoxy-2-tetralon und 12.5 g (14.5 ml, 0.175 Mol) Pyrrolidin wurden in 150 ml Toluol gelöst. Das Gemisch wurde 2 Stunden am Wasserabscheider gekocht, abgekühlt, mit 31.7 g (0.19 Mol) Bromessigsäureethylester versetzt und 2 Stunden unter Rückfluss gekocht. Das Gemisch wurde dann eingeengt, in 150 ml Ethanol/Wasser (5:1) gelöst und erneut 2 Stunden unter Rückfluss gekocht. Das Lösungsmittel wurde im Vakuum abdestilliert, und der Rückstand wurde in Essigester gelöst. Die Lösung wurde mit Wasser und gesättigter Kochsalzlösung gewaschen, mit $MgSO_4$ getrocknet, filtriert und eingeengt. Der Rückstand wurde in Ether gelöst, mit Aktivkohle behandelt und umkristallisiert. Man erhielt 18.4 g rac-1,2,3,4-Tetrahydro-5-methoxy-2-oxo-1-naphthylessigsäureethylester als weisse Kristalle. Aus den Mutterlaugen konnten durch Chromatographieren über Kieselgel mit Cyclohexan/Essigester (4:1) weitere 10.3 g weisse Kristalle erhalten werden. Gesamtausbeute: 28.7 g (68%) weisse Kristalle mit Smp. 36-40°.
b) Eine Lösung von 6.30 g (0.024 Mol) 1,2,3,4-Tetrahydro-5-methoxy-2-oxo-1-naphthylessigsäureethylester in 100 ml Toluol wurde mit 3.1 ml (0.024 Mol) 4-(2-Aminoethyl)morpholin versetzt, worauf das Gemisch 20 Stunden am Wasserabscheider gekocht wurde. Nach Einengen wurde mit 1 g Raney-Nickel in 100 ml Ethanol bei 120° und 180 Bar hydriert. Das Produkt wurde über Kieselgel mit Methylenchlorid/Methanol (9:1) chromatographiert. Man erhielt 6.80 g (86%) öliges rac-cis-1,3,3a, 4,5,9b-Hexahydro-6-methoxy-3-(2-morpholinoethyl)-2H-benzo[e]indol-2-on.
MS: m/e (% Basispeak): 330 ($M^+$,3), 299(1), 160(3), 149(4), 113(23), 100(100).
c) 1.0 g (27.2 mMol) Lithiumaluminiumhydrid wurde in 20 ml Tetrahydrofuran unter Argon suspendiert. Nach Zutropfen einer Lösung von 6.8 g (0.021 Mol) rac-cis-1,3,3a,4,5,9b-Hexahydro-6-methoxy-3-(2-morpholinoethyl)-2H-benzo[e]indol-2-on in 20 ml Tetrahydrofuran wurde das Gemisch 1 Stunde unter

Rückfluss gekocht und dann vorsichtig tropfenweise zunächst mit 5 ml Essigester und dann mit gesättigter wässriger $Na_2SO_4$-Lösung versetzt, bis ein völlig weisser Niederschlag entstanden war. Nach Filtrieren und Einengen des Filtrats wurde der Rückstand in 100 ml Ethanol gelöst. Die Lösung wurde tropfenweise mit 7 ml (0.041 Mol) 21-prozentiger ethanolischer HCl-Lösung versetzt und 4 Stunden bei Raumtemperatur gerührt, wobei Kristalle ausfielen. Umkristallisation dieser Kristalle aus Ethanol ergab 6.35 g (79%) rac-cis-2,3,3a, 4,5,9b-Hexahydro-6-methoxy-3-(2-morpholinoethyl)-1H-benzo[e]indol-hydrochlorid als weisse Kristalle mit Smp. 125-155° (Zers.).

d) 6.28 g (0.016 Mol) rac-cis-2,3,3a,4,5,9b-Hexahydro-6-methoxy-3-(2-morpholinoethyl)-1H-benzo[e]indol-hydrochlorid wurden in 0.5 l 48-prozentiger wässriger HBr gelöst, 4 Stunden unter Rückfluss gekocht und dann eingeengt, worauf der Rückstand in Wasser gelöst wurde. Die Lösung wurde neutralisiert, indem zuerst festes $Na_2CO_3$ und dann gesättigte wässrige $NaHCO_3$-Lösung zugegeben wurde. Man schüttelte dreimal mit $CH_2Cl_2$ aus, wusch die organische Phase mit gesättigten wässrigen $NaHCO_3$- und NaCl-Lösungen, trocknete sie mit $Na_2SO_4$, filtrierte, engte das Filtrat ein und chromtographierte den Rückstand über 0.27 kg Kieselgel mit Methylenchlorid/Methanol (4:1). Das Produkt (4.8 g) wurde in 150 ml Ethanol gelöst. Man tropfte 6 ml (0.035 Mol) 21-prozentige ethanolische HCl-Lösung zu und rührte 4 Stunden bei Raumtemperatur, wobei Kristalle ausfielen. Umkristallisieren aus Ethanol ergab 4.97 g (82%) rac-cis-2,3,3a,4,5,9b-Hexahydro-3-(2-morpholinoethyl)-1H-benzo[e]indol-6-ol-dihydrochlorid als weisse Kristalle mit Smp. 230-275° (Zers.).

e) 1.00 g (0.003 Mol) rac-cis-2,3,3a,4,5,9b-Hexahydro-3-(2-morpholinoethyl)1H-benzo[e]indol-6-ol-dihydrochlorid, gelöst in 50 ml Wasser, wurde mit 5.6 ml 1N Natronlauge (5.6 mMol, 2.1 Aeq.) neutralisiert. Man extrahierte mit Ether, wusch die organische Phase mit gesättigten wässrigen $NaHCO_3$- und NaCl-Lösungen, trocknete sie mit $Na_2SO_4$, filtrierte ab und engte das Filtrat ein. Der ölige Rückstand wurde in 50 ml Toluol gelöst. Man gab nacheinander 0.30 g (0.41 ml, 0.003 Mol) Triethylamin, 32.5 mg (266 µMol, 0.1 Aeq.) 4-Dimethylaminopyridin und 0.27 ml (0.003 Mol) Dimethylcarbamoylchlorid zu. Das Gemisch wurde 2 Stunden unter Rückfluss gekocht, auf Wasser gegossen und mit $CH_2Cl_2$ extrahiert. Man wusch die organische Phase mit gesättigten wässrigen $NaHCO_3$- und NaCl-Lösungen, trocknete sie mit $Na_2SO_4$, filtrierte ab, engte das Filtrat ein und chromatographierte den Rückstand auf 220 g Kieselgel mit Methylenchlorid/Methanol (9:1). Das erhaltene Oel (0.8 g) wurde in 10 ml Ethanol gelöst. Man tropfte 0.92 ml (0.0045 Mol) 4.89N ethanolische HCl-Lösung zu und fällte mit Essigester. Die Kristalle wurden abfiltriert, in heissem Ethanol gelöst und mit Aktivkohle behandelt. Nach Abfiltrieren, Einengen und Umkristallisieren aus Ethanol/Essigester erhielt man 0.57 g (48%) rac-cis-2,3,3a,4,5,9b-Hexahydro-3-(2-morpholinoethyl)-1H-benzo[e]indol-6-yl-dimethylcarbamat-dihydrochlorid als weisse Kristalle mit Smp. 170-220° (Zers.).

## Beispiel 7

a) Eine Lösung von 15.0 g (0.057 Mol) 1,2,3,4-Tetrahydro-5-methoxy-2-oxo-1-naphthylessigsäureethylester in 250 ml Toluol wurde mit 9.45 ml (0.114 Mol) Propylamin versetzt, worauf das Gemisch 17 Stunden am Wasserabscheider gekocht wurde. Nach Einengen und dreimaligem Umkristallisieren wurden 4.4 g weisse Kristalle erhalten. Chromatographie der Mutterlaugen über Kieselgel mit Ether/Hexan (2:1) und Umkristallisieren ergab 2.45 g zusätzliches Material. Gesamtausbeute: 6.85 g, Smp. 81-83°.

6.80 g (0.026 Mol) des erhaltenen Produkts wurden mit 1.5 g Raney-Nickel in 400 ml Ethanol bei 120° und 140 bar während 15 Stunden hydriert. Das Produkt wurde über Kieselgel mit Hexan/Ether (1:1) chromatographiert. Man erhielt 5.20 g öliges rac-cis-1,3,3a,4,5,9b-Hexahydro-6-methoxy-3-propyl-2H-benzo[e]indol-2-on.

b) 2.73 g (0.027 Mol) Diisopropylamin wurden in 150 ml trockenem Tetrahydrofuran unter Argon gelöst. Bei -78° wurden 16.7 ml 1.61M Butyllithium in Hexan (0.027 Mol) zugetropft, worauf das Gemisch 15 Minuten bei 0° gerührt, wieder auf -78° abgekühlt und tropfenweise mit einer Lösung von 6.40 g (0.025 Mol) rac-cis-1,3,3a,4,5,9b-Hexahydro-6-methoxy-3-propyl-2H-benzo[e]indol-2-on in 100 ml Tetrahydrofuran versetzt wurde. Man liess die Temperatur der gelben Lösung bis 0° steigen, kühlte wieder auf -78° ab, und tropfte dann eine Lösung von 3.83 g (0.027 Mol) Methyljodid in 30 ml Tetrahydrofuran zu. Man liess die Temperatur des Gemisches auf 0° steigen und rührte 3 Stunden bei dieser Temperatur. Vorsichtig wurden dann 10 ml einer gesättigten wässrigen $NH_4Cl$-Lösung zugetropft Das Gemisch wurde mit Essigester ausgeschüttelt, worauf die organische Phase mit Wasser gewaschen, mit $MgSO_4$ getrocknet, filtriert und eingeengt wurde. Man chromatographierte den Rückstand über 150 g Kieselgel mit Hexan/Ether (1:1) und erhielt 4.8 g (71%) rac-1,3,3aα,4,5,9bα-Hexahydro-6-methoxy-1α-methyl-3-propyl-2H-benzo[e]indol-2-on als hellgelbes Oel.

c) 1.33 g (0.0035 Mol) Lithiumaluminiumhydrid wurden zu einer Lösung von 4.8 g (0.018 Mol) rac-1,3,3aα,4,5,9bα-Hexahydro-6-methoxy-1α-methyl-3-propyl-2H-benzo[e]indol-2-on in 100 ml Tetrahydrofuran unter Argon gegeben. Das Gemisch wurde 2 Stunden unter Rückfluss gekocht und dann bei 5-10° vorsichtig zunächst mit 10 ml einer gesättigten wässrigen $NH_4Cl$-Lösung und dann mit 50 ml Wasser versetzt und schliesslich mit Essigester ausgeschüttelt. Die organische Phase wurde mit $MgSO_4$ getrocknet, filtriert und eingeengt, worauf der Rückstand über 130 g Kieselgel mit Methylenchlorid/Methanol (49:1) chromatographiert wurde. Man erhielt 4.35 g (95%) rac-2,3,3aα,4,5,9bα-Hexahydro-6-methoxy-1α-methyl-3-propyl-1H-benzo[e]indol als hellgelbes Oel.

d) 4.35 g (0.017 Mol) rac-2,3,3aα,4,5,9bα-Hexahydro-6-methoxy-1α-methyl-3-propyl-1H-benzo[e]indol wurden in 150 ml 48-prozentiger wässriger HBr während 1.5 Stunden unter Rückfluss gekocht, worauf das Gemisch eingeengt wurde. Der Rückstand wurde mit 50 ml Wasser, 5 ml 2N wässriger NaOH-Lösung und 50 ml gesättigter $NaHCO_3$-Lösung neutralisiert und dreimal mit je 100 ml $CH_2Cl_2$ extrahiert. Die vereinigten organischen Phasen wurden über $MgSO_4$ getrocknet, filtriert und eingeengt. Der braune Rückstand (4.8 g) wurde in 10 ml Ethanol und 30 ml Essigester gelöst, worauf 3.7 ml (21.5 mMol) 5.8N ethanolische HCl-Lösung zugetropft wurden. Das Gemisch wurde 4 Stunden bei Raumtemperatur gerührt, wobei Kristalle ausfielen. Die Kristalle wurden abgenutscht, mit Essigester und Ether gewaschen und im Vakuum getrocknet (2.7 g). Aus der Mutterlauge konnten durch Kristallisation noch weitere 1.1 g gewonnen werden. Die gesamte Menge wurde dann in Methanol gelöst, worauf die Lösung mit Aktivkohle behandelt, filtriert und eingeengt wurde. Der Rückstand wurde zweimal aus Methanol/Essigester umkristallisiert, und man erhielt 2.65 g (56%) rac-2,3,3aα,4,5,9bα,Hexahydro-1α-methyl-3-propyl-1H-benzo[e]indol-6-ol-hydrochlorid mit Smp. 201-203°.

e) 1 g (0.0035 Mol) rac-2,3,3aα,4,5,9bα-Hexahydro-1α-methyl-3-propyl-1H-benzo[e]indol-6-ol-hydrochlorid, gelöst in 50 ml Wasser, wurde mit 3.7 ml 1N Natronlauge neutralisiert, worauf mit Ether extrahiert wurde. Die organische Phase wurde mit gesättigten wässrigen $NaHCO_3$- und NaCl-Lösungen gewaschen, über $Na_2SO_4$ getrocknet, filtriert und eingeengt. Der ölige Rückstand wurde mit 50 ml Toluol verdünnt, worauf nacheinander 0.38 g (0.52 ml, 0.004 Mol) Triethylamin und 43.3 mg (355 μMol, 0.1 Aeq.) 4-Dimethylaminopyridin zugegeben und dann 0.34 ml (0.004 Mol) Dimethylcarbamoylchlorid zugetropft wurden. Das Gemisch wurde 2 Stunden unter Rückfluss gekocht, dann abgekühlt und auf Wasser gegossen, worauf mit $CH_2Cl_2$ ausgeschüttelt wurde. Die organische Phase wurde mit gesättigten wässrigen $NaHCO_3$- und NaCl-Lösungen gewaschen, mit $Na_2SO_4$ getrocknet, filtriert und eingeengt. Der Rückstand wurde über 220 g Kieselgel mit Methylenchlorid/Methanol (9:1) chromatographiert. Das erhaltene Oel (1.11 g) wurde in 10 ml Ethanol gelöst, worauf tropfenweise 0.75 ml (3.7 mMol) 4.89N ethanolische HCl-Lösung und dann, bis die Lösung trüb wurde, Essigester zugegeben wurde. Anschliessend wurde das Gemisch 4 Stunden bei Raumtemperatur und 1 Stunde bei 0° gerührt. Die ausgeschiedenen Kristalle wurden abgenutscht, mit Essigester gewaschen und bei 150°/0.02 Torr während 3 Stunden getrocknet. Man erhielt 1.14 g (91%) rac-2,3,3aα,4,5,9bα-Hexahydro-1α-methyl-3-propyl-1H-benzo[e]indol-6-yl-dimethylcarbamat-hydrochlorid als weisse Kristalle mit Smp. 240-247°.

Beispiel 8

a) 8.81 g (0.05 Mol) 8-Methoxy-2-tetralon und 3.91 g (4.55 ml, 0.055 Mol) Pyrrolidin wurden in 50 ml Toluol gelöst. Das Gemisch wurde 2 Stunden am Wasserabscheider gekocht, nach Abkühlen mit 10.2 g (0.06 Mol) Bromessigsäureethylester versetzt, dann weitere 2 Stunden unter Rückfluss gekocht und schliesslich eingeengt. Der Rückstand wurde in 60 ml Ethanol/Wasser (5:1) gelöst, worauf die Lösung 2 Stunden unter Rückfluss gekocht wurde. Dann wurde das Lösungsmittel im Vakuum abdestilliert. Eine Lösung des Rückstandes in Essigester wurde mit Wasser und gesättigter Kochsalzlösung gewaschen, mit $MgSO_4$ getrocknet, filtriert und eingeengt. Der Rückstand wurde über Kieselgel mit Cyclohexan/Essigester (4:1) chromatographiert. Man erhielt 12.0 g (92%) öligen rac-1,2, 3,4-Tetrahydro-8-methoxy-2-oxo-1-naphthylessigsäureethylester.

b) Eine Lösung von 11.8 g (0.045 Mol) rac-1,2,3,4-Tetrahydro-8-methoxy-2-oxo-1-naphthylessigsäureethylester in 100 ml Toluol, wurde mit 7.4 ml (0.09 Mol) Propylamin versetzt. Das Gemisch wurde 20 Stunden am Wasserabscheider gekocht und dann eingeengt. Der Rückstand wurde mit 4 g Raney-Nickel in 200 ml Ethanol bei 120° und 140 Bar hydriert. Das Produkt wurde über Kieselgel mit Cyclohexan/Essigester (1:1) chromatographiert. Man erhielt 10.1 g (87%) öliges rac-cis-1,3,3a, 4,5,9b-Hexahydro-9-methoxy-3-propyl-2H-benzo[e]indol-2-on. Ein Muster wurde aus Hexan kristallisiert und ergab weisse Kristalle mit Smp. 74-76°,

c) 1.44 g (0.038 Mol) Lithiumaluminiumhydrid wurden in 20 ml Tetrahydrofuran unter Argon suspendiert, worauf tropfenweise eine Lösung von 9.83 g (0.038 Mol) rac-cis-1,3,3a,4,5,9b-Hexahydro-9-methoxy-3-propyl-2H-benzo[e]indol-2-on in 50 ml Tetrahydrofuran zugegeben wurde. Das Gemisch wurde 1 Stunde unter Rückfluss gekocht und dann nach dem Abkühlen vorsichtig tropfenweise zunächst mit 10 ml Essigester und dann mit gesättigter wässriger $Na_2SO_4$-Lösung versetzt, bis ein völlig weisser Niederschlag entstanden war. Nach Filtrieren und Einengen des Filtrates erhielt man rac-cis-2,3,3a,4,5,9b-Hexahydro-9-methoxy-3-propyl-1H-benzo[e]indol als Oel, das ohne weitere Reinigung in die nächste Stufe eingesetzt wurde.

d) 8.58 g (0.035 Mol) rac-cis-2,3,3a,4,5,9b-Hexahydro-9-methoxy-3-propyl-1H-benzo[e]indol wurden in 0.5 l 48-prozentiger wässriger HBr 4 Stunden unter Rückfluss gekocht. Das Gemisch wurde auf eine eiskalte wässrige Lösung von 200 g (5 Mol) NaOH gegossen. Man gab festes $NaHCO_3$ zu und schüttelte dreimal mit $CH_2Cl_2$ aus. Die vereinigten organischen Phasen wurden mit gesättigten wässrigen $NaHCO_3$- und NaCl-Lösungen gewaschen, mit $Na_2SO_4$ getrocknet, filtriert und eingeengt. Der Rückstand wurde über Kieselgel mit Essigester chromatographiert. Das Produkt wurde in Hexan kristallisiert. Man erhielt 6.4 g (80%) rac-cis-2,3,3a,4,5,9b-Hexahydro-3-propyl-1H-benzo[e]indol-9-ol als weisse Kristalle mit Smp. 130-132°.

6.22 g (0.027 Mol) davon wurden in 220 ml Essigester/Ethanol (10:1) gelöst, worauf 6 ml (0.035 Mol) einer 5.87M ethanolischen HCl-Lösung zugetropft und 4 Stunden bei 0° gerührt wurde. Man erhielt 6.18 g (82%) rac-cis-2,3,3a,4,5,9b-Hexahydro-3-propyl-1H-benzo[e]indol-9-ol- hydrochlorid als weisse Kristalle mit Smp. 207-213°.

e) Ein Gemisch von 1.10 g (0.004 Mol) rac-cis-2,3,3a,4,5,9b-Hexahydro-3-propyl-1H-benzo[e]indol-9-ol-hydrochlorid, 0.61 g (0.005 Mol) Dimethylcarbamoylchlorid, 1.4 ml (0.01 Mol) Triethylamin und 0.61 g (0.005 Mol) 4-Dimethylaminopyridin in 40 ml Toluol wurde 4 Stunden unter Rückfluss gekocht und dann auf eine gesättigte wässrige Natriumhydrogencarbonatlösung gegossen, worauf mit Methylenchlorid extrahiert wurde. Der Extrakt wurde mit Natriumsulfat getrocknet, filtriert und in Vakuum eingedampft. Der Rückstand wurde durch Chromatographie über Kieselgel mit Methylenchlorid/Methanol (99:1) und über Aluminiumoxid mit Methylenchlorid gereinigt und in Ethanol gelöst. Nach Zugabe von ethanolischer HCl wurde das Lösungsmittel abdestilliert. Es wurden 1.20 g (86%) rac-cis-2,3,3a,4,5,9b-Hexahydro-3-propyl-1H-benzo[e]indol-9-yl-dimethylcarbamat-hydrochlorid als Schaum erhalten.

MS: m/e (% Basispeak): 302 ($M^+$,9) 273(51), 72(100), 42(18).

Beispiel 9

a) Man löste 4,0 g (0.01525 Mol) 1,2,3,4-Tetrahydro-5-methoxy-2-oxo-1-naphthylessigsäureethylester in 80 ml Toluol, fügte 1,98 ml (0.01525 Mol) Aminomethyl-cyclohexan zu und kochte 23 Stunden am Wasserabscheider. Nach Einengen wurde der Rückstand mit 1,5 g Raney-Nickel in 150 ml Ethanol bei 120° und 140 Bar während 10 Stunden hydriert. Nach Chromatographie über Kieselgel mit Cyclohexan/Essigester 3:1 wurde das Produkt in heissem Isopropylether gelöst und bei Raumtemperatur kristallisiert. Man erhielt 3,82 g (80%) rac-cis-3-Cyclohexylmethyl-1,3,3a,4,5,9b-hexahydro-6-methoxy-2H-benzo[e]indol-2-on als weisse Kristalle mit Smp. 136-137°.

b) 0,92 g (0,02412 Mol) Lithiumaluminiumhydrid wurden in 40 ml THF unter Argon suspendiert. Man tropfte eine Lösung aus 3,78 g (0,01206 Mol) rac-cis-3-Cyclohexylmethyl-1,3,3a,4,5,9b-hexahydro-6-methoxy-2H-benzo[e]indol-2-on in 40 ml THF zu und kochte 2 Stunden unter Rückfluss. Vorsichtig tropfte man dann nacheinander 0,9 ml Wasser, 0,9 ml 4N Natronlauge und 2,7 ml Wasser zu, worauf das Gemisch am Rückfluss gekocht wurde, bis ein völlig weisser Niederschlag entstanden war. Nach Zugabe von trockenem $Na_2SO_4$, Filtrieren und Einengen erhielt man ein Oel, das über Kieselgel mit Cyclohexan/Essigester 4:1 chromatographiert wurde. Man erhielt 3,53 g (98%) öliges rac-cis-3-Cyclohexylmethyl-2,3,3a,4,5,9b-hexahydro-6-methoxy-1H-benzo[e]indol.

MS: m/e (% Basis peak) = 299 ($C_{20}H_{29}NO^+$, 5), 216(100), 185(8), 150(10), 144 (7), 115(14).

c) 3,46 g (0,01155 Mol) rac-cis-3-Cyclohexylmethyl-2,3,3a,4,5,9b-hexahydro-6-methoxy-1H-benzo[e]indol wurden in 0,13 l 48%iger wässriger HBr gelöst und 5 Stunden unter Rückfluss gekocht. Das Gemisch wurde auf eine eiskalte wässrige Lösung von 46,3 g (1,16 Mol) NaOH gegossen, worauf nach Zugabe von festem $NaHCO_3$ dreimal mit $CH_2Cl_2$ ausgeschüttelt wurde. Die organische Phase wurde mit gesättigten wässrigen $NaHCO_3$- und NaCl-Lösungen gewaschen, mit $Na_2SO_4$ getrocknet und dann filtriert, worauf das Filtrat eingeengt wurde. Durch Chromatographie des Rückstands über Kieselgel mit Cyclohexan/Essigester 4:1 erhielt man 3,38 g (100%) öliges rac-cis-3-Cyclohexylmethyl-2,3,3a,4,5,9b-hexahydro-1H-benzo[e]indol-6-ol.

0,49 g (0,00171 Mol) des erhaltenen Oels wurden in 15 ml Methanol gelöst. Man tropfte 0,35 ml (0,00173 Mol) 4,93N ethanolische HCl-Lösung zu und engte ein. Das als Rückstand verbleibende Hydrochlorid wurde aus Ethanol/Ether kristallisiert. Ausbeute: 0,47 g (85%) weisse Kristalle mit Smp.

195-197°.

d) 2,78 g (0,00973 Mol) rac-cis-3-Cyclohexylmethyl-2,3,3a,4,5,9b-hexahydro-1H-benzo[e]indol-6-ol wurden in 50 ml Toluol gelöst. Man gab nacheinander 1,97 g (2,71 ml, 0,01948 Mol) Triethylamin und 120 mg (980 µMol, 0,1 Aeq.) 4-Dimethylaminopyridin zu und tropfte dann 1,79 ml (0,01948 Mol) Chlorameisensäuredimethylamid zu. Das Gemisch wurde 2 Stunden unter Rückfluss gekocht, dann abgekühlt, auf Wasser gegossen und mit Toluol ausgeschüttelt. Die organische Phase wurde mit Wasser gewaschen, mit $Na_2SO_4$ getrocknet, filtriert und eingeengt. Durch Chromatographie des Rückstands über 220 g Kieselgel mit Cyclohexan/Essigester 1:1 erhielt man 3,3 g (95%) öliges rac-cis-3-Cyclohexylmethyl-2,3,3a,4,5,9b-hexahydro-1H-benzo[e]indol-6-yl-dimethylcarbamat.

3,24 g (0,00909 Mol) des erhaltenen Oels wurden in 30 ml Ethanol gelöst. Man tropfte 1,83 ml (9,12 mMol) 4,93N ethanolische HCl-Lösung zu und engte ein. Das als Rückstand verbleibende Hydrochlorid wurde aus Acetonitril/Ether kristallisiert. Ausbeute: 2,99 g (82%) weisse Kristalle vom Smp. 192-194°.

Beispiel 10

a) Man löste 4,0 g (0,01525 Mol) 1,2,3,4-Tetrahydro-5-methoxy-2-oxo-1-naphthylessigsäureethylester in 80 ml Toluol, fügte 2,29 ml (0,016 Mol) 3-Phenylpropylamin zu und kochte 20 Stunden am Wasserabscheider. Nach Einengen wurde der Rückstand mit 1,5 g Raney-Nickel in 150 ml Ethanol bei 120° und 140 Bar während 10 Stunden hydriert. Das Produkt wurde über Kieselgel mit Cyclohexan/Essigester 2:1 chromatographiert. Man erhielt 3,13 g (61%) öliges rac-cis-3-(3-Cyclohexyl-propyl)-1,3,3a,4,5,9b-hexahydro-6-methoxy-2H-benzo[e]indol-2-on.

MS: m/e (% Basis peak) = 341 ($C_{22}H_{31}NO_2^+$, 92), 258(52), 245(26), 230(100), 173(72), 158(52), 144(23), 128(19), 115(31).

b) 0,38 g (0,010 Mol) Lithiumaluminiumhydrid wurden in 40 ml THF unter Argon suspendiert. Man tropfte eine Lösung aus 3,26 g (0,00955 Mol) rac-cis-3-(3-Cyclohexyl-propyl)-1,3,3a,4,5,9b-hexahydro-6-methoxy-2H-benzo-[e]indol-2-on in 30 ml THF zu und kochte 2 Stunden unter Rückfluss. Vorsichtig tropfte man dann nacheinander 10 ml Essigester und eine gesättigte wässrige $Na_2SO_4$-Lösung zu, bis ein völlig weisser Niederschlag entstanden war. Nach Filtrieren und Einengen erhielt man ein Oel, das über Kieselgel mit Cyclohexan/Essigester 1:1 chromatographiert wurde. Man erhielt 2,64 g (84%) öliges rac-cis-3-(3-Cyclohexyl-propyl)-2,3,3a,4,5,9b-hexahydro-6-methoxy-1H-benzo[e]indol.

MS: m/e (% Basis peak) = 327 ($C_{22}H_{33}NO^+$, 10), 216(100), 185(5,5), 159(7), 144 (4,5).

c) 2,62 g (0,008 Mol) rac-cis-3-(3-Cyclohexyl-propyl)-2,3,3a,4,5,9b-hexahydro-6-methoxy-1H-benzo[e]-indol wurden in 0,09 l 48%iger wässriger HBr gelöst und 5 Stunden unter Rückfluss gekocht. Das Gemisch wurde auf eine eiskalte wässrige Lösung von 32 g (0,8 Mol) NaOH gegossen, worauf nach Zugabe von festem $NaHCO_3$ dreimal mit $CH_2Cl_2$ ausgeschüttelt wurde. Die organische Phase wurde mit gesättigten wässrigen $NaHCO_3$- und NaCl-Lösungen gewaschen, mit $Na_2SO_4$ getrocknet, filtriert und eingeengt. Durch Chromatographie des Rückstands über Kieselgel mit Cyclohexan/Essigester 1:1 erhielt man 0,92 g (37%) öliges rac-cis-3-(3-Cyclohexyl-propyl)-2,3,3a,4,5,9b-hexahydro-1H-benzo[e]indol-6-ol.

0,201 g des erhaltenen Oels wurden in 1 ml Ethanol gelöst. Man tropfte 0,135 ml (0,673 Mol) 5N ethanolische HCl-Lösung und dann Essigester zu, bis die Lösung trüb wurde. Dann rührte man 1 Stunde bei Raumtemperatur und 1 Stunde bei 0°. Die ausgeschiedenen Kristalle wurden abgenutscht, mit Essigester gewaschen und bei 150°/0,02 Torr während 2 Stunden getrocknet. Man erhielt 0,20 g (89%) des Hydrochlorids als weisse Kristalle mit Smp. 216-221°.

d) 0,67 g (0,00214 Mol) rac-cis-3-(3-Cyclohexyl-propyl)-2,3,3a,4,5,9b-hexahydro-1H-benzo[e]indol-6-ol wurden in 20 ml Toluol gelöst. Man gab nacheinander 0,277 g (0,31 ml, 0,00224 Mol) Triethylamin und 26 mg (214 µMol, 0,1 Aeq.) 4-Dimethylaminopyridin zu und tropfte dann 0,21 ml (0,00224 Mol) Chlorameisensäuredimethylamid zu. Das Gemisch wurde 5 Stunden unter Rückfluss gekocht, dann abgekühlt, auf Wasser gegossen und mit $CH_2Cl_2$ ausgeschüttelt. Die organische Phase wurde mit gesättigten wässrigen $NaHCO_3$- und NaCl-Lösungen gewaschen, mit $Na_2SO_4$ getrocknet, filtriert und eingeengt. Durch Chromatographie des Rückstands über 220 g Kieselgel mit $CH_2Cl_2$/MeOH 9:1 erhielt man 0,787 g eines Oels, welches in ca. 5 ml Ethanol gelöst wurde. Man tropfte 0,42 ml (2,1 mMol) 5N ethanolische HCl-Lösung und dann Essigester zu, bis die Lösung trüb wurde, worauf 16 Stunden bei Raumtemperatur gerührt wurde. Die ausgeschiedenen Kristalle wurden abgenutscht, umkristallisiert und bei 100°/0,02 Torr während 3 Stunden getrocknet. Man erhielt 0,55 g (61%) rac-cis-3-(3-Cyclohexyl-propyl)-2,3,3a,4,5,9b-hexahydro-1H-benzo[e]indol-6-yl-dimethylcarbamat-hydrochlorid als weisse Kristalle mit Smp. 158-163°.

Beispiel 11

a) Man löste 5,0 g (0,0191 Mol) 1,2,3,4-Tetrahydro-5-methoxy-2-oxo-1-naphthylessigsäureethylester in 100 ml Toluol, fügte 3,0 ml (0,0191 Mol) 4-Phenyl-butylamin zu und kochte 16 Stunden am Wasserabscheider. Nach Einengen wurde der Rückstand mit 2,0 g Raney-Nickel in 200 ml Ethanol bei 120° und 140 Bar während 10 Stunden hydriert. Das Produkt wurde über Kieselgel mit n-Hexan/Essigester 3:1 chromatographiert. Man erhielt 5,60 g (82,5%) öliges rac-cis-3-(4-Cyclohexyl-butyl)-1,3,3a,4,5,9b-hexahydro-6-methoxy-2H-benzo[e]indol-2-on.
MS: m/e (% Basis peak) = 355 ($C_{23}H_{33}NO_2^+$, 60), 272(16), 258(11), 244(12), 230 (60), 173(40), 159(30), 115(25), 83(18), 55(100), 41(83).
b) 0,79 g (0,0208 Mol) Lithiumaluminiumhydrid wurden in 20 ml THF unter Argon suspendiert. Man tropfte eine Lösung aus 5,60 g (0,01575 Mol) rac-cis-3-(4-Cyclohexyl-butyl)-1,3,3a,4,5,9b-hexahydro-6-methoxy-2H-benzo[e]indol-2-on in 60 ml THF zu und kochte 1/2 Stunde unter Rückfluss. Vorsichtig tropfte man nacheinander 10 ml THF/Wasser 4:1, 4 ml einer 15%igen wässrigen NaOH-Lösung und 2 ml Wasser zu, worauf das Gemisch 15 Minuten unter Rückfluss gekocht wurde, bis ein völlig weisser Niederschlag entstanden war. Nach Filtrieren, Einengen und Chromatographie des Rückstands über Kieselgel mit n-Hexan/Essigester 1:1 erhielt man 5,0 g (93%) öliges rac-cis-3-(4-Cyclohexyl-butyl)-2,3,3a,4,5,9b-hexahydro-6-methoxy-1H-benzo[e]indol.
c) 5,0 g (0,0146 Mol) rac-cis-3-(4-Cyclohexyl-butyl)-2,3,3a,4,5,9b-hexahydro-6-methoxy-1H-benzo[e]indol-6-ol wurden in 0,17 l 48%iger wässriger HBr gelöst und 5 Stunden unter Rückfluss gekocht. Das Gemisch wurde auf ~200 g Eis gegossen und mit 200 ml 28%iger wässriger NaOH-Lösung versetzt. Man schüttelte dreimal mit Essigester aus, worauf die organische Phase mit gesättigter Kochsalzlösung gewaschen, mit $MgSO_4$ getrocknet, filtriert und eingeengt wurde. Durch Chromatographie des Rückstands über Kieselgel mit n-Hexan/Essigester 1:1 erhielt man 4,3 g (90%) öliges rac-cis-3-(4-Cyclohexyl-butyl)-2,3,3a,4,5,9b-hexahydro-1H-benzo[e]indol-6-ol.

1,0 g (0,00305 Mol) des erhaltenen Oels wurde in 50 ml Ethanol gelöst. Man tropfte 0,74 ml (3,36 mMol) 4,56N ethanolische HCl-Lösung zu, kühlte auf 0° ab und versetzte mit 100 ml Ether. Die ausgeschiedenen Kristalle wurden abgenutscht, mit kaltem Ether gewaschen und bei 150°/0,05 Torr während 1 Stunde getrocknet. Man erhielt 0,98 g (88%) des Hydrochlorids als weisse Kristalle mit Smp. 228-230°.
d) 3,30 g (0,01008 Mol) rac-cis-3-(4-Cyclohexyl-butyl)-2,3,3a,4,5,9b-hexahydro-1H-benzo[e]indol-6-ol wurden in 70 ml 1,2-Dichlorethan gelöst. Man gab nacheinander 3,05 g (4,2 ml, 0,0301 Mol) Triethylamin und 330 mg (2,70 µMol, 0,27 Aeq.) 4-Dimethylaminopyridin zu und tropfte dann 2,8 ml (0,03024 Mol) Chlorameisensäuredimethylamid zu. Das Gemisch wurde 1 Stunde unter Rückfluss gekocht, dann abgekühlt, auf Wasser gegossen und mit $CH_2Cl_2$ ausgeschüttelt. Die organische Phase wurde mit gesättigter wässriger Kochsalzlösung gewaschen, mit $MgSO_4$ getrocknet, filtriert und eingeengt, worauf der Rückstand über Kieselgel mit n-Hexan/Essigester 1:1 chromatographiert wurde. Das erhaltene Oel (3,2 g) wurde in 100 ml Ether gelöst. Man tropfte 1,85 ml (8,83 mMol) 4,78N ethanolische HCl-Lösung zu und beschallte die ölige Suspension 15 Minuten in einem Ultraschallbad. Dann rührte man 1 Stunde bei Raumtemperatur und nutschte die ausgeschiedenen Kristalle ab. Nach Umkristallisation aus 120 ml heissem Essigester und Trocknung bei 100°/0,02 Torr während 3 Stunden erhielt man 2,90 g (83%) rac-cis-3-(4-Cyclohexyl-butyl)-2,3,3a,4,5,9b-hexahydro-1H-benzo-[e]indol-6-yl-dimethylcarbamat-hydrochlorid als weisse Kristalle mit Smp. 145-147°.

Beispiel 12

a) Man löste 5,0 g (0,0246 Mol) rac-cis-2,3,3a,4,5,9b-Hexahydro-6-methoxy-1H-benzo[e]indol in 100 ml $CH_2Cl_2$, fügte 4,1 ml (0,0295 Mol) Triethylamin zu und tropfte bei 15° unter Argon eine Lösung von 4,3 ml (0,0295 Mol) 3-Cyclopentylessigsäurechlorid in 40 ml $CH_2Cl_2$ zu. Das Gemisch wurde 1/2 Stunde bei Raumtemperatur gerührt, dann auf Wasser gegossen und mit $CH_2Cl_2$ausgeschüttelt. Die organische Phase wurde mit gesättigten wässrigen $NaHCO_3$- und NaCl-Lösungen gewaschen, mit $MgSO_4$ getrocknet, filtriert und eingeengt. Durch Chromatographie des Rückstands über Kieselgel mit n-Hexan/Essigester 3:1 erhielt man 6,50 g (84%) hellgelbes öliges rac-cis-3-(Cyclopentyl-acetyl)-2,3,3a,4,5,9b-hexahydro-6-methoxy-1H-benzo[e]indol.
MS: m/e (% Basis peak) = 313 ($C_{20}H_{27}NO_2^+$, 28) 245(26), 186(34), 173(16), 160 (17), 115(13), 87(100).
b) 1,02 g (0,0269 Mol) Lithiumaluminiumhydrid wurden in 30 ml THF unter Argon suspendiert. Man tropfte eine Lösung aus 6,5 g (0,0207 Mol) rac-cis-3-(Cyclopentyl-acetyl)-2,3,3a,4,5,9b-hexahydro-6-methoxy-1H-benzo[e]indol in 80 ml THF zu und kochte 1 Stunde unter Rückfluss. Vorsichtig tropfte man

nacheinander 10 ml THF/Wasser 9:1, 2 ml einer 4N wässrigen NaOH-Lösung und 2 ml Wasser zu, worauf das Gemisch 15 Minuten unter Rückfluss gekocht wurde, bis ein völlig weisser Niederschlag entstanden war. Nach Filtrieren und Einengen erhielt man ein Oel, das in 100 ml Ethanol gelöst wurde. Man tropfte 5,1 ml (0,0245 Mol) 4,78N ethanolische HCl-Lösung zu, rührte 15 Minuten nach und engte ein. Der Rückstand kristallisierte aus 100 ml heissem Essigester. Man erhielt 5,87 g (86%) rac-cis-3-(2-Cyclopentyl-ethyl)-6-methoxy-2,3,3a,4,5,9b-hexahydro-1H-benzo[e]indol-hydrochlorid als weisse Kristalle mit Smp. 185-187° (Zers.).

c) 5,80 g (0,0173 Mol) rac-cis-3-(2-Cyclopentyl-ethyl)-6-methoxy-2,3,3a,4,5,9b-hexahydro-1H-benzo[e]-indol-hydrochlorid wurden in 0,18 l 48%iger wässriger HBr gelöst und 5 Stunden unter Rückfluss gekocht. Das Gemisch wurde auf ~200 g Eis gegossen und mit 200 ml 28%iger wässriger NaOH-Lösung versetzt. Man schüttelte dreimal mit Essigester aus, worauf die organische Phase mit gesättigter Kochsalzlösung gewaschen, mit $MgSO_4$ getrocknet, filtriert und eingeengt wurde. Durch Chromatographie des Rückstands über Kieselgel mit Essigester erhielt man 4,20 g (85%) öliges rac-cis-3-(2-Cyclopentyl-ethyl)-2,3,3a,4,5,9b-hexahydro-1H-benzo[e]indol-6-ol.

1,2 g (0,0042 Mol) des gelblichen Oels wurden in 25 ml Ethanol gelöst. Man tropfte 1,05 ml (0,00505 Mol) 4,80N ethanolische HCl-Lösung zu und versetzte die weisse Suspension mit 50 ml Ether. Man rühte 1 Stunde bei 0°. Die ausgeschiedenen Kristalle wurden abgenutscht, mit Ether gewaschen und bei 150°/0,02 Torr während 2 Stunden getrocknet. Man erhielt 1,20 g (89%) des Hydrochlorids als weisse Kristalle mit Smp. 205-207° (Zers.).

d) 3,0 g (0,0105 Mol) rac-cis-3-(2-Cyclopentyl-ethyl)-2,3,3a,4,5,9b-hexahydro-1H-benzo[e]indol-6-ol wurden in 100 ml 1,2-Dichlorethan gelöst. Man gab nacheinander 3,2 g (4,4 ml, 0,0315 Mol) Triethylamin und 300 mg (0,00446 Mol, 0,25 Aeq.) 4-Dimethylaminopyridin zu und tropfte dann 3,1 ml (0,0315 Mol) Chlorameisensäuredimethylamid zu. Das Gemisch wurde 1,5 Stunden unter Rückfluss gekocht, dann abgekühlt, auf Wasser gegossen und mit $CH_2Cl_2$ ausgeschüttelt. Die organische Phase wurde mit gesättigter wässriger Kochsalzlösung gewaschen, mit $MgSO_4$ getrocknet, filtriert und eingeengt. Durch Chromatographie des Rückstands über Kieselgel mit Essigester/n-Hexan 1:1 erhielt man 3,2 g eines Oels, welches in 60 ml Essigester gelöst wurde. Man tropfte 2,1 ml (0,010 Mol) 4,78N ethanolische HCl-Lösung zu, worauf sich beim Abkühlen auf 0° Kristalle ausschieden. Die Kristalle wurden abgenutscht und bei 130°/0,02 Torr während 1,5 Stunden getrocknet. Man erhielt 2,93 g (83%) rac-cis-3-(2-Cyclopentyl-ethyl)-2,3,3a,4,5,9b-hexahydro-1H-benzo[e]indol-6-yl-dimethylcarbamat-hydrochlorid als weisse Kristalle mit Smp. 178-180° (Zers.).

Beispiel 13

a) Man löste 4,06 g (0,020 Mol) rac-cis-2,3,3a,4,5,9b-Hexahydro-6-methoxy-1H-benzo[e]indol in 80 ml $CH_2Cl_2$, fügte 3,35 ml (0,024 Mol) Triethylamin zu und tropfte bei 20° unter Argon eine Lösung von 3,7 ml (0,024 Mol) 3-Cyclopentylpropionsäurechlorid in 40 ml $CH_2Cl_2$ zu. Das Gemisch wurde 1 Stunde bei Raumtemperatur gerührt, dann auf Wasser gegossen und mit $CH_2Cl_2$ ausgeschüttelt. Die organische Phase wurde mit gesättigten wässrigen $NaHCO_3$- und NaCl-Lösungen gewaschen, mit $MgSO_4$ getrocknet, filtriert und eingeengt. Durch Chromatographie des Rückstands über Kieselgel mit n-Hexan/Essigester 1:1 erhielt man 4,75 g (72,5%) rac-cis-3-(3-Cyclopentyl-propionyl)-2,3,3a,4,5,9b-hexahydro-6-methoxy-1H-benzo[e]indol als hellgelbes öliges Material.

MS: m/e (% Basis peak) = 327 ($C_{21}H_{29}NO_2^+$, 32), 258(20), 245(43), 186(42), 159 (23), 115(21), 100(41), 87(100).

b) 0,71 g (0,0187 Mol) Lithiumaluminiumhydrid wurden in 20 ml THF unter Argon suspendiert. Man tropfte eine Lösung aus 4,7 g (0,01435 Mol) rac-cis-3-(3-Cyclopentyl-propionyl)-2,3,3a,4,5,9b-hexahydro-6-methoxy-1H-benzo[e]indol in 50 ml THF zu und kochte 1/2 Stunde unter Rückfluss. Vorsichtig tropfte man nacheinander 10 ml THF/Wasser 4:1, 4 ml einer 15%igen wässrigen NaOH-Lösung und 2 ml Wasser zu, worauf das Gemisch 10 Minuten unter Rückfluss gekocht wurde, bis ein völlig weisser Niederschlag entstanden war. Nach Filtrieren und Einengen erhielt man ein Oel, das über Kieselgel mit n-Hexan/Essigester 1:1 chromatographiert wurde. Man erhielt 4,40 g (98%) rac-cis-3-(3-Cyclopentyl-propyl)-2,3,3a,4,5,9b-hexahydro-6-methoxy-1H-benzo[e]indol als gelbliches, öliges Material.

MS: m/e (% Basis peak) = 313 ($C_{21}H_{31}NO^+$, 6,8), 216(100), 185(7), 159(6), 144 (3), 128(3), 115(4), 101-(5).

c) 4,40 g (0,014 Mol) rac-cis-3-(3-Cyclopentyl-propyl)-2,3,3a,4,5,9b-hexahydro-6-methoxy-1H-benzo[e]-indol wurden in 0,16 l 48%iger wässriger HBr gelöst und 17 Stunden unter Rückfluss gekocht. Das Gemisch wurde auf ~200 g Eis gegossen und mit 180 ml 28%iger wässriger NaOH-Lösung versetzt. Man schüttelte dreimal mit $CH_2Cl_2$ aus, worauf die organische Phase mit Wasser gewaschen, mit

$MgSO_4$ getrocknet, filtriert und eingeengt wurde. Durch Chromatographie des Rückstands über Kieselgel mit n-Hexan/Essigester 1;1, erhielt man 3,1 g (74%) rac-cis-3-(3-Cyclopentylpropyl)-2,3,3a,4,5,9b-hexahydro-1H-benzo[e]indol-6-ol als öliges Material.

3,0 g (0,010 Mol) des gelbliches Oels wurden in 40 ml Ethanol gelöst. Man tropfte 2,4 ml (11,0 mMol) 4,56N ethanolische HCl-Lösung zu, versetzte die weisse Suspension mit 100 ml Ether und rührte 1 Stunde bei 0°. Die ausgeschiedenen Kristalle wurden abgenutscht, mit Ether gewaschen und bei 150°/0,05 Torr während 1 Stunde getrocknet. Man erhielt 2,95 g (88%) des Hydrochlorids als weisse Kristalle mit Smp. 206-209° (Zers.).

d) 2,0 g (0,00668 Mol) rac-cis-3-(3-Cyclopentyl-propyl)-2,3,3a,4,5,9b-hexahydro-1H-benzo[e]indol-6-ol wurden in 40 ml 1,2-Dichlorethan gelöst. Man gab nacheinander 2,03 g (2,8 ml, 0,020 Mol) Triethylamin und 200 mg (1,64 µMol, 0,25 Aeq.) 4-Dimethylaminopyridin zu und tropfte dann 1,8 ml (0,020 Mol) Chlorameisensäuredimethylamid zu. Das Gemisch wurde 1,5 Stunden unter Rückfluss gekocht, dann abgekühlt, auf Wasser gegossen und mit $CH_2Cl_2$ ausgeschüttelt. Die organische Phase wurde mit gesättigter wässriger Kochsalzlösung gewaschen, mit $MgSO_4$ getrocknet, filtriert und eingeengt. Durch Chromatographie des Rückstands über Kieselgel mit n-Hexan/Essigester 1:1 erhielt man 1,9 g eines Oels, welches in 80 ml Ether gelöst wurde. Man tropfte 1,2 ml (5,64 mMol) 4,78N ethanolische HCl-Lösung zu und beschallte die ölige Suspension 20 Minuten in einem Ultraschallbad. Man nutschte die ausgeschiedenen Kristalle ab und kristallisierte sie aus 100 ml heissem Essigester um. Nach Trocknung bei 100°/0,010 Torr während 2 Stunden erhielt man 1,90 g (91%) rac-cis-3-(3-Cyclopentylpropyl)-2,3,3a,4,5,9b-hexahydro-1H-benzo[e]indol-6-yl-dimethylcarbamat-hydrochlorid als weisse Kristalle mit Smp. 127-130° (Zers.).

Beispiel 14

a) Man löste 4,6 g (0,01755 Mol) 1,2,3,4-Tetrahydro-5-methoxy-2-oxo-1-naphthylessigsäureethylester in 80 ml Toluol, fügte 2,04 ml (0,01755 Mol) 3-Methyl-butylamin zu und kochte 24 Stunden am Wasserabscheider. Nach Einengen wurde der Rückstand mit 1,8 g Raney-Nickel in 200 ml Ethanol bei 120° und 140 Bar während 10 Stunden hydriert. Das erhaltene Material wurde über Kieselgel mit Cyclohexan/Essigester 3:1 chromatographiert. Das Produkt wurde in heissem Isopropylether gelöst und bei Raumtemperatur kristallisiert. Man erhielt 2,84 g (56%) rac-cis-1,3,3a,4,5,9b-Hexahydro-6-methoxy-3-(3-methyl-butyl)-2H-benzo[e]indol-2-on als weisse Kristalle mit Smp. 81-82° und 0,79 g (16%) öliges Material aus den Mutterlaugen.

b) 0,98 g (0,02593 Mol) Lithiumaluminiumhydrid wurden in 50 ml THF unter Argon suspendiert. Man tropfte eine Lösung aus 3,61 g (0,01297 Mol) rac-cis-1,3,3a,4,5,9b-Hexahydro-6-methoxy-3-(3-methyl-butyl)-2H-benzo[e]indol-2-on in 50 ml THF zu und kochte 2 Stunden unter Rückfluss. Vorsichtig tropfte man nacheinander 1,0 ml Wasser, 1,0 ml einer 4N wässrigen NaOH-Lösung und 3,0 ml Wasser zu, worauf das Gemisch am Rückfluss gekocht wurde, bis ein völlig weisser Niederschlag entstanden war. Nach Zugabe von trockenem $Na_2SO_4$, Filtrieren und Einengen erhielt man ein Oel, das über Kieselgel mit Cyclohexan/Essigester 1:1 chromatographiert wurde. Man erhielt 3,23 g (94%) öliges rac-cis-2,3,3a,4,5,9b-Hexahydro-6-methoxy-3-(3-methyl-butyl)-1H-benzo[e]indol.

MS: m/e (% Basis peak) = 273 ($C_{18}H_{27}NO^+$, 10), 216(100), 185(9), 159(8), 144 (3), 128(3), 101(6).

c) 3,19 g (0,01167 Mol) rac-cis-2,3,3a,4,5,9b-Hexahydro-6-methoxy-3-(3-methyl-butyl)-1H-benzo[e]indol wurden in 0,13 l 48%iger wässriger HBr gelöst und 5 Stunden unter Rückfluss gekocht. Das Gemisch wurde auf eine eiskalte wässrige Lösung von 46,3 g (1,16 Mol) NaOH gegossen. Dazu gab man festes $NaHCO_3$ und schüttelte dreimal mit $CH_2Cl_2$ aus. Die organische Phase wurde mit gesättigten wässrigen $NaHCO_3$- und NaCl-Lösungen gewaschen, mit $Na_2SO_4$ getrocknet, filtriert und eingeengt. Das durch Chromatographie des Rückstandes über Kieselgel mit Cyclohexan/Essigester 2:1 erhaltene Produkt wurde in heissem n-Hexan gelöst und bei Raumtemperatur kristallisiert. Man erhielt 2,52 g (75%) rac-cis-2,3,3a,4,5,9b-Hexahydro-3-(3-methyl-butyl)-1H-benzo[e]indol-6-ol als weisse Kristalle mit Smp. 70-72° und 0,59 g (16%) öliges Material aus den Mutterlaugen.

0,40 g (0,00154 Mol) der erhaltenen Kristalle wurden in 40 ml Ethanol gelöst. Man tropfte 0,32 ml (0,00158 Mol) 4,93N ethanolische HCl-Lösung zu und engte ein. Das Hydrochlorid wurde aus heissem Ethanol kristallisiert. Ausbeute: 0,37 g (81%), weisse Kristalle mit Smp. 215-217°.

d) 2,71 g (0,01045 Mol) rac-cis-2,3,3a,4,5,9b-Hexahydro-3-(3-methyl-butyl)1H-benzo[e]indol-6-ol wurden in 60 ml Toluol gelöst. Man gab nacheinander 2,1 g (2,90 ml, 0,0208 Mol) Triethylamin und 128 mg (1045 µMol, 0,1 Aeq.) 4-Dimethylaminopyridin zu und tropfte dann 1,92 ml (0,02088 Mol) Chlorameisensäuredimethylamid zu. Das Gemisch wurde 2 Stunden unter Rückfluss gekocht, dann abgekühlt, auf Wasser gegossen und mit Toluol ausgeschüttelt. Die organische Phase wurde mit Wasser gewaschen,

mit $Na_2SO_4$ getrocknet, filtriert und eingeengt. Durch Chromatographie des Rückstands über Kieselgel mit Essigester erhielt man 2,91 g (84%) öliges rac-cis-2,3,3a,4,5,9b-Hexahydro-3-(3-methyl-butyl)-1H-benzo[e]indol-6-yl-dimethylcarbamat.

2,60 g (0,00786 Mol) des erhaltenen Oels wurden in 30 ml Ethanol gelöst. Man tropfte 1,60 ml (7,89 mMol) 4,93N ethanolische HCl-Lösung zu und engte ein. Das Hydrochlorid wurde aus Acetonitril/Ether kristallisiert. Ausbeute: 2,62 g (91%), weisse Kristalle mit Smp. 206-209°.

Beispiel 15

a) Man löste 4,0 g (0,01525 Mol) 1,2,3,4-Tetrahydro-5-methoxy-2-oxo-1-naphthylessigsäureethylester in 80 ml Toluol, fügte 2,02 ml (0,016 Mol) 2-Phenylethylamin zu und kochte 20 Stunden am Wasserabscheider. Nach Einengen wurde der Rückstand mit 1,5 g Raney-Nickel in 150 ml Ethanol bei 70° und 80 Bar während 10 Stunden hydriert. Das Produkt wurde über Kieselgel mit Cyclohexan/Essigester 1:1 chromatographiert. Das Produkt wurde in Hexan/Essigester kristallisiert. Man erhielt 3,13 g (64%) rac-cis-1,3,3a,4,5,9b-Hexahydro-6-methoxy-3-phenethyl-2H-benzo[e]indol-2-on als weisse Kristalle mit Smp. 89-90°.

b) 0,3557 g (0,00936 Mol) Lithiumaluminiumhydrid wurden in 40 ml THF unter Argon suspendiert. Man tropfte eine Lösung aus 3,01 g (0,00936 Mol) rac-cis-1,3,3a,4,5,9b-Hexahydro-6-methoxy-3-phenethyl-2H-benzo[e]indol-2-on in 40 ml THF zu und kochte 2 Stunden unter Rückfluss. Vorsichtig tropfte man nacheinander 10 ml Essigester und eine gesättigte wässrige $Na_2SO_4$-Lösung zu, bis ein völlig weisser Niederschlag entstanden war. Nach Filtrieren und Einengen erhielt man 2,9 g (100%) öliges rac-cis-2,3,3a,4,5,9b-Hexahydro-6-methoxy-3-phenethyl-1H-benzo[e]indol.

c) 2,88 g (0,00936 Mol) rac-cis-2,3,3a,4,5,9b-Hexahydro-6-methoxy-3-phenethyl-1H-benzo[e]indol wurden in 0,106 l 48%iger wässriger HBr gelöst und 5 Stunden unter Rückfluss gekocht. Das Gemisch wurde auf eine eiskalte wässrige Lösung von 37,4 g (0,936 Mol) NaOH gegossen. Dann gab man festes $NaHCO_3$ zu und schüttelte dreimal mit $CH_2Cl_2$ aus. Die organische Phase wurde mit gesättigten wässrigen $NaHCO_3$- und NaCl-Lösungen gewaschen, mit $Na_2SO_4$ getrocknet, filtriert und eingeengt. Durch Chromatographie des Rückstands über Kieselgel mit Cyclohexan/Essigester 1:1 erhielt man 2,69 g (98%) öliges rac-cis-2,3,3a,4,5,9b-Hexahydro-3-phenethyl-1H-benzo[e]indol-6-ol.

Eine Portion des erhaltenen Oels wurde in 1 ml Ethanol und 10 ml Essigester gelöst. Man tropfte eine 5N ethanolische HCl-Lösung zu und rührte 1 Stunde bei Raumtemperatur und 1 Stunde bei 0°. Die ausgeschiedenen Kristalle wurden abgenutscht und mit Essigester gewaschen. Nach Umkristallisation wurden die Kristalle bei 150°/0,02 Torr während 2 Stunden getrocknet. Man erhielt das Hydrochlorid als weisse Kristalle mit Smp. 270-271°.

d) 2,69 g (0,00917 Mol) rac-cis-2,3,3a,4,5,9b-Hexahydro-3-phenethyl-1H-benzo[e]indol-6-ol wurden in 90 ml Toluol gelöst. Man gab nacheinander 0,974 g (1,34 ml, 0,00963 Mol) Triethylamin und 112 mg (917 µMol, 0,1 Aeq.) 4-Dimethylaminopyridin zu und tropfte dann 0,88 ml (0,00963 Mol) Chlorameisensäuredimethylamid zu. Das Gemisch wurde 5 Stunden unter Rückfluss gekocht, dann abgekühlt, auf Wasser gegossen und mit $CH_2Cl_2$ ausgeschüttelt. Die organische Phase wurde mit gesättigten wässrigen $NaHCO_3$- und NaCl-Lösungen gewachsen, mit $Na_2SO_4$ getrocknet, filtriert und eingeengt. Durch Chromatographie des Rückstands über Kieselgel mit Dichlormethan/Isopropylalkohol und Kristallisation des Produkts in Hexan/Essigester erhielt man 3,08 g (92%) rac-cis-2,3,3a,4,5,9b-Hexahydro-3-phenethyl-1H-benzo[e]indol-6-yl-dimethylcarbamat als gelbliche Kristalle mit Smp. 98-102°.

2,59 g (0,00711 Mol) dieser Kristalle wurden in 10 ml Ethanol und 40 ml Essigester gelöst. Man tropfte 1,64 ml (0,00746 Mol) 4,56N ethanolische HCl-Lösung zu. Dann rührte man 1 Stunde bei Raumtemperatur und 1 Stunde bei 0°. Die ausgeschiedenen Kristalle wurden abgenutscht, umkristallisiert und bei 120°/0,02 Torr während 3 Stunden getrocknet. Man erhielt 2,32 g (81%) des Hydrochlorids als weisse Kristalle mit Smp. 222-226°.

Beispiel 16

a) Man löste 2,35 g (0,0116 Mol) rac-cis-2,3,3a,4,5,9b-Hexahydro-6-methoxy-1H-benzo[e]indol in 40 ml $CH_2Cl_2$, fügte 2,80 ml (0,0201 Mol) Triethylamin zu und tropfte bei 25° unter Argon eine Lösung von 1,1 ml (0,0127 Mol) Propionsäurechlorid in 10 ml $CH_2Cl_2$ zu. Das Gemisch wurde 1 Stunde bei Raumtemperatur gerührt, dann auf Wasser gegossen und mit $CH_2Cl_2$ ausgeschüttelt. Die organische Phase wurde mit $MgSO_4$ getrocknet, filtriert und eingeengt. Durch Chromatographie des Rückstands über Kieselgel mit Ether/Essigester 1:1 erhielt man 3,0 g (99,7%) rac-cis-2,3,3a,4,5,9b-Hexahydro-6-methoxy-3-propionyl-1H-benzo[e]indol als hellgelbes öliges Material, das über Nacht kristallisierte; Smp. 81°.

b) 0,90 g (0,0232 Mol) Lithiumaluminiumhydrid wurden in 40 ml THF unter Argon suspendiert. Man tropfte eine Lösung aus 3,0 g (0,0116 Mol) rac-cis-2,3,3a,4,5,9b-Hexahydro-6-methoxy-3-propionyl-1H-benzo[e]indol in 50 ml THF zu und kochte 1 Stunde unter Rückfluss. Vorsichtig tropfte man 25 ml einer gesättigten wässrigen $NH_4Cl$-Lösung zu, filtrierte und schüttelte das Filtrat mit Essigester aus. Die organische Phase wurde mit Wasser gewaschen, mit $MgSO_4$ getrocknet, filtriert und eingeengt. Durch Chromatographie des Rückstands über Kieselgel mit $CH_2Cl_2$/MeOH 49:1 erhielt man 2,65 g (93%) rac-cis-2,3,3a,4,5,9b-Hexahydro-6-methoxy-3-propyl-1H-benzo[e]indol als farbloses, öliges Material.

c) 2,65 g (0,0108 Mol) rac-cis-2,3,3a,4,5,9b-Hexahydro-6-methoxy-3-propyl-1H-benzo[e]indol wurden in 0,15 l 48%iger wässriger HBr gelöst und 1 Stunde unter Rückfluss gekocht. Das Gemisch wurde eingeengt, und der Rückstand wurde in $CH_2Cl_2$ aufgenommen, worauf mit einem Gemisch einer gesättigten $NaHCO_3$-Lösung (100 ml) und einer 2N NaOH-Lösung (20 ml) ausgeschüttelt wurde. Die Extrakte wurden mit $MgSO_4$ getrocknet, filtriert und eingeengt. Das leicht rosa Oel (2,5 g) wurde in 40 ml Methanol gelöst. Man tropfte 2,05 ml (11,89 mMol) 5,8N ethanolische HCl-Lösung zu und rührte 1 Stunde bei Raumtemperatur und 1 Stunde bei 0°. Die ausgeschiedenen Kristalle wurden abgenutscht und mit Essigester gewaschen. Nach Aufarbeitung der Mutterlauge wurden insgesamt 2,4 g (83%) rac-cis-2,3,3a,4,5,9b-Hexahydro-3-propyl-1H-benzo[e]indol-6-ol-hydrochlorid als weisse Kristalle mit Smp. 242-244° erhalten.

d) 0,68 g (0,00293 Mol) rac-cis-2,3,3a,4,5,9b-Hexahydro-3-propyl-1H-benzo[e]indol-6-ol wurden in 40 ml Toluol gelöst. Man gab nacheinander 0,31 g (0,43 ml, 0,00308 Mol) Triethylamin und 40 mg (0,3 mMol, 0,1 Aeq.) 4-Dimethylaminopyridin zu und tropfte dann 0,28 ml (0,00308 Mol) Chlorameisensäuredimethylamid zu. Das Gemisch wurde 2 Stunden unter Rückfluss gekocht, dann abgekühlt, auf Wasser gegossen und mit Toluol ausgeschüttelt. Die organische Phase wurde mit Wasser gewaschen, mit $Na_2SO_4$ getrocknet, filtriert und eingeengt. Durch Chromatographie des Rückstands über Kieselgel mit Essigester/Ethanol 9:1 erhielt man 0,72 g (0,00238 Mol, 81%) eines Oels, welches in 30 ml Ethanol aufgenommen wurde. Man tropfte 0,48 ml (2,37 mMol) 4,93N ethanolische HCl-Lösung zu und engte ein. Der Rückstand wurde in Ethanol/Ether umkristallisiert, und das rac-cis-2,3,3a,4,5,9b-Hexahydro-3-propyl-1H-benzo[e]indol-6-yl-dimethylcarbamat-hydrochlorid wurde bei 120°/0,02 Torr während 1 Stunde getrocknet. Ausbeute: 0,69 g (85%), weisse Kristalle mit Smp. 168-170°.

Beispiel 17

a) Man löste 4,0 g (0,01525 Mol) 1,2,3,4-Tetrahydro-5-methoxy-2-oxo-1-naphthylessigsäureethylester in 80 ml Toluol, fügte 2,29 ml (0,016 Mol) 3-Phenylpropylamin zu und kochte 20 Stunden am Wasserabscheider. Nach Einengen wurde der Rückstand mit 1,5 g Raney-Nickel in 150 ml Ethanol bei 70° und 80 Bar während 10 Stunden hydriert. Das Produkt wurde über Kieselgel mit Cyclohexan/Essigester 1:1 chromatographiert. Man erhielt 4,02 g (76%) öliges rac-cis-1,3,3a,4,5,9b-Hexahydro-6-methoxy-3-(3-phenylpropyl)-2H-benzo[e]indol-2-on.

MS: m/e (% Basis peak) = 335 ($C_{22}H_{25}NO_2^+$, 52), 231(100), 230(49), 183(31), 159(32), 158(42), 144(16), 115(25), 91(55).

b) 0,457 g (0,01204 Mol) Lithiumaluminiumhydrid wurden in 40 ml THF unter Argon suspendiert. Man tropfte eine Lösung aus 4,04 g (0,01204 Mol) rac-cis-1,3,3a,4,5,9b-Hexahydro-6-methoxy-3-(3-phenylpropyl)-2H-benzo[e]indol-2-on in 40 ml THF zu und kochte 2 Stunden unter Rückfluss. Vorsichtig tropfte man nacheinander 10 ml Essigester und eine gesättigte wässrige $Na_2SO_4$-Lösung zu, bis ein völlig weisser Niederschlag entstanden war. Nach Filtrieren und Einengen erhielt man ein Oel, das über Kieselgel mit Cyclohexan/Essigester 3:1 chromatographiert wurde. Man erhielt 3,46 g (89%) öliges rac-cis-2,3,3a,4,5,9b-Hexahydro-6-methoxy-3-(3-phenyl-propyl)1H-benzo[e]indol.

MS: m/e (% Basis peak) = 321 ($C_{22}H_{27}NO^+$, 10), 216(100), 185(7), 159(7), 144 (3), 115(5,5), 91(16).

c) 3,45 g (0,0107 Mol) rac-cis-2,3,3a,4,5,9b-Hexahydro-6-methoxy-3-(3-phenyl-propyl)-1H-benzo[e]indol wurden in 0,121 l 48%iger wässriger HBr gelöst und 5 Stunden unter Rückfluss gekocht. Das Gemisch wurde auf eine eiskalte wässrige Lösung von 42,8 g (1,07 Mol) NaOH gegossen. Nach Zugabe von festem $NaHCO_3$ wurde dreimal mit $CH_2Cl_2$ ausgeschüttelt. Die organische Phase wurde mit gesättigten wässrigen $NaHCO_3$- und NaCl-Lösungen gewaschen, mit $Na_2SO_4$ getrocknet, filtriert und eingeengt. Durch Chromatographie über Kieselgel mit Cyclohexan/Essigester 1:1 und Kristallisation in Hexan/Essigester erhielt man 2,53 g (77%) rac-cis-2,3,3a,4,5,9b-Hexahydro-3-(3-phenyl-propyl)-1H-benzo-[e]indol-6-ol als weissliche Kristalle mit Smp. 98-100°. Die Mutterlauge wurde eingeengt, und der Rückstand wurde über Kieselgel mit Cyclohexan/Essigester 1:1 chromatographiert. Man erhielt 0,62 g zusätzliches öliges Material (2,12 mMol, 19%); Gesamtausbeute: 96%.

Das erhaltene Oel (0,62 g, 2,12 mMol) wurde in 1 ml Ethanol und 10 ml Essigester gelöst. Man tropfte 0,42 ml (2,12 mMol) 5N ethanolische HCl-Lösung zu und rührte 1 Stunde bei Raumtemperatur und 1 Stunde bei 0°. Die ausgeschiedenen Kristalle wurden abgenutscht und mit Essigester gewaschen. Nach Umkristallisation wurden die Kristalle bei 150°/0,02 Torr während 2 Stunden getrocknet. Man erhielt 0,38 g (55%) des Hydrochlorids als weisse Kristalle mit Smp. 180-183°.

d) 2,5 g (0,00813 Mol) rac-cis-2,3,3a,4,5,9b-Hexahydro-3-(3-phenyl-propyl)-1H-benzo[e]indol-6-ol wurden in 50 ml 1,2-Dichlorethan gelöst. Man gab nacheinander 2,46 g (3,4 ml, 0,0244 Mol) Triethylamin und 250 mg (2,05 mMol, 0,25 Aeq.) 4-Dimethylaminopyridin zu und tropfte dann 2,2 ml (0,0244 Mol) Chlorameisensäuredimethylamid zu. Das Gemisch wurde 1 Stunde unter Rückfluss gekocht, dann abgekühlt, auf Wasser gegossen und mit $CH_2Cl_2$ ausgeschüttelt. Die organische Phase wurde mit gesättigten wässrigen $NaHCO_3$- und NaCl-Lösungen gewaschen, mit $Na_2SO_4$ getrocknet, filtriert und eingeengt, worauf der Rückstand über Kieselgel mit Essigester chromatographiert wurde.

Das erhaltene Oel (3,0 g) wurde in 60 ml Essigester aufgenommen. Man tropfte 1,90 ml (8,66 mMol) 4,56N ethanolische HCl-Lösung zu. Dann rührte man 2 Stunden bei Raumtemperatur und 1 Stunde bei 0°. Die ausgeschiedenen Kristalle wurden abgenutscht, mit Ether gewaschen und bei 120°/0,02 Torr während 1 Stunde getrocknet. Man erhielt 2,80 g (85%) rac-cis-2,3,3a,4,5,9b-Hexahydro-3-(3-phenyl-propyl)-1H-benzo[e]indol-6-yl-dimethylcarbamat-hydrochlorid als weisse Kristalle mit Smp. 173-176°.

Beispiel 18

a) Man löste 4,0 g (0,01525 Mol) 1,2,3,4-Tetrahydro-5-methoxy-2-oxo-1-naphthylessigsäureethylester in 80 ml Toluol, fügte 2,22 ml (0,01525 Mol) N-(3-Aminopropyl)-morpholin zu und kochte 43 Stunden am Wasserabscheider. Nach Einengen wurde der Rückstand mit 1,5 g Raney-Nickel in 150 ml Ethanol bei 120° und 140 Bar während 10 Stunden hydriert. Das Produkt wurde über Kieselgel mit Essigester/Methanol 5:1 chromatographiert, in heissem Isopropylether gelöst und bei Raumtemperatur kristallisiert. Man erhielt 3,58 g (68%) rac-cis-1,3,3a,4,5,9b-Hexahydro-6-methoxy-3-(3-morpholin4-yl-propyl)-2H-benzo[e]indol-2-on als weisse Kristalle mit Smp. 65-67° und 0,93 g (18%) öliges Material aus den Mutterlaugen.

b) 0,99 g (0,02595 Mol) Lithiumaluminiumhydrid wurden in 50 ml THF unter Argon suspendiert. Man tropfte eine Lösung aus 4,47 g (0,01298 Mol) rac-cis-1,3,3a,4,5,9b-Hexahydro-6-methoxy-3-(3-morpholin-4-yl-propyl)-2H-benzo[e]indol-2-on in 50 ml THF zu und kochte 2 Stunden unter Rückfluss. Vorsichtig tropfte man nacheinander 1,0 ml Wasser, 1,0 ml einer 4N wässrigen NaOH-Lösung und 3,0 ml Wasser zu, worauf das Gemisch am Rückfluss gekocht wurde, bis ein völlig weisser Niederschlag entstanden war. Nach Zugabe von trockenem $Na_2SO_4$, Filtrieren und Einengen erhielt man ein Oel, das über Kieselgel mit Essigester/Methanol 1:1 chromatographiert wurde. Man erhielt 3,79 g (88%) öliges rac-cis-2,3,3a,4,5,9b-Hexahydro-6-methoxy-3-(3-morpholin-4-yl-propyl)-1H-benzo[e]indol. MS: m/e (% Basis peak) = 330 ($C_{20}H_{30}N_2O_2^+$, 18), 243(33), 228(46), 216(95), 202 (51), 160(19), 159(19), 127(52), 112(35), 100(100).

c) 3,75 g (0,01135 Mol) rac-cis-2,3,3a,4,5,9b-Hexahydro-6-methoxy-3-(3-morpholin-4-yl-propyl)-1H-benzo-[e]indol wurden in 0,13 l 48%iger wässriger HBr gelöst und 5 Stunden unter Rückfluss gekocht. Das Gemisch wurde auf eine eiskalte wässrige Lösung von 46,3 g (1,16 Mol) NaOH gegossen. Nach Zugabe von festem $NaHCO_3$ schüttelte man dreimal mit $CH_2Cl_2$ aus. Die organische Phase wurde mit gesättigten wässrigen $NaHCO_3$- und NaCl-Lösungen gewaschen, mit $Na_2SO_4$ getrocknet, filtriert und eingeengt. Durch Chromatographie über Kieselgel mit Methanol erhielt man 3,20 g (89%) öliges rac-cis-2,3,3a,4,5,9b-Hexahydro-3-(3-morpholin-4-yl-propyl)-1H-benzo[e]indol-6-ol.

0,39 g (0,00123 Mol) des erhaltenen Oels wurden in 50 ml Ethanol gelöst. Man tropfte 0,50 ml (0,002475 Mol) 4,93N ethanolische HCl-Lösung zu und engte ein. Das Dihydrochlorid wurde aus Ethanol/Ether kristallisiert. Ausbeute: 0,37 g (77%), weisse Kristalle mit Smp. 219-221°.

d) 3,37 g (0,01065 Mol) rac-cis-2,3,3a,4,5,9b-Hexahydro-3-(3-morpholin-4-yl-propyl)-1H-benzo[e]indol-6-ol wurden in 60 ml Toluol gelöst. Man gab nacheinander 2,155 g (2,97 ml, 0,0214 Mol) Triethylamin und 130 mg (1065 µMol, 0,1 Aeq.) 4-Dimethylaminopyridin zu und tropfte dann 1,96 ml (0,0213 Mol) Chlorameisensäuredimethylamid zu. Das Gemisch wurde 2 Stunden unter Rückfluss gekocht, dann abgekühlt, auf Wasser gegossen und mit Toluol ausgeschüttelt. Die organische Phase wurde mit Wasser gewaschen, mit $Na_2SO_4$ getrocknet, filtriert und eingeengt. Durch Chromatographie des Rückstands über Kieselgel mit Methanol erhielt man 3,66 g (89%) öliges rac-cis-2,3,3a,4,5,9b-Hexahydro-3-(3-morpholin-4-yl-propyl)-1H-benzo[e]indol-6-yl-dimethylcarbamat.

3,61 g (0,01017 Mol) des erhaltenen Oels wurden in 40 ml Ethanol gelöst. Man tropfte 4,17 ml (20,56 mMol) 4,93N ethanolische HCl-Lösung zu und engte ein. Das Dihydrochlorid wurde aus Ethanol/Ether

kristallisiert. Ausbeute: 3,90 g (91%), weisse Kristalle mit Smp. 233-235°.

Beispiel 19

a) Man löste 4,0 g (0,01525 Mol) 1,2,3,4-Tetrahydro-5-methoxy-2-oxo-1-naphthylessigsäurethylester in 250 ml Toluol, fügte 4,05 ml (0,0305 Mol) 2-Piperidinoethylamin und 0,13 g p-Toluolsulfonsäure-monohydrat zu und kochte 4 Stunden am Wasserabscheider. Nach Einengen wurde der Rückstand mit 1,3 g Raney-Nickel in 500 ml Ethanol bei 120° und 140 Bar während 10 Stunden hydriert. Das Produkt wurde über Kieselgel mit $CH_2Cl_2$/MeOH 49:1 chromatographiert. Man erhielt 4,45 g (89%) öliges rac-cis-1,3,3a,4,59b-Hexahydro-6-methoxy-3-(2-piperidinoethyl)-2H-benzo[e]indol-2-on.

b) 1,0 g (0,0268 Mol) Lithiumaluminiumhydrid wurden in 50 ml THF unter Argon suspendiert. Man tropfte eine Lösung aus 4,4 g (0,0134 Mol) rac-cis-1,3,3a,4,5,9b-Hexahydro-6-methoxy-3-(2-piperidinoethyl)-2H-benzo[e]indol-2-on in 100 ml THF zu und kochte 1 Stunde unter Rückfluss. Vorsichtig tropfte man 25 ml einer gesättigten wässrigen $NH_4Cl$-Lösung zu, filtrierte und schüttelte das Filtrat mit Essigester aus. Die organische Phase wurde mit Wasser gewaschen, mit $MgSO_4$ getrocknet, filtriert und eingeengt. Man erhielt 3,8 g (90%) rac-cis-2,3,3a,4,5,9b-Hexahydro-6-methoxy-3-(2-piperidinoethyl)-1H-benzo[e]indol als farbloses Oel.

c) 3,8 g (0,0121 Mol) rac-cis-2,3,3a,4,5,9b-Hexahydro-6-methoxy-3-(2-piperidinoethyl)-1H-benzo[e]indol wurden in 0,35 l 48%iger wässriger HBr gelöst und 5 Stunden unter Rückfluss gekocht. Das Gemisch wurde eingeengt, und der Rückstand wurde in $CH_2Cl_2$ aufgenommen, worauf mit einem Gemisch von 100 ml gesättigter $NaHCO_3$-Lösung und 20 ml 2N NaOH-Lösung ausgeschüttelt wurde. Die Extrakte wurden mit $MgSO_4$ getrocknet, filtriert und eingeengt. Der als Rückstand verbleibende Schaum (3,8 g) wurde in 50 ml Ethanol gelöst. Man filtrierte, tropfte 4,8 ml (27,84 mMol) 5,8N ethanolische HCl-Lösung zu und rührte 1 Stunde bei Raumtemperatur und 1 Stunde bei 0°. Die ausgeschiedenen Kristalle wurden abgenutscht und mit Essigester gewaschen. Man erhielt 4,3 g (95%) rac-cis-2,3,3a,4,5,9b-Hexahydro-3-(2-piperidinoethyl)-1H-benzo[e]indol-6-ol-dihydrochlorid als weisse Kristalle mit Smp. 265-267°.

d) 1,0 g (0,00268 Mol) rac-cis-2,3,3a,4,5,9b-Hexahydro-3-(2-piperidinoethyl)-1H-benzo[e]indol-6-ol-dihydrochlorid wurde in einer verdünnten wässrigen NaOH-Lösung gelöst und mit Essigester geschüttelt. Die organische Phase wurde mit Wasser gewaschen, über $Na_2SO_4$ getrocknet, filtriert und eingeengt. Der Rückstand wurde in 40 ml Toluol gelöst, worauf nacheinander 0,28 g (0,39 ml, 0,0279 Mol) Triethylamin und 35 mg (0,28 mMol, 0,1 Aeq.) 4-Dimethylaminopyridin zugegeben und dann 0,26 ml (0,0279 Mol) Chlorameisensäuredimethylamid zugetropft wurden. Das Gemisch wurde 2 Stunden unter Rückfluss gekocht, dann abgekühlt, auf Wasser gegossen und mit Toluol ausgeschüttelt. Die organische Phase wurde mit Wasser gewaschen, mit $Na_2SO_4$ getrocknet, filtriert und eingeengt. Durch Chromatographie des Rückstands über Kieselgel mit Essigester/Ethanol 1:1 erhielt man 0,82 g (82%) öliges rac-cis-2,3,3a,4,5,9b-Hexahydro-3-(2-piperidinoethyl)-1H-benzo[e]indol-6-yl-dimethylcarbamat.

0,76 g (0,00205 Mol) des erhaltenen Oels wurden in 30 ml Ethanol gelöst. Man tropfte 0,83 ml (4,09 mMol) 4,93N ethanolische HCl-Lösung zu, engte ein und kristallisierte aus Ethanol/Ether um. Die Kristalle wurden abgenutscht, mit Ether gewaschen und bei 120°/0,02 Torr während 1 Stunde getrocknet. Man erhielt 0,84 g (92%) des Dihydrochlorids als weisse Kristalle mit Smp. 183-185°.

Beispiel 20

a) 10,67 g (52,5 mMol) rac-cis-2,3,3a,4,5,9b-Hexahydro-6-methoxy-1H-benzo[e]indol wurden mit 250 ml Toluol, 14,5 g (63 mMol) N-(4-Brombutyl)-3,3-dimethylglutarimid und 6,9 g (9,5 ml; 68,2 mMol) Triethylamin versetzt und in einem Oelbad über Nacht bei einer Oelbadtemperatur von 115° gerührt. Nach dem Abkühlen wurde der ausgefallene Niederschlag abgesaugt und mit Toluol gewaschen, worauf das Filtrat eingedampft wurde. Das erhaltene braune Oel (20,7 g) wurde über eine 20-fache Menge Kieselgel mit Essigester filtriert. Die erhaltene Base (12,6 g bräunliches Oel) wurde auf übliche Art in das Hydrochlorid übergeführt, und man erhielt 11,9 g (23%) rac-cis-1-[4-(6-Methoxy-2,3,3a,4,5,9b-hexahydro-1H-benzo[e]-indol-3-yl)-butyl]-4,4-dimethyl-piperidin-2,6-dion-hydrochlorid in Form gelber Kristalle vom Smp. 115-116°.

b) 8,8 g (22,1 mMol) rac-cis-1-[4-(6-Methoxy-2,3,3a,4,5,9b-hexahydro-1H-benz[e]indol-3-yl)-butyl]-4,4-dimethyl-piperidin-2,6-dion wurden mit 100 g Pyridin-hydrochlorid gut vermischt und in einem Oelbad während 4 Stunden bei einer Oelbadtemperatur von 180° unter Argonatmosphäre gerührt. Nach dem Abkühlen wurde das Gemisch mit Eiswasser versetzt, worauf die braune Lösung mit Ammoniumhydroxidlösung alkalisch gestellt und dann mit Methylenchlorid extrahiert wurde. Die organische Phase wurde mit Wasser gewaschen, über Magnesiumsulfat getrocknet und eingedampft. Das erhaltene braune ölige

Produkt (9,8 g) wurde an der 30-fachen Menge Kieselgel chromatographiert. Die im Dünnschichtchromatogramm einheitlichen mittels Methylenchlorid/Essigester 1:1 erhaltenen Eluate wurden zusammengefasst und eingedampft. Das erhaltene Oel kristallisierte nach Versetzen mit Hexan. Man erhielt 5,5 g (65%) rac-cis-1-[4-(6-Hydroxy-2,3,3a,4,5,9b-hexahydro-1H-benzo[e]indol-3-yl)-butyl]-4,4-dimethylpiperidin-2,6-dion in Form gelblicher Kristalle vom Smp. 131-132°.

c) 5,0 g (13,0 mMol) rac-cis-1-[4-(6-Hydroxy-2,3,3a,4,5,9b-hexahydro-1H-benzo[e]indol-3-yl)-butyl]-4,4-dimethyl-piperidin-2,6-dion wurden in 250 ml Toluol gelöst. Die Lösung wurde mit 2,76 g (3,8 ml, 27 mMol) Triethylamin und 1,68 g (1,43 ml, 16 mMol) Dimethylcarbamoylchlorid und 250 mg 4-Dimethylaminopyridin versetzt und unter Argonatmosphäre in einem Oelbad über Nacht bei einer Oelbadtemperatur von 110° gerührt. Nach dem Abkühlen wurde die organische Phase nacheinander mit Wasser, Natriumbicarbonatlösung und Wasser gewaschen, über Magnesiumsulfat getrocknet und eingedampft. Das erhaltene braune Oel (6,5 g) wurde über eine 30-fache Menge Kieselgel chromatographiert. Die im Dünnschichtchromatogramm einheitlichen mittels Methylenchlorid/Essigester 1:1 erhaltenen Eluate wurden zusammengefasst und eingedampft. Die erhaltene gelbe ölige Base (4,0 g) wurde auf übliche Weise ins Hydrochlorid übergeführt, wobei 4,0 g (62%) rac-cis-3-[4-(4,4-Dimethyl-2,6-dioxo-piperidin-1-yl)-butyl]-2,3,3a,4,5,9b-hexahydro-1H-benzo[e]indol-6-yl-dimethylcarbamat-hydrochlorid als gelbliche Kristalle vom Smp. 190-192° (Zers.) erhalten wurden.

Beispiel 21

a) Analog wie in Beispiel 20a) beschrieben wurde durch Umsetzung von rac-cis-2,3,3a,4,5,9b-Hexahydro-6-methoxy-1H-benzo[e]indol mit N-(5-Brompentyl)-3,3-dimethylglutarimid rac-cis-1-[5-(6-Methoxy-2,3,3a,4,5,9b-hexahydro-1H-benzo[e]indol-3-yl)pentyl]-4,4-dimethylpiperidin-2,6-dion erhalten und in Form des Hydrochlorids als gelbliche Kristalle vom Smp. 112-114° (Zers.) isoliert; Ausbeute: 59%.

b) Analog wie in Beispiel 20b) beschrieben wurden aus 9,2 g (21,6 mMol) rac-cis-1-[5-(6-Methoxy-2,3,3a,4,5,9b-hexahydro-1H-benzo[e]indol-3-yl)pentyl]4,4-dimethylpiperidin-2,6-dion mittels Pyridin-hydrochlorid 7,9 g rac-cis-1-[5-(6-Hydroxy-2,3,3a,4,5,9b-hexahydro-1H-benzo[e]indol-3-yl)pentyl]-4,4-dimethylpiperidin-2,6-dion erhalten; gelbliche Kristalle vom Smp. 133-134° nach Kristallisation aus t-Butylmethyläther/Hexan.

c) 5,0 g (12,54 mMol) rac-cis-1-[5-(6-Hydroxy-2,3,3a,4,5,9b-hexahydro-1H-benzo[e]indol-3-yl)pentyl]-4,4-dimethylpiperidin-2,6-dion wurden in 250 ml Toluol gelöst, mit 2,67 g (3,7 ml; 26,4 mMol) Triethylamin, 1,62 g (1,4 ml, 15,4 mMol) Dimethylcarbamoylchlorid und 250 mg 4-Dimethylaminopyridin versetzt und in einem Oelbad über Nacht bei einer Oelbadtemperatur von 110° gerührt. Nach dem Abkühlen wurde die organische Phase nacheinander mit Wasser, Natriumbicarbonatlösung und Wasser gewaschen, über Magnesiumsulfat getrocknet und eingedampft. Das erhaltene braune Oel (6,3 g) wurde über eine 30-fache Menge Kieselgel mit Methylenchlorid/Essigester 1:1 chromatographiert, und die gereinigte Base (5,9 g hellgelbes Oel) wurde wie üblich in das Hydrochlorid übergeführt. Nach Kristallisation aus Essigester/Ether erhielt man 5,1 g (80%) rac-cis-3-[5-(4,4-Dimethyl-2,6-dioxopiperidin-1-yl)pentyl]-2,3,3a,4,5,9b-hexahydro-1H-benzo[e]indol-6-yl-dimethylcarbamat-hydrochlorid in Form gelblicher Kristalle vom Smp. 133-135° (Zers.).

Beispiel 22

a) Analog wie in Beispiel 20a) beschrieben wurde aus rac-cis-2,3,3a,4,5,9b-Hexahydro-6-methoxy-1H-benzo[e]indol durch Umsetzung mit N-(6-Bromhexyl)-3,3-dimethylglutarimid rac-cis-1-[6-(6-Methoxy-2,3,3a,4,5,9b-hexahydro-1H-benzo[e]indol-3-yl)-hexyl]-4,4-dimethylpiperidin-2,6-dion erhalten; Smp. des Hydrochlorids 99-101°, gelbliche Kristalle, Ausbeute 72%.

b) Analog wie in Beispiel 20b) beschrieben wurden aus 9,2 g (21,56 mMol) rac-cis-1-[6-(6-Methoxy-2,3,3a,4,5,9b-hexahydro-1H-benzo[e]indol-3-yl)-hexyl]-4,4-dimethylpiperidin-2,6-dion mittels Pyridin-hydrochlorid 7,9 g (89%) rac-cis-1-[6-(6-Hydroxy-2,3,3a,4,5,9b-hexahydro-1H-benzo[e]indol-3-yl)hexyl]-4,4-dimethylpiperidin-2,6-dion erhalten; gelbliche Kristalle vom Smp. 98,5-99°, nach Kristallisation aus Ether/Hexan.

c) 5,0 g (12,12 mMol) rac-cis-1-[6-(6-Hydroxy-2,3,3a,4,5,9b-hexahydro-1H-benzo[e]indol-3-yl)hexyl]-4,4-dimethylpiperidin-2,6-dion wurden in 200 ml Toluol gelöst, worauf die Lösung mit 2,57 g (3,55 ml, 25,45 mMol) Triethylamin, 1,56 g (1,33 ml, 14,53 mMol) Dimethylcarbamoylchlorid und 250 mg 4-Dimethylaminopyridin versetzt und in einem Oelbad unter Argonatmosphäre über Nacht bei einer Oeldbadtemperatur von 110° gerührt wurde. Nach dem Abkühlen wurde die organische Phase nacheinander mit Wasser, Natriumbicarbonatlösung und Wasser gewaschen, über Magnesiumsulfat getrocknet und eingedampft.

Das erhaltene braune Oel (6,0 g) wurde in der 30-fachen Menge Kieselgel chromatographiert. Nach Einengen der mittels Methylenchlorid/Essigester 1:1 erhaltenen Eluate erhielt man 3,0 g gelbliches rac-cis-3-[6-(4,4-Dimethyl-2,6-dioxo-piperidin-1-yl)hexyl]-2,3,3a,4,5,9b-hexahydro-1H-benzo[e]indol-6-yl-dimethylcarbamat, welches in üblicher Weise in das Hydrochlorid übergeführt wurde. Man erhielt 3,0 g (48%) Hydrochlorid in Form gelblicher Kristalle vom Smp. 144,5-146°.

Beispiel 23

Eine Suspension von 2,00 g (0,0067 Mol) (+)-cis-3-(2-Cyclohexylethyl)-2,3,3a,4,5,9b-hexahydro-1H-benzo[e]indol-6-ol in 100 ml Acetonitril wurde mit 1,9 ml (0,013 Mol) Triethylamin und 1,6 ml (0,027 Mol) Methylisocyanat versetzt. Das Gemisch wurde 5 Stunden unter Rückfluss gekocht und dann auf wässrige Natriumhydrogencarbonat-Lösung gegossen, worauf mit Methylenchlorid extrahiert wurde. Nach Trocknen der organischen Phase mit Natriumsulfat und Abdestillieren des Lösungsmittels wurde der Rückstand über Kieselgel mit Methylenchlorid/Methanol 20:1 chromatographiert. Das erhaltene Oel wurde in Ethanol gelöst und mit einer äquimolaren Menge ethanolischer HCl versetzt. Mach Einengen löste man den Rückstand in Methylenchlorid, behandelte mit Aktivkohle und Natriumsulfat, filtrierte und destillierte das Lösungsmittel ab. Der Rückstand wurde in Essigester suspendiert, abfiltriert und im Vakuum getrocknet. Man erhielt 1,58 g (60%) (+)-cis-3-(2-Cyclohexylethyl)-2,3,3a,4,5,9b-hexahydro-1H-benzo[e]indol-6-yl-methylcarbamat-hydrochiorid mit Smp. 209-212°.
MS: m/e (% Basis peak) = 356 ($M^+$, 9), 299(4), 259(100), 202(66), 171(6).

Beispiel 24

Eine Suspension von 2,00 g (0,0067 Mol) (+)-cis-3-(2-Cyclohexylethyl)-2,3,3a,4,5,9b-hexahydro-1H-benzo[e]indol-6-ol in 100 ml Acetonitril wurde mit 1,9 ml (0,013 Mol) Triethylamin und 2,1 ml (0,027 Mol) Ethylisocyanat versetzt. Das Gemisch wurde 20 Stunden unter Rückfluss gekocht und dann auf wässrige Natriumhydrogencarbonat-Lösung gegossen, worauf mit Methylenchlorid extrahiert wurde. Nach Trocknen der organischen Phase mit Natriumsulfat und Abdestillieren des Lösungsmittels wurde der Rückstand über Kieselgel mit Methylenchlorid/Methanol 20:1 chromatographiert. Das erhaltene Oel wurde in Ethanol gelöst und mit einer äquimolaren Menge ethanolischer HCl versetzt. Mach Einengen löste man den Rückstand in Methylenchlorid, behandelte mit Aktivkohle und Natriumsulfat, filtrierte und destillierte das Lösungsmittel im Vakuum ab. Der Rückstand wurde in Essigester suspendiert, abfiltriert und im Vakuum getrocknet. Man erhielt 1,39 g (51%) (+)-cis-3-(2-Cyclohexylethyl)-2,3,3a,4,5,9b-hexahydro-1H-benzo[e]indol-6-yl-ethylcarbamat-hydrochlorid mit Smp. 133-136°.
MS: m/e (% Basis peak) = 370 ($M^+$, 4), 299(4), 273(71), 202(100), 171(9), 145 (8).

Beispiel 25

Eine Suspension von 2,00 g (0,0067 Mol) (+)-cis-3-(2-Cyclohexylethyl)-2,3,3a,4,5,9b-hexahydro-1H-benzo[e]indol-6-ol in 100 ml Acetonitril wurde mit 1,9 ml (0,013 Mol) Triethylamin und 2,6 ml (0,027 Mol) Propylisocyanat versetzt. Das Gemisch wurde 20 Stunden unter Rückfluss gekocht und dann auf wässrige Natriumhydrogencarbonat-Lösung gegossen, worauf mit Methylenchlorid extrahiert wurde. Nach Trocknen der organischen Phase mit Natriumsulfat und Abdestillieren des Lösungsmittels wurde der Rückstand über Kieselgel mit Methylenchlorid/Methanol 20:1 chromatographiert. Das erhaltene Oel wurde in Essigester gelöst und mit einer äquimolaren Menge ethanolischer HCl versetzt. Nach Trocknen mit Natriumsulfat und Einengen auf ein Volumen von ca. 30 ml konnten durch Filtration 1,78 g (63%) (+)-cis-3-(2-Cyclohexylethyl)-2,3,3a,4,5,9b-hexahydro-1H-benzo[e]indol-6-yl-propylcarbamat-hydrochlorid mit Smp. 125-131° erhalten werden.
MS: m/e (% Basis peak) = 384 ($M^+$, 6), 287(64), 202(100), 171(7).

Beispiel 26

Eine Suspension von 2,00 g (0,0067 Mol) rac-cis-3-(2-Cyclohexylethyl)-2,3,3a,4,5,9b-hexahydro-1H-benzo[e]indol-6-ol in 100 ml Acetonitril wurde mit 1,9 ml (0,013 Mol) Triethylamin und 3,0 ml (0,027 Mol) Butylisocyanat versetzt. Das Gemisch wurde 20 Stunden unter Rückfluss gekocht und dann auf wässrige Natriumhydrogencarbonat-Lösung gegossen, worauf mit Methylenchlorid extrahiert wurde. Nach Trocknen der organischen Phase mit Natriumsulfat und Abdestillieren des Lösungsmittels wurde der Rückstand über Kieselgel mit Methylenchlorid/Methanol 20:1 chromatographiert. Das erhaltene Oel wurde in Methanol gelöst

und mit einer äquimolaren Menge ethanolischer HCl versetzt. Nach Trocknen mit Natriumsulfat, Einengen auf ein Volumen von ca. 30 ml und Filtrieren wurden 1,11 g (38%) rac-cis-3-(2-Cyclohexylethyl)-2,3,3a,4,5,9b-hexahydro-1H-benzo[e]indol-6-yl-butylcarbamat-hydrochlorid mit Smp. 192-194° erhalten.
MS: m/e (% Basis peak) = 398 ($M^+$, 5), 301(69), 202(100).

Beispiel 27

Eine Suspension von 2,00 g (0,0067 Mol) (+)-cis-3-(2-Cyclohexylethyl)-2,3,3a,4,5,9b-hexahydro-1H-benzo[e]indol-6-ol in 100 ml Toluol wurde mit 1,1 ml (0,008 Mol) Triethylamin, 1,00 g (0,008 Mol) 4-Dimethylaminopyridin und 1,00 g (0,008 Mol) Chlorameisensäureethylmethylamid versetzt. Das Gemisch wurde 4 Stunden unter Rückfluss gekocht und dann auf wässrige Natriumhydrogencarbonat-Lösung gegossen, worauf mit Methylenchlorid extrahiert wurde. Nach Trocknen der organischen Phase mit Natriumsulfat und Abdestillieren des Lösungsmittels wurde der Rückstand über Kieselgel mit Methylenchlorid/Methanol 20:1 chromatographiert. Das erhaltene Oel wurde in Essigester gelöst und mit einer äquimolaren Menge ethanolischer HCl versetzt. Nach Trocknen mit Natriumsulfat, Einengen auf ein Volumen von ca. 25 ml und Filtrieren wurden 24,9 g (88%) (+)-cis-3-(2-Cyclohexylethyl)-2,3,3a,4,5,9b-hexahydro-1H-benzo-[e]indol-6-yl-ethyl-methylcarbamat-hydrochlorid mit Smp. 181-184° erhalten.
MS: m/e (% Basis peak) = 384 ($M^+$, 9), 287(100), 200(3), 86(24).

Beispiel 28

a) Eine Lösung von 1,00 g (0,005 Mol) rac-cis-2,3,3a,4,5,9b-Hexahydro-6-methoxy-1H-benzo[e]indol in 60 ml Ethylmethylketon wurde mit 0,86 g (0,006 Mol) Kaliumcarbonat, 0,29 g (0,002 Mol) Natriumjodid und 1,50 g (0,005 Mol) N-(5-Brompentyl)phthalimid versetzt. Das Gemisch wurde 24 Stunden unter Rückfluss erhitzt und nach Abkühlen mit Wasser versetzt. Man extrahierte mit Essigester, trocknete die Extrakte mit Natriumsulfat und destillierte das Lösungsmittel im Vakuum ab. Chromatographie über Kieselgel mit Methylenchlorid/Methanol 20:1 ergab 1,97 g (95%) rac-N-[5-(cis-1,2,3a,4,5,9b-Hexahydro-6-methoxy-3H-benzo[e]indol-3-yl)pentyl]phthalimid.
MS: m/e (% Basis peak) = 418 ($M^+$, 7), 216(100), 185(7). Smp. des Hydrochlorids 158-160°.
b) Eine Lösung von 15,0 g (0,036 Mol) rac-N-[5-(cis-1,2,3a,4,5,9b-Hexahydro-6-methoxy-3H-benzo[e]-indol-3-yl)pentyl]phthalimid in 120 ml Methylenchlorid wurde tropfenweise mit einer Lösung von 17,3 ml (0,179 Mol) Bortribromid in 60 ml Methylenchlorid versetzt, worauf 2 Stunden bei 0° gerührt wurde. Unter Kühlen mit einem Eisbad wurde eine Lösung aus 20,4 g (0,51 Mol) Natriumhydroxid in 70 ml Wasser zugetropft. Das Gemisch wurde auf Wasser gegossen, worauf wässrige Natriumhydrogencarbonat-Lösung zugegeben wurde, bis ein pH von 8 erreicht war. Man extrahierte mit Methylenchlorid, trocknete die Extrakte über Natriumsulfat und destillierte das Lösungsmittel im Vakuum ab. Chromatographie über Kieselgel mit Essigester lieferte 3,1 g (21%) rac-N-[5-(cis-1,2,3a,4,5,9b-Hexahydro-6-hydroxy-3H-benzo[e]indol-3-yl)pentyl]phthalimid als braunes Oel. Eine kleine Probe wurde mit äthanolischer HCl in das Hydrochlorid überführt, Smp. 246-248°.
MS: m/e (% Basis peak) = 404 ($M^+$, 7), 202(100), 160(6), 145(5).
c) Ein Gemisch aus 2,50 g (0,006 Mol) rac-N-[5-(cis-1,2,3a,4,5,9b-Hexahydro-6-hydroxy-3H-benzo[e]-indol-3-yl)pentyl]phthalimid, 0,68 g (0,007 Mol) Dimethylcarbamoylchlorid, 1,0 ml (0,007 Mol) Triethylamin und 0,91 g (0,007 Mol) 4-Dimethylaminopyridin in 100 ml Toluol wurde 4 Stunden unter Rückfluss gekocht und dann auf wässrige Natriumhydrogencarbonat-Lösung gegossen. Man extrahierte mit Toluol, trocknete die Extrakte mit Natriumsulfat und destillierte das Lösungsmittel im Vakuum ab. Der Rückstand wurde über Kieselgel mit Essigester chromatographiert, und man erhielt 2,39 g (81%) rac-3-[5-(1,3-Dioxo-isoindolin-2-yl)pentyl]-2,3,3a,4,5,9b-hexahydro-1H-cis-benzo[e]indol-6-yl-dimethylcarbamat als gelbes Oel. 0,6 g dieses Oels wurden mit ethanolischer HCl in das entsprechende Hydrochlorid überführt.
MS: m/e (% Basis peak) = 475 ($M^+$, 7), 273(100), 160(7), 72(50).

Beispiel 29

a) Eine Lösung von 8,55 g (0,02 Mol) rac-cis-N-[4-(1,2,3a,4,5,9b-Hexahydro-6-methoxy-3H-benzo[e]indol-3-yl)butyl]phthalimid in 250 ml Essigsäure wurde mit 3,88 g (0,06 Mol) Zinkpulver versetzt. Man kochte 1,25 Stunden unter Rückfluss und destillierte dann die Essigsäure ab. Der Rückstand wurde in Essigester aufgenommen, worauf mit Wasser und gesättigter Kochsalzlösung extrahiert wurde. Die organische Phase wurde mit Natriumsulfat getrocknet und eingeengt, worauf der Rückstand über Kieselgel mit Essigester/Methanol 9:1 chromatographiert wurde. Man erhielt 1,19 g (14%) rac-2-[4-(6-Methoxy-2,3,3a,4,5,9b-hexahydro-1H-cis-benzo[e]indol-3-yl)butyl]isoindolin-1-on als gelben Schaum.
MS: m/e (% Basis peak) = 390 ($M^+$, 12), 216(100), 202(19), 146(14), 91(10).

b) Eine Lösung aus 1,14 g (0,003 Mol) rac-2-[4-(6-Methoxy-2,3,3a,4,5,9b-hexahydro-1H-cis-benzo[e]-indol-3-yl)butyl]isoindolin-1-on in 30 ml Methylenchlorid wurde bei 0° tropfenweise mit einer Lösung von 1,4 ml (0,015 Mol) Bortribromid in 10 ml Methylenchlorid versetzt, worauf 1,5 Stunden bei 0° gerührt wurde. Unter Kühlen mit einem Eisbad wurde eine Lösung aus 1,65 g (0,04 Mol) Natriumhydroxid in 20 ml Wasser zugetropft, worauf weitere 30 Minuten gerührt wurde. Dann wurde das Gemisch auf wässrige Natriumbicarbonat-Lösung gegossen und mit Methylenchlorid extrahiert. Die organische Phase wurde mit Natriumsulfat getrocknet und eingeengt. Chromatographie des Rückstands über Kieselgel mit $CH_2Cl_2$/MeOH 9:1 lieferte 0,61 g (55%) rac-3-[4-(1-Oxo-isoindolin-2-yl)butyl]-2,3,3a,4,5,9b-hexahydro-1H-cis-benzo[e]indol-6-ol als weissen Schaum. Davon wurden 0,10 g mit 0,5 ml 8,6 molarer ethanolischer Salzsäure versetzt und eingeengt. Der Rückstand wurde in Methylenchlorid gelöst. Nach Trocknen mit $Na_2SO_4$ und Einengen erhielt man 0,08 g Hydrochlorid als weissen Schaum.
MS: m/e (% Basis peak) = 376 ($M^+$, 8), 202(100), 188(18), 171(6), 146(12).

c) Ein Gemisch aus 0,50 g (0,0013 Mol) rac-3-[4-(1-Oxo-isoindolin-2-yl)-butyl]-2,3,3a,4,5,9b-hexahydro-1H-cis-benzo[e]indol-6-ol, 0,15 ml (0,0016 Mol) Dimethylcarbamoylchlorid, 0,49 ml (0,0035 Mol) Triethylamin und 0,20 g (0,0016 Mol) 4-Dimethylaminopyridin in 30 ml Toluol wurde 2 Stunden unter Rückfluss gekocht. Man destillierte das Lösungsmittel im Vakuum ab, löste den Rückstand in Methylenchlorid und extrahierte mit Wasser und gesättigter wässriger Kochsalzlösung. Nach Trocknen mit Natriumsulfat, Einengen und Chromatographieren über Kieselgel mit Methylenchlorid/Methanol 9:1 erhielt man 560 mg gelbes Oel, das mit 2 ml 8,6 molarer ethanolischer Salzsäure versetzt wurde. Man engte ein, löste den Rückstand in Methylenchlorid, trocknete mit Natriumsulfat, filtrierte und destillierte das Lösungsmittel ab. Es wurden 455 mg (71%) rac-3-[4-(1-Oxo-indolin-2-yl)-butyl]-2,3,3a,4,5,9b-hexahydro-1H-cis-benzo[e]-indol-6-yl-dimethylcarbamat-hydrochlorid als weisser Schaum erhalten.
MS: m/e (% Basis peak) = 447 ($M^+$, 16), 273(100), 259(14), 146(15), 72(75).

Beispiel 30

a) Eine Lösung von 3,10 g (0,007 Mol) rac-N-[5-(cis-1,2,3a,4,5,9b-Hexahydro-3H-benzo[e]indol-3-yl)-pentyl]phthalimid in 100 ml Eisessig wurde mit 1,0 g 10% Palladium/Kohle versetzt und bei 60° 6 Stunden bei 20 bar Wasserstoffdruck hydriert. Die Essigsäure wurde abdestilliert, und der Rückstand wurde in Methylenchlorid aufgenommen. Die Lösung wurde mit 2N Natronlauge gewaschen, mit Natriumsulfat getrocknet und eingeengt. Chromatographie über Kieselgel mit Essigester/Methanol 19:1 lieferte 0,80 g (27%) rac-cis-2-[5-(6-Methoxy-2,3,3a,4,5,9b-hexahydro-1H-benzo[e]indol-3-yl)-pentyl]-isoindolin-1-on als gelbes Oel [MS: m/e (% Basis peak) = 404 ($M^+$, 6), 216(100), 202(8), 185(6), 146(7)] sowie 1,64 g (55%) rac-2-[5-(6-Methoxy-2,3,3a,4,5,9b-hexahydro-1H-cis-benzo[e]indol-6-yl)pentyl]-octahydro-cis-isoindol-1,3-dion als gelbes Oel [MS: m/e (% Basis peak) = 424 ($M^+$, 7), 216 (100), 185-(5)].

b) Ein Gemisch aus 0,8 g (0,002 Mol) rac-cis-2-[5-(6-Methoxy-2,3,3a,4,5,9b-hexahydro-1H-benzo[e]indol-3-yl)pentyl]isoindolin-1-on und 8,0 g (0,07 Mol) Pyridinhydrochlorid wurde mit 3 Tropfen Dimethylformamid versetzt und 2 Stunden bei 180° gerührt. Nach dem Abkühlen goss man das Gemisch auf wässrige Natriumhydrogencarbonat-Lösung und extrahierte mit Essigester, dem ca. 1% Methanol zugesetzt worden war. Nach Trocknen mit Natriumsulfat, Einengen und Chromatographieren über Kieselgel mit Methylenchlorid/Methanol/Ammoniakwasser 110:8:1 erhielt man 0,5 g (65%) rac-2-[5-(6-Hydroxy-2,3,3a,4,5,9b-hexahydro-1H-cis-benzo[e]indol-3-yl)-pentyl]isoindolin-1-on. Davon wurden 120 mg ins Hydrochlorid überführt.
MS: m/e (% Basis peak) = 390 ($M^+$, 7), 202(100), 188(9), 146(19).

c) Ein Gemisch aus 0,33 g (0,0008 Mol) rac-2-[5-(6-Hydroxy-2,3,3a,4,5,9b-hexahydro-1H-cis-benzo[e]-indol-3-yl)pentyl]isoindolin-1-on, 0,09 ml (0,001 Mol) Dimethylcarbamoylchlorid, 0,14 ml (0,001 Mol) Triethylamin und 0,12 g (0,001 Mol) 4-Dimethylaminopyridin in 10 ml Toluol wurde 3 Stunden unter Rückfluss gekocht und dann auf wässrige Natriumhydrogencarbonat-Lösung gegossen, worauf mit Methylenchlorid extrahiert wurde. Nach Trocknen mit Natriumsulfat und Einengen wurde der Rückstand über Kieselgel mit Methylenchlorid/Methanol/Ammoniakwasser 110:8:1 chromatographiert. Das resultierende farblose Oel wurde mit ethanolischer HCl versetzt, worauf die Lösung mit Natriumsulfat getrocknet und eingeengt wurde. Man erhielt 0,3 g (71%) rac-3-[5-(1-Oxo-isoindolin-2-yl)pentyl]-2,3,3a,4,5,9b-hexahydro-1H-cis-benzo[e]indol-6-yl-dimethylcarbamat-hydrochlorid als weissen Schaum.
MS: m/e (% Basis peak) = 461 ($M^+$, 3), 389(2), 273(54), 146(19), 72(100).

Beispiel 31

a) Ein Gemisch aus 1,50 g (0,0035 Mol) rac-2-[5-(6-Methoxy-2,3,3a,4,5,9b-hexahydro-1H-cis-benzo[e]-indol-6-yl)pentyl]octahydro-cis-isoindol-1,3-dion und 15,0 g (0,13 Mol) Pyridinhydrochlorid wurde mit 6 Tropfen Dimethylformamid versetzt und 2 Stunden bei 180° gerührt. Nach dem Abkühlen goss man das Gemisch auf wässrige Natriumhydrogencarbonat-Lösung und extrahierte mit Methylenchlorid. Nach Trocknen mit Natriumsulfat, Einengen und Chromatographieren über Kieselgel mit Methylenchlorid/Methanol/Ammoniakwasser 110:8:1 erhielt man 0,66 g (69%) rac-2-[5-(6-Hydroxy-2,3,3a,4,5,9b-hexahydro-1H-cis-benzo[e]indol-3-yl)pentyl]octahydro-cis-isoindol-1,3-dion. Eine kleine Probe wurde in das Hydrochlorid überführt.
MS: m/e (% Basis peak) = 410 ($M^+$, 8), 202(100), 171(6), 81(10).
b) Ein Gemisch aus 0,50 g (0,001 Mol) rac-2-[5-(6-Hydroxy-2,3,3a,4,5,9b-hexahydro-1H-cis-benzo[e]-indol-3-yl)pentyl]octahydro-cis-isoindol-1,3-dion, 0,14 ml (0,0015 Mol) Dimethylcarbamoylchlorid, 0,2 ml (0,0015 Mol) Triethylamin und 0,18 g (0,0015 Mol) 4-Dimethylaminopyridin in 20 ml Toluol wurde 3 Stunden unter Rückfluss gekocht und dann auf wässrige Natriumhydrogencarbonat-Lösung gegossen, worauf mit Methylenchlorid extrahiert wurde. Nach Trocknen und Einengen wurde der Rückstand über Kieselgel mit Essigester/Methanol 9:1 chromatographiert. Das erhaltene farblose Oel wurde mit ethanolischer HCl versetzt, und die Lösung wurde mit Natriumsulfat getrocknet und eingeengt. Man erhielt 0,5 g (85%) rac-3-[5-(1,3-Dioxo-octahydro-cis-isoindol-2-yl)pentyl]-2,3,3a,4,5,9b-hexahydro-1H-cis-benzo[e]-indol-6-yl-dimethylcarbamat-hydrochlorid als weissen Schaum.
MS: m/e (% Basis peak) = 481 ($M^+$, 8), 273(100), 200(5), 72(90).

Beispiel 32

a) Eine Lösung von 9,72 g (0,024 Mol) rac-cis-N-[6-(1,2,3a,4,5,9b-Hexahydro-6-methoxy-3H-benzo[e]-indol-3-yl)hexyl]phthalimid in 200 ml Essigsäure wurde mit 4,70 g (0,072 Mol) Zinkpulver versetzt. Man kochte 1,25 Stunden unter Rückfluss und destillierte dann die Essigsäure ab. Der Rückstand wurde in Essigester aufgenommen und mit Wasser und gesättigter Kochsalzlösung extrahiert. Die organische Phase wurde mit Natriumsulfat getrocknet und eingeengt, worauf der Rückstand über Kieselgel mit Essigester/Methanol 9:1 chromatographiert wurde. Man erhielt 0,98 g (10%) rac-2-[6-(6-Methoxy-2,3,3a,4,5,9b-hexahydro-1H-cis-benz-[e]indol-3-yl)hexyl]isoindolin-1-on als gelbes Oel.
MS: m/e (% Basis peak) = 418 ($M^+$, 10), 216(100), 146(20).
b) Eine Lösung aus 0,92 g (0,002 Mol) rac-2-[6-(6-Methoxy-2,3,3a,4,5,9b-hexahydro-1H-cis-benzo[e]-indol-3-yl)hexyl]isoindolin-1-on in 30 ml Methylenchlorid wurde bei 0° tropfenweise mit einer Lösung von 1,06 ml (0,012 Mol) Bortribromid in 10 ml Methylenchlorid versetzt, worauf 2 Stunden bei 0° gerührt wurde. Unter Kühlen mit einem Eisbad wurde eine Lösung aus 1,23 g (0,03 Mol) Natriumhydroxid in 20 ml Wasser zugetropft, worauf weitere 30 Minuten gerührt wurde. Dann wurde das Gemisch auf wässrige Natriumhydrogencarbonat-Lösung gegossen und mit Methylenchlorid extrahiert. Trocknen mit Natriumsulfat, Einengen und Chromatographieren über Kieselgel mit Methylenchlorid/Methanol 14:1 lieferte 0,41 g (46%) rac-3-[6-(1-Oxo-isoindolin-2-yl)hexyl]-2,3,3a,4,5,9b-hexahydro-1H-cis-benzo[e]indol-6-ol als weissen Schaum.
MS: m/e (% Basis peak) = 404 ($M^+$, 7), 202(100), 121(41).
c) Ein Gemisch aus 0,41 g (0,001 Mol) rac-3-[6-(1-Oxo-isoindolin-2-yl)-hexyl]-2,3,3a,4,5,9b-hexahydro-1H-cis-benzo[e]indol-6-ol, 0,11 ml (0,0012 Mol) Dimethylcarbamoylchlorid, 0,31 ml (0,0022 Mol) Triethylamin und 0,15 g (0,0012 Mol) 4-Dimethylaminopyridin in 50 ml Toluol wurde 1,5 Stunden unter Rückfluss gekocht. Man destillierte das Lösungsmittel im Vakuum ab, löste den Rückstand in Methylenchlorid und extrahierte mit Wasser und gesättigter wässriger Kochsalzlösung. Nach Trocknen mit Natriumsulfat, Einengen und Chromatographieren über Kieselgel mit Methylenchlorid/Methanol 19:1 erhielt man 0,39 g Rohprodukt, das mit 0,18 ml 8,6 molarer ethanolischer Salzsäure versetzt wurde. Man engte die Lösung ein, löste den Rückstand in Methylenchlorid, behandelte die Lösung mit Natriumsulfat und Aktivkohle, filtrierte und destillierte das Lösungsmittel ab. Dabei wurden 0,3 g (58%) rac-3-[6-(1-Oxo-isoindolin-2-yl)-hexyl]-2,3,3a,4,5,9b-hexahydro-1H-cis-benzo[e]indol-6-yl-dimethylcarbamat-hydrochlorid als beiger Schaum erhalten. MS: m/e (% Basis peak) = 475 ($M^+$, 7), 274(18), 273(100), 146(10), 72(54).

Beispiel 33

a) Eine Lösung aus 5,00 g (0,025 Mol) rac-cis-2,3,3a,4,5,9b-Hexahydro-6-methoxy-1H-benzo[e]indol, 4,5 g (0,025 Mol) $\gamma$-Chlorbutyrophenon und 6,9 ml (0,05 Mol) Triethylamin in 150 ml Toluol wurde 2 Tage

unter Rückfluss gekocht. Filtrieren, Einengen und Chromatographieren über Kieselgel mit Essigester/Hexan 1:1 ergab 3,18 g (37%) rac-4-(6-Methoxy-2,3,3a,4,5,9b-hexahydro-1H-cis-benzo[e]indol-3-yl)-1-phenylbutan-1-on als braunes Oel. Eine Probe wurde mit ethanolischer Salzsäure ins Hydrochlorid überführt. Smp. des Hydrochlorids 178-182°.

MS: m/e (% Basis peak) = 349 ($M^+$, 8), 229(34), 216(100), 214(19), 185(8), 159 (8), 147(8), 105(19), 77-(17), 71(14).

b) Eine Lösung von 2,41 g (0,007 Mol) rac-4-(6-Methoxy-2,3,3a,4,5,9b-hexahydro-1H-cis-benzo[e]indol-3-yl)-2-phenylbutan-1-on in 100 ml 48%iger Bromwasserstoffsäure wurde 2 Stunden unter Rückfluss gekocht. Nach Einengen wurde der Rückstand in Methylenchlorid und Wasser aufgenommen. Die Wasserphase wurde mit 2N Natronlauge versetzt, bis ein pH-Wert von 14 erreicht war. Dann wurde einmal mit gesättigter wässriger Ammoniumchloridlösung und einmal mit gesättigter wässriger Natriumhydrogencarbonat-Lösung extrahiert. Nach Trocknen der organischen Phase mit Natriumsulfat und Einengen wurde der Rückstand mit Essigester über Kieselgel chromatographiert. Den erhaltenen Feststoff löste man in einem Gemisch von Ethanol und Methylenchlorid, versetzte mit 0,8 ml 8,8M wässriger Bromwasserstoffsäure und destillierte die Lösungsmittel ab. Man erhielt 21 g (73%) rac-4-(6-Hydroxy-2,3,3a,4,5,9b-hexahydro-1H-cis-benzo[e]indol-3-yl)-1-phenylbutanon-hydrobromid als braune Kristalle. Eine Probe wurde aus Ethanol/Ether umkristallisiert und zeigte einen Smp. von 217-220°.

MS: m/e (% Basis peak) = 335 ($M^+$, 7), 215(41), 202(100), 171(8), 145(9), 105 (16).

c) Ein Gemisch aus 1,00 g (0,003 Mol) rac-4-(6-Hydroxy-2,3,3a,4,5,9b-hexahydro-1H-cis-benzo[e]indol-3-yl)-1-phenylbutanon-hydrobromid, 0,33 ml (0,0036 Mol) Dimethylcarbamoylchlorid, 1,0 ml (0,007 Mol) Triethylamin und 0,44 g (0,0036 Mol) 4-Dimethylaminopyridin in 30 ml Toluol wurde 3 Stunden unter Rückfluss gekocht. Man destillierte das Lösungsmittel im Vakuum ab, löste den Rückstand in Methylenchlorid und extrahierte die Lösung mit Wasser und gesättigter wässriger Kochsalzlösung. Nach Trocknen der organischen Phase mit Natriumsulfat, Einengen und Chromatographieren über Kieselgel mit Essigester/Methanol 19:1 erhielt man 0,92 g gelbes Oel, das in Ethanol gelöst wurde. Man versetzte die Lösung mit 0,36 ml 8,8M wässriger Bromwasserstoffsäure und engte ein. Der Rückstand wurde in heissem Ethanol gelöst und mit Aktivkohle behandelt. Nach Heissfiltration destillierte man das Lösungsmittel ab und suspendierte den Rückstand in Essigester. Nach Filtrieren erhielt man 0,87 g (74%) rac-3-(4-Oxo-4-phenylbutyl)-2,3,3a,4,5,9b-hexahydro-1H-cis-benzo[e]indol-6-yl-dimethylcarbamat-hydrobromid als beige Kristalle mit Smp. 183-186°.

MS: m/e (% Basis peak) = 406 ($M^+$, 4), 286(33), 273(100), 147(14), 105(21), 72 (91).

## Beispiel 34

a) Eine Lösung aus 8,00 g (0,039 Mol) rac-cis-2,3,3a,4,5,9b-Hexahydro-6-methoxy-1H-benzo[e]indol, 9,49 g (0,039 Mol) 5-Brom-1-phenylpentan-1-on und 10,9 ml (0,079 Mol) Triethylamin in 240 ml Toluol wurden 2 Tage unter Rückfluss gekocht. Das Lösungsmittel wurde im Vakuum abdestilliert, der Rückstand wurde in Methylenchlorid aufgenommen, und die Lösung wurde mit Wasser und gesättigter Kochsalzlösung extrahiert. Trocknen der organischen Phase über Natriumsulfat, Einengen und Chromatographieren über Kieselgel mit Essigester ergab 9,7 g (68%) rac-5-(6-Methoxy-2,3,3a,4,5,9b-hexahydro-1H-cis-benzo[e]-indol-3-yl)-1-phenylpentan-1-on als braunes Oel. Eine Probe wurde mit ethanolischer Salzsäure ins Hydrochlorid überführt. Smp. des Hydrochlorids 145-150°.

MS (Hydrochlorid): m/e (% Basis peak) = 363 ($M^+$, 12), 216(100), 185(7), 159 (5), 105(9).

b) Eine Suspension von 9,50 g (0,026 Mol) rac-5-(6-Methoxy-2,3,3a,4,5,9b-hexahydro-1H-cis-benzo[e]-indol-3-yl)-1-phenylpentan-1-on in 200 ml 48%iger Bromwasserstoffsäure wurde 2 Stunden unter Rückfluss gekocht. Nach Einengen wurde der Rückstand in Methylenchlorid und Wasser aufgenommen. Die Wasserphase wurde mit 2N Natronlauge versetzt, bis ein pH-Wert von 14 erreicht war. Dann wurde mit gesättigter wässriger Ammoniumchloridlösung extrahiert. Trocknen der organischen Phase mit Natriumsulfat, Einengen und Chromatographieren über Kieselgel mit Essigester lieferte 8,43 g (92%) rac-3-(5-Oxo-5-phenylpentyl)-2,3,3a,4,5,9b-hexahydro-1H-cis-benzo[e]indol-6-ol als braune Kristalle. Eine Probe wurde mit 8,8N wässriger Bromwasserstoffsäure ins Hydrobromid überführt. Smp. des Hydrobromids: 261-264°.

MS (Hydrobromid): m/e (% Basis peak) = 349 ($M^+$, 9), 202(100), 171(5), 145(7), 105(13), 77(8).

c) Ein Gemisch aus 4,0 g (0,011 Mol) rac-3-(5-Oxo-5-phenylpentyl)-2,3,3a,4,5,9b-hexahydro-1H-cis-benzo[e]indol-6-ol, 1,3 ml (0,014 Mol) Dimethylcarbamoylchlorid, 1,9 ml (0,014 Mol) Triethylamin und 1,68 g (0,014 Mol) 4-Dimethylaminopyridin in 120 ml Toluol wurde 3 Stunden unter Rückfluss gekocht. Man destillierte das Lösungsmittel im Vakuum ab, löste den Rückstand in Methylenchlorid und extrahierte die Lösung mit Wasser und gesättigter wässriger Kochsalzlösung. Nach Trocknen der organischen

Phase mit Natriumsulfat, Einengen und Chromatographieren über Kieselgel mit Essigester/Methanol 19:1 erhielt man 3,9 g gelbes Oel, das in Ethanol gelöst wurde. Man versetzte die Lösung mit 1,8 ml 8,6M ethanolischer HCl, behandelte mit Aktivkohle und engte ein. Der Rückstand wurde in Essigester suspendiert und filtriert. Man erhielt 3,64 g (70%) rac-3-(5-Oxo-5-phenylpentyl)-2,3,3a,4,5,9b-hexahydro-1H-cis-benz[e]indol-6-yl-dimethylcarbamat-hydrochlorid als amorphes Pulver.
MS: m/e (% Basis peak) = 420 ($M^+$, 8), 273(100), 200(3), 105(17), 77(11), 72 (70).

Beispiel 35

a) Eine Lösung von 10,0 g (0,049 Mol) rac-cis-2,3,3a,4,5,9b-Hexahydro-6-methoxy-1H-benzo[e]indol in 250 ml 48%iger Bromwasserstoffsäure wurde 2 Stunden unter Rückfluss gekocht. Man destillierte die Bromwasserstoffsäure ab, löste den Rückstand in einem Gemisch aus 30 ml Methanol, 500 ml Methylenchlorid und ca. 200 ml Wasser und versetzte die Lösung mit 2N Natronlauge, bis ein pH-Wert von 10-11 erreicht war. Anschliessend gab man gesättigte wässrige Ammoniumchloridlösung und Salzsäure zu, bis ein pH-Wert von 7,5 erreicht war. Man extrahierte zweimal mit je 200 ml einer 20%igen Lösung von Methanol in Methylenchlorid und viermal mit je 150 ml Chloroform. Nach Trocknen der Extrakte mit Natriumsulfat und Einengen löste man den Rückstand in 200 ml Methanol und fällte das Produkt durch Zugabe von Ether aus. Filtrieren ergab 9,57 g rac-2,3,3a,4,5,9b-Hexahydro-1H-cis-benzo-[e]indol-6-ol (teilweise als Hydrochlorid).
MS: m/e (% Basis peak) = 189 ($M^+$, 100), 172(16), 145(35), 131(15), 115(15), 68 (15), 56 (48).
b) Eine Suspension aus 3,0 g (0,016 Mol) rac-2,3,3a,4,5,9b-Hexahydro-1H-cis-benzo[e]indol-6-ol, 5,2 g (0,017 Mol) 5-Brom-3',4'-dimethoxyvalerophenon (erhältlich in Analogie zu dem in EP-A2-310126 beschriebenen Verfahren) und 2,4 ml (0,017 Mol) Triethylamin in 150 ml Toluol wurde 22 Stunden unter Rückfluss gekocht. Nach Abkühlen goss man das Gemisch auf wässrige Natriumhydrogencarbonat-Lösung und extrahierte mit Essigester. Chromatographieren über Kieselgel mit Methylenchlorid/Methanol 20:1 ergab 0,94 g (15%) rac-5-(6-Hydroxy-2,3,3a,4,5,9b-hexahydro-1H-cis-benzo[e]indol-3-yl)-1-(3,4-dimethoxyphenyl)-pentan-1-on als Feststoff. Eine kleine Probe wurde mit ethanolischer HCl ins Hydrochlorid überführt; Smp. des Hydrochlorids 230-232°.
MS: m/e (% Basis peak) = 409 ($M^+$, 12), 202(100), 188(5), 165(5), 145(5).
c) Ein Gemisch aus 0,70 g (0,0017 Mol) rac-5-(6-Hydroxy-2,3,3a,4,5,9b-hexahydro-1H-cis-benzo[e]indol-3-yl)-1-(3,4-dimethoxyphenyl)-pentan-1-on, 0,19 ml (0,002 Mol) Dimethylcarbamoylchlorid, 0,28 ml (0,002 Mol) Triethylamin und 0,25 g (0,002 Mol) 4-Dimethylaminopyridin in 40 ml Toluol wurde 21 Stunden unter Rückfluss gekocht und dann auf wässrige Natriumhydrogencarbonat-Lösung gegossen. Man extrahierte mit Essigester und chromatographierte über Kieselgel mit Methylenchlorid/Methanol 20:1. Das erhaltene Oel wurde in wenig Methanol gelöst. Nach Zugabe einer äquimolaren Menge ethanolischer HCl wurde mit Natriumsulfat getrocknet und eingeengt. Man erhielt 0,84 g (95%) rac-3-[5-(3,4-Dimethoxyphenyl)-5-oxo-pentyl]-2,3,3a,4,5,9b-hexahydro-1H-cis-benzo[e]indol-6-yl-dimethylcarbamat-hydrochlorid als amorphes Pulver.
MS: m/e (% Basis peak) = 480 ($M^+$, 8), 273(100), 259(5), 221(5), 200(5), 165 (11), 72(70).

Beispiel 36

a) Eine Suspension von 3,10 g (0,014 Mol) rac-2,3,3a,4,5,9b-Hexahydro-1H-cis-benzo[e]indol-6-ol, 4,80 g (0,015 Mol) 6-Brom-(3,4-dimethoxyphenyl)hexan-1-on (erhältlich gemäss EP-A2-310.126) und 3,8 ml (0,027 Mol) Triethylamin in 150 ml Toluol wurde 16 Stunden unter Rückfluss gekocht und nach Abkühlen auf wässrige Natriumhydrogencarbonat-Lösung gegossen, worauf mit Essigester extrahiert wurde. Chromatographie des beim Eindampfen des Extrakts verbleibenden Rückstands über Kieselgel mit Methylenchlorid/Methanol 20:1 ergab 1,6 g (17%) rac-6-(6-Hydroxy-2,3,3a,4,5,9b-hexahydro-1H-cis-benzo[e]indol-3-yl)-1-(3,4-dimethoxyphenyl)hexan-1-on als braunen Feststoff. Eine kleine Probe wurde mit ethanolischer HCl ins Hydrochlorid überführt; Smp. 165-168°.
MS: m/e (% Basis peak) = 423 ($M^+$, 17), 202(100), 165(8), 145(5).
b) Ein Gemisch aus 1,30 g (0,003 Mol) rac-6-(6-Hydroxy-2,3,3a,4,5,9b-hexahydro-1H-cis-benzo[e]indol-3-yl)-1-(3,4-dimethoxyphenyl)hexan-1-on, 0,34 ml (0,0037 Mol) Dimethylcarbamoylchlorid, 0,52 ml (0,0037 Mol) Triethylamin und 0,45 g (0,0037 Mol) 4-Dimethylaminopyridin in 80 ml Toluol wurde 21 Stunden unter Rückfluss gekocht und dann auf wässrige Natriumhydrogencarbonat-Lösung gegossen, worauf mit Essigester extrahiert wurde. Chromatographie des beim Eindampfen des Extrakts verbleibenden Rückstands über Kieselgel mit Methylenchlorid/Methanol 20:1 ergab ein Oel, welches in 20 ml Ethanol gelöst und mit einer äquimolaren Menge ethanolischer HCl versetzt wurde. Nach Einengen wurde der Rückstand in Methylenchlorid gelöst. Man behandelte die Lösung mit Natriumsulfat und Aktivkohle, filtrierte und engte das Filtrat ein. Der Rückstand wurde in Ether suspendiert, worauf die Suspension filtriert wurde. Man erhielt 0,92 g (56%) rac-3-[6-(3,4-Dimethoxyphenyl)-6-oxohexyl]-2,3,3a,4,5,9b-hexahydro-1H-cis-benzo[e]indol-yl-dimethylcarbamat-hydrochlorid als amorphes Pulver.
MS: m/e (% Basis peak) = 494 ($M^+$, 10), 273(100), 165(12), 72(72).

Beispiel 37

a) Eine Suspension aus 2,03 g (0,01 Mol) rac-cis-2,3,3a,4,5,9b-Hexahydro-6-methoxy-1H-benzo[e]indol, 2,76 g (0,02 Mol) Kaliumcarbonat, 0,2 g (0,001 Mol) Natriumjodid und 4,00 g (0,02 Mol) $\gamma$-Chlor-p-fluorbutyrophenon in 40 ml Ethylmethylketon wurde 18 Stunden unter Rückfluss gekocht und dann auf Wasser gegossen, worauf bei pH 8-9 mit Essigester extrahiert wurde. Chromatographie des beim Eindampfen des Extrakts verbleibenden Rückstands über Kieselgel mit Essigester/Hexan 2:1 lieferte 3,35 g (91%) rac-1-(4-Fluorphenyl)-4-(6-methoxy-2,3,3a,4,5,9b-hexahydro-1H-cis-benzo-[e]indol-3-yl)butan-1-on als farbloses Oel.
MS: m/e (% Basis peak) = 367 ($M^+$, 9), 229(32), 216(100), 185(7), 165(7), 159 (7), 123(18).
b) 3,3 g (0,009 Mol) rac-1-(4-Fluorphenyl)-4-(6-methoxy-2,3,3a,4,5,9b-hexahydro-1H-cis-benzo[e]indol-3-yl)butan-1-on in 200 ml 48%iger wässriger Bromwasserstoffsäure wurden 1 Stunde unter Rückfluss gekocht. Nach Einengen wurde auf Wasser gegossen und bei pH 8-9 mit Methylenchlorid extrahiert. Chromatographie des beim Eindampfen des Extrakts verbleibenden Rückstands über Kieselgel mit Methylenchlorid/Methanol 20:1 lieferte 2,45 g (77%) rac-1-(4-Fluorphenyl)-4-(6-hydroxy-2,3,3a,4,5,9b-hexahydro-1H-cis-benzo[e]indol-3-yl)butan-1-on als grauen Feststoff. Eine kleine Probe wurde mit einer äquimolaren Menge ethanolischer HCl ins Hydrochlorid überführt; Smp. 232-234°.
MS: m/e (% Basis peak) = 353 ($M^+$, 8), 215(37), 202(100), 165(8), 145(11), 123 (27), 95(17).
c) Ein Gemisch aus 2,00 g (0,006 Mol) rac-1-(4-Fluorphenyl)-4-(6-hydroxy-2,3,3a,4,5,9b-hexahydro-1H-cis-benzo[e]indol-3-yl)butan-1-on, 0,64 ml (0,007 Mol) Dimethylcarbamoylchlorid, 1,0 ml (0,007 Mol) Triethylamin und 0,85 g (0,007 Mol) 4-Dimethylaminopyridin in 100 ml Toluol wurde 5 Stunden unter Rückfluss gekocht und dann auf wässrige Natriumhydrogencarbonat-Lösung gegossen, worauf mit Essigester extrahiert wurde. Chromatographie des beim Eindampfen des Extrakts verbleibenden Rückstands über Kieselgel mit Methylenchlorid/Methanol 40:1 lieferte 2,14 g Feststoff. Diesen löste man in 15 ml Essigester, versetzte mit 35 ml n-Hexan und kühlte im Kühlschrank. Nach Abfiltrieren und Trocknen erhielt man 1,59 g (66%) rac-3-[4-(4-Fluorphenyl)-4-oxobutyl]-2,3,3a,4,5,9b-hexahydro-1H-cis-benzo[e]-indol-6-yl-dimethylcarbamat als beige Kristalle mit Smp. 95-97°.
MS: m/e (% Basis peak) = 424 ($M^+$, 1), 286(9), 273(41), 165(5), 123(23), 95(16), 72(100).

Beispiel 38

Eine Lösung aus 6,40 g (0,014 Mol) rac-cis-2,3,3a,4,5,9b-Hexahydro-3-(4-phthalimidobutyl)-1H-benzo[e]-indol-6-yl-dimethylcarbamat und 7,7 ml (0,037 Mol) Hydrazinhydrat in 200 ml Ethanol wurde 2 Stunden unter Rückfluss gekocht und dann über Nacht bei Raumtemperatur gerührt. Nach Filtrieren destillierte man das Lösungsmittel ab und löste den Rückstand in Methylenchlorid. Nach Extrahieren mit Wasser und mit gesättigter wässriger Kochsalzlösung wurde die organische Phase mit Natriumsulfat getrocknet und eingeengt. Man erhielt 3,66 g(80%) rac-3-(4-Aminobutyl)-2,3,3a,4,5,9b-hexahydro-1H-cis-benzo[e]indol-6-yl-dimethylcarbamat als dunkles Oel. Davon löste man 0,2 g in Ethanol, versetzte mit 0,1 ml 8,6M ethanolischer HCl und engte ein. Der Rückstand wurde in Methylenchlorid gelöst und mit Aktivkohle und Natriumsulfat behandelt. Nach Filtrieren und Einengen löste man den Rückstand erneut in 2 ml Methylenchlorid und gab unter Rühren 20 ml Ether und dann 30 ml Hexan zu. Nach 1 Stunde Rühren wurde der Festkörper abfiltriert und getrocknet. Man erhielt 0,17 g rac-3-(4-Aminobutyl)-2,3,3a,4,5,9b-hexahydro-1H-cis-benzo[e]indol-6-yl-dimethylcarbamat-hydrochlorid als hygroskopischen Feststoff.
MS: m/e (% Basis peak) = 331 ($M^+$, 6), 273(74), 259(12), 216(11), 72(100).

Beispiel 39

Eine Lösung von 3,46 g (0,01 Mol) rac-3-(4-Aminobutyl)-2,3,3a,4,5,9b-hexahydro-1H-cis-benzo[e]indol-6-yl-dimethylcarbamat, 2,10 g (0,01 Mol) 4-(Methylsulfonyl)benzoesäure, 4,00 g (0,01 Mol) 2-(1H-Benzotriazol-1-yl)-1,1,3,3-tetramethyluronium-hexafluorophosphat und 2,9 ml (0,021 Mol) Triethylamin in 80 ml Dimethylformamid wurde 22 Stunden bei Raumtemperatur gerührt und dann auf wässrige Natriumhydrogencarbonat-Lösung gegossen, worauf mit Methylenchlorid extrahiert wurde. Nach Trocknen des Extrakts

mit Natriumsulfat und Einengen wurde der Rückstand dreimal über Kieselgel chromatographiert, und zwar zuerst mit Methylenchlorid/Methanol 9:1, dann mit Essigester/Methanol 4:1 und schliesslich mit Methylenchlorid/Methanol 20:1. Der erhaltene Feststoff wurde in Essigester/Methanol gelöst und mit einer äquimolaren Menge ethanolischer HCl versetzt. Die erhaltene Lösung wurde mit Natriumsulfat getrocknet und filtriert. Abdestillieren des Lösungsmittels ergab 2,10 g (37%) rac-cis-3-[4-(4-Methylsulfonyl-benzoylamino)-butyl]-2,3,3a,4,5,9b-hexahydro-1H-benzo[e]indol-6-yl-dimethylcarbamat-hydrochlorid als beigen Schaum.
MS: m/e (% Basis peak) = 513 ($M^+$, 7), 273(100), 259(5), 216(7), 183(12), 121 (5), 107(5), 72(65).

Beispiel 40

Eine Lösung aus 1,79 g (0,004 Mol) rac-3-[5-(1,3-Dioxo-isoindolin-2-yl)-pentyl]-2,3,3a,4,5,9b-hexahydro-1H-cis-benzo[e]indol-6-yl-dimethylcarbamat und 0,48 ml (0,01 Mol) Hydrazinhydrat in 35 ml Ethanol wurde 2 Stunden unter Rückfluss gekocht und dann über Nacht bei Raumtemperatur gerührt. Nach Filtrieren destillierte man das Lösungsmittel ab und löste den Rückstand in Methylenchlorid. Nach Extrahieren mit Wasser wurde die organische Phase mit Natriumsulfat getrocknet und eingeengt. Chromatographieren des Rückstands über Kieselgel mit Methylenchlorid/Methanol/Ammoniakwasser 90:10:1 lieferte 0,9 g (69%) rac-3-(5-Aminopentyl)-2,3,3a,4,5,9b-hexahydro-1H-cis-benzo[e]indol-6-yl-dimethylcarbamat als hellgelbes Oel. Eine kleine Probe wurde mit einer äquimolaren Menge ethanolischer HCl ins Bis-hydrochlorid überführt.
MS: m/e (% Basis peak) = 345 ($M^+$, 6), 273(100), 259(14), 84(12), 72(73).

Beispiel 41

Eine Lösung von 0,60 g (0,002 Mol) rac-3-(5-Aminopentyl)-2,3,3a,4,5,9b-hexahydro-1H-cis-benzo[e]-indol-6-yl-dimethylcarbamat, 0,35 g (0,002 Mol) 4-(Methylsulfonyl)-benzoesäure, 0,66 g (0,002 Mol) 2-(1H-Benzotriazol-1-yl)-1,1,3,3-tetramethyluronium-hexafluorophosphat und 0,48 ml (0,003 Mol) Triethylamin in 10 ml Dimethylformamid wurde über Nacht bei Raumtemperatur gerührt und dann auf wässrige Natriumhydrogencarbonat-Lösung gegossen, worauf mit Methylenchlorid extrahiert wurde. Nach Trocknen der organischen Phase mit Natriumsulfat und Einengen wurde der Rückstand zweimal über Kieselgel chromatographiert, und zwar zuerst mit Methylenchlorid/Methanol/Ammoniakwasser 105:8:1 und dann mit Essigester/Methanol 4:1. Das erhaltene Oel (0,6 g) wurde mit einer äquimolaren Menge ethanolischer HCl ins Hydrochlorid überführt. Man erhielt 0,57 g (58%) rac-cis-3-[5-(4-Methansulfonylbenzoylamino)pentyl]-2,3,3a,4,5,9b-hexahydro-1H-benzo[e]indol-6-yl-dimethylcarbamat-hydrochlorid als weisses, amorphes Pulver.
MS: m/e (% Basis peak) = 527 ($M^+$, 1), 273(44), 200(7), 183(12), 121(9), 104(5), 72(100).

Beispiel 42

Eine Lösung aus 6,60 g (0,014 Mol) rac-cis-2,3,3a,4,5,9b-Hexahydro-3-(6-phthalimidohexyl)-1H-benzo-[e]indol-6-yl-dimethylcarbamat und 7 ml (0,035 Mol) Hydrazinhydrat in 200 ml Ethanol wurde 4 Stunden unter Rückfluss gekocht und über das Wochenende bei Raumtemperatur gerührt. Nach Filtrieren destillierte man das Lösungsmittel ab und löste den Rückstand in Methylenchlorid. Man extrahierte mit Wasser, trocknete die organische Phase mit Natriumsulfat, filtrierte und engte das Filtrat ein. Von den erhaltenen 4,57 g Oel wurden 0,5 g über Kieselgel mit Methylenchlorid/Methanol/Ammoniakwasser 90:10:1 chromatographiert. Das erhaltene Oel wurde in Ethanol gelöst und mit ethanolischer HCl versetzt. Abdestillieren des Lösungsmittels lieferte 0,4 g rac-3-(6-Aminohexyl)-2,3,3a,4,5,9b-hexahydro-1H-cis-benzo[e]indol-6-yl-dimethylcarbamat-bishydrochlorid als beigen amorphen Feststoff.
MS: m/e (% Basis peak) = 359 ($M^+$, 4), 273(100), 259(8), 72(82).

Beispiel 43

Eine Lösung von 2,5 g (0,007 Mol) rac-3-(6-Aminohexyl)-2,3,3a,4,5,9b-hexahydro-1H-cis-benzo[e]indol-6-yl-dimethylcarbamat (Rohprodukt), 1,40 g (0,007 Mol) 4-(Methylsulfonyl)benzoesäure, 2,65 g (0,007 Mol) 2-(1H-Benzotriazol-1-yl)-1,1,3,3-tetramethyluronium-hexafluorophosphat und 1,40 g (0,014 Mol) Triethylamin in 50 ml Dimethylformamid wurde 20 Stunden bei Raumtemperatur gerührt und dann auf wässrige Natriumhydrogencarbonat-Lösung gegossen, worauf mit Methylenchlorid extrahiert wurde. Nach Trocknen mit Natriumsulfat und Einengen wurde zweimal über Kieselgel chromatographiert, und zwar zuerst mit Methylenchlorid/Methanol 20:1 und dann mit Essigester/Methanol 4:1. Die resultierenden 1,8 g Feststoff wurden in Methylenchlorid gelöst, worauf 0,5 ml 8,2N ethanolische HCl zugegeben wurden. Nach Trocknen

über Natriumsulfat, Filtrieren und Einengen erhielt man 1,96 g (49%) rac-3-[6-(4-Methylsulfonyl-benzoylamino)-hexyl]-2,3,3a,4,5,9b-hexahydro-1H-cis-benzo[e]indol-6-yl-dimethylcarbamat als amorphen Feststoff.
MS: m/e (% Basis peak) = 541 ($M^+$, 3), 448(7), 273(100), 183(13), 121(6), 107 (8), 72(90).

Beispiel 44

a) Ein Gemisch aus 30,0 g (0,07 Mol) rac-cis-N-[4-(1,2,3a,4,5,9b-Hexahydro-6-methoxy-3H-benzo[e]indol-3-yl)butyl]phthalimid und 120 g (1,0 Mol) Pyridinhydrochlorid wurde mit 10 Tropfen Dimethylformamid versetzt und 18 Stunden bei 180° gerührt. Nach Abkühlen wurde das Gemisch in wässriger Natriumhydrogencarbonat-Lösung suspendiert und mit Methylenchlorid extrahiert. Der ungelöste Rückstand in der Wasserphase wurde abfiltriert und in Methanol, dem 4 Tropfen 6N Salzsäure zugesetzt worden waren, suspendiert. Der ungelöste Anteil wurde abfiltriert und in einem Gemisch Methylenchlorid/Methanol ca. 1:1 aufgenommen. Dann filtrierte man und engte das Filtrat ein. Das so erhaltene schwarze Oel (11,2 g) löste man in 100 ml Dimethylformamid und gab 36,3 ml (0,26 Mol) Triethylamin und 15,1 ml (0,13 Mol) Benzoylchlorid zu. Nach Rühren über Nacht bei Raumtemperatur versetzte man mit 2N Natronlauge, rührte 30 Minuten, gab 6N Salzsäure zu, bis ein pH-Wert von 1 erreicht war, und stellte dann mit Natriumhydrogencarbonat einen pH-Wert von ca. 8 ein. Extrahieren mit Methylenchlorid lieferte 4,0 g schwarzes Oel, das über Kieselgel mit Methylenchlorid/Methanol 9:1 chromatographiert wurde. Man erhielt 1,38 g rac-N-[4-(6-Hydroxy-2,3,3a,4,5,9b-hexahydro-1H-cis-benzo[e]indol-3-yl)butyl]-benzamid als braunes Oel. Eine kleine Probe wurde mit einer äquimolaren Menge ethanolischer HCl ins Hydrochlorid überführt.
MS: m/e (% Basis peak) = 364 ($M^+$, 7), 202(100), 188(8), 171(7), 145(5), 105 (19).

b) Ein Gemisch aus 1,18 g (0,001 Mol) rac-N-[4-(6-Hydroxy-2,3,3a,4,5,9b-hexahydro-1H-cis-benzo[e]-indol-3-yl)butyl]benzamid, 0,36 ml (0,004 Mol) Dimethylcarbamoylchlorid, 0,54 ml (0,004 Mol) Triethylamin und 0,48 g (0,004 Mol) 4-Dimethylaminopyridin in 60 ml Toluol wurde 17 Stunden unter Rückfluss erhitzt und dann auf wässrige Natriumhydrogencarbonat-Lösung gegossen, worauf mit Essigester extrahiert wurde. Chromatographie über Kieselgel mit Methylenchlorid/Methanol 20:1 lieferte einen Feststoff, welcher in 30 ml Methylenchlorid gelöst und mit einer äquimolaren Menge ethanolischer HCl versetzt wurde. Nach Behandeln mit Aktivkohle und Natriumsulfat wurde filtriert und das Lösungsmittel abdestilliert. Man erhielt 0,83 g (54%) rac-cis-3-(4-Benzoylaminobutyl)-2,3,3a,4,5,9b-hexahydro-1H-benzo[e]indol-6-yl-dimethylcarbamat-hydrochlorid als beigen amorphen Feststoff.
MS: m/e (% Basis peak) = 435 ($M^+$, 7), 273(100), 259(8), 105(27), 72(61).

Beispiel 45

Zu einer Lösung von 0,15 g (0,36 mMol) rac-3-[5-Aminopentyl)-2,3,3a,4,5,9b-hexahydro-1H-cis-benzo-[e]indol-6-yl-dimethylcarbamat-bishydrochloridund 0,06 ml (0,43 mMol) Triethylamin in 10 ml Methylenchlorid tropfte man 0,05 ml (0,43 mMol) Benzoylchlorid, gelöst in 2 ml Methylenchlorid, und rührte 1 Stunde bei Raumtemperatur. Dann wurde das Lösungsmittel abdestilliert, und der Rückstand wurde über Kieselgel mit Methylenchlorid/Methanol/Ammoniakwasser 110:8:1 chromatographiert. Das erhaltene Oel wurde in Ethanol gelöst und mit ethanolischer HCl versetzt. Nach Einengen löste man den Rückstand in Methylenchlorid, trocknete die Lösung mit Natriumsulfat, filtrierte und destillierte das Lösungsmittel ab. Man erhielt 0,11 g (63%) rac-3-(5-Benzoylaminopentyl)-2,3,3a,4,5,9b-hexahydro-1H-cis-benzo[e]indol-6-yl-dimethylcarbamat-hydrochlorid als beigen amorphen Feststoff.
MS: m/e (% Basis peak) = 449 ($M^+$, 3), 273(66), 200(8), 105(44), 77(32), 72 (100).

Beispiel 46

Zu einer Lösung von 1,50 g (0,004 Mol) rac-3-(6-Aminohexyl)-2,3,3a,4,5,9b-hexahydro-1H-cis-benzo[e]-indol-6-yl-dimethylcarbamat und 0,7 ml (0,005 Mol) Triethylamin in 70 ml Methylenchlorid tropfte man 0,6 ml Benzoylchlorid, gelöst in 5 ml Methylenchlorid, und rührte 1 Stunde bei Raumtemperatur. Man goss das Gemisch auf wässrige Natriumhydrogencarbonat-Lösung,extrahierte mit Methylenchlorid und chromatographierte über Kieselgel mit Methylenchlorid/Methanol 20:1. Die erhaltenen 1,24 g Feststoff wurden in Methylenchlorid gelöst und mit einer äquimolaren Menge ethanolischer HCl versetzt. Nach Trocknen mit Natriumsulfat, Filtrieren und Abdestillieren des Lösungsmittels im Vakuum erhielt man 1,14 g (55%) rac-3-(6-Benzoylaminohexyl)-2,3,3a,4,5,9b-hexahydro-1H-cis-benzo[e]indol-6-yl-dimethylcarbamat-hydrochlorid als amorphen Schaum.
MS: m/e (% Basis peak) = 463 ($M^+$, 7), 273(100), 105(20), 72(48).

Beispiel 47

a) Ein Gemisch aus 10,0 g (0,049 Mol) rac-cis-2,3,3a,4,5,9b-Hexahydro-6-methoxy-1H-benzo[e]indol, 7,44 g (0,054 Mol) Kaliumcarbonat, 2,46 g (0,016 Mol) Natriumjodid und 6,1 ml (0,054 Mol) Benzylchlorid in 20 ml Ethylmethylketon wurde 24 Stunden unter Rückfluss gekocht und dann auf wässrige Natriumhydrogencarbonat-Lösung gegossen, worauf mit Essigester extrahiert wurde. Chromatographie über Kieselgel mit Methylenchlorid/Methanol 98:2 lieferte 8,77 g (60%) rac-3-Benzyl-6-methoxy-2,3,3a,4,5,9b-hexahydro-1H-cis-benzo[e]indol als farbloses Oel. Eine kleine Probe wurde mit ethanolischer HCl ins Hydrochlorid überführt; Smp. 203-206°.
MS: m/e (% Basis peak) = 293 ($M^+$, 82), 264(4), 216(15), 202(26), 173(15), 159 (15), 91(100).
b) Man kochte 8,55 g (0,029 Mol) rac-3-Benzyl-6-methoxy-2,3,3a,4,5,9b-hexahydro-1H-cis-benzo[e]indol in 100 ml 48%iger wässriger Bromwasserstoffsäure 1 Stunde lang unter Rückfluss und destillierte dann die Bromwasserstoffsäure im Vakuum ab. Der Rückstand wurde in 30 ml eiskaltem Wasser suspendiert, worauf filtriert wurde. Nach Trocknen des Filtrats erhielt man 9,89 g (94%) rac-3-Benzyl-2,3,3a,4,5,9b-hexahydro-1H-cis-benzo[e]indol-6-ol-hydrobromid als beige Kristalle mit Smp. 237-239°.
MS: m/e (% Basis peak) = 279 ($M^+$, 79), 202(17), 188(28), 159(19), 120(13), 91(100).
c) Ein Gemisch aus 5,25 g (0,015 Mol) rac-3-Benzyl-2,3,3a,4,5,9b-hexahydro-1H-cis-benzo[e]indol-6-ol-hydrobromid, 1,65 ml (0,018 Mol) Dimethylcarbamoylchlorid, 6,3 ml (0,045 Mol) Triethylamin und 2,20 g (0,018 Mol) 4-Dimethylaminopyridin in 250 ml Toluol wurde 17 Stunden unter Rückfluss gekocht und dann auf wässrige Natriumhydrogencarbonat-Lösung gegossen, worauf mit Essigester extrahiert wurde. Chromatographie über Kieselgel mit Essigester/Hexan 1:2 ergab ein Oel (4,5 g), welches man in 50 ml Methylenchlorid löste. Nach Zugabe von 1,6 ml 8,6M ethanolischer HCl, Behandeln mit Aktivkohle und Natriumsulfat und Filtrieren gab man 100 ml Essigester zu und engte auf ein Volumen von ca. 40 ml ein. Es kristallisierten 4,43 g (76%) rac-3-Benzyl-2,3,3a,4,5,9b-hexahydro-1H-cis-benzo[e]indol-6-yl-dimethylcarbamat-hydrochlorid mit Smp. 177-180°.
MS: m/e (% Basis peak) = 350 ($M^+$, 40), 273(7), 259(9), 132(16), 120(7), 91(76), 72(100).

Beispiel 48

Eine Lösung aus 0,60 g (0,0015 Mol) rac-N-[5-(cis-1,2,3a,4,5,9b-Hexahydro-6-hydroxy-3H-benzo[e]indol-3-yl)pentyl]phthalimid, 0,31 ml (0,002 Mol) Triethylamin und 0,23 ml (0,003 Mol) Ethylisocyanat wurde 4 Stunden unter Rückfluss erhitzt, dann eingeengt und auf Wasser gegossen, worauf mit Methylenchlorid extrahiert wurde. Das beim Eindampfen des Extrakts zurückbleibende Oel wurde über Aluminiumoxid (neutral) mit Methylenchlorid chromatographiert. Man erhielt 0,45 g farbloses Oel, das mit einer äquimolaren Menge ethanolischer Salzsäure ins Hydrochlorid überführt wurde. Man erhielt 0,35 g (46%) rac-3-[5-(1,3-Dioxoisoindolin-2-yl)pentyl]-2,3,3a,4,5,9b-hexahydro-1H-cis-benzo[e]indol-6-yl-ethylcarbamat-hydrochlorid als weisses amorphes Pulver.
MS: m/e (% Basis peak) = 475 ($M^+$, 2), 404(8), 273(28), 202(100), 160(9).

Beispiel 49

a) Man löste 4,0 g (0,01525 Mol) 1,2,3,4-Tetrahydro-5-methoxy-2-oxo-1-naphthylessigsäureethylester in 80 ml Toluol, fügte 3,0 ml (0,0305 Mol) Cyclopentylamin zu und kochte 40 Stunden am Wasserabscheider. Nach Einengen wurde der Rückstand mit 1,2 g Raney-Nickel in 500 ml Ethanol bei 120° und 140 Bar während 10 Stunden hydriert. Das Produkt wurde über Kieselgel mit Cyclohexan/Ether 1:1 chromatographiert. Man erhielt 2,0 g (46%) rac-cis-3-Cyclopentyl-1,3,3a,4,5,9b-hexahydro-6-methoxy-2H-benzo[e]-indol-2-on als weisse Kristalle mit Smp. 84-88°.
b) 0,53 g (0,014 Mol) Lithiumaluminiumhydrid wurden in 25 ml THF unter Argon suspendiert. Man tropfte eine Lösung aus 2,0 g (0,007 Mol) rac-cis-3-Cyclopentyl-1,3,3a,4,5,9b-hexahydro-6-methoxy-2H-benzo-[e]indol-2-on in 50 ml THF zu und kochte 1 Stunde unter Rückfluss. Vorsichtig tropfte man 10 ml einer gesättigten wässrigen $NH_4Cl$-Lösung zu und filtrierte ab. Man schüttelte das Filtrat mit Essigester aus, wusch die organische Phase mit Wasser, trocknete sie mit $MgSO_4$, filtrierte und engte das Filtrat ein. Man erhielt 1,8 g (95%) öliges rac-cis-3-Cyclopentyl-2,3,3a,4,5,9b-hexahydro-6-methoxy-1H-benzo[e]-indol.
c) 1,8 g (0,0066 Mol) rac-cis-3-Cyclopentyl-2,3,3a,4,5,9b-hexahydro-6-methoxy-1H-benzo[e]indol wurden in 0,2 l 48%iger wässriger HBr gelöst und 1 Stunde unter Rückfluss gekocht. Das Gemisch wurde abgekühlt und eingeengt, worauf man 100 ml einer wässrigen $NaHCO_3$-Lösung und wenig 2N NaOH-Lösung zugab, dreimal mit $CH_2Cl_2$ ausschüttelte und die Extrakte mit $MgSO_4$ trocknete, filtrierte und

einengte. Die braunen Kristalle (1,7 g) wurden in 30 ml Ethanol gelöst. Man tropfte 1,3 ml (0,0075 Mol) 5,8N ethanolische HCl-Lösung zu und rührte 1 Stunde bei Raumtemperatur und 1 Stunde bei 0°. Die ausgeschiedenen Kristalle wurden abgenutscht und mit Essigester gewaschen. Man erhielt 1,65 g (85%) rac-cis-3-Cyclopentyl-2,3,3a,4,5,9b-hexahydro-1H-benzo[e]indol-6-ol-hydrochlorid als hellrosa Kristalle mit Smp. 256-258°.

d) 0,5 g (0,0017 Mol) rac-cis-3-Cyclopentyl-2,3,3a,4,5,9b-hexahydro-1H-benzo[e]indol-6-ol-hydrochlorid, gelöst in 30 ml Wasser, wurden mit 0,6 ml 2N Natronlauge neutralisiert und mit Ether extrahiert. Man wusch die organische Phase mit Wasser, trocknete sie über $Na_2SO_4$, filtrierte und engte das Filtrat ein. Der feste Rückstand wurde in 5 ml heissem THF gelöst, worauf mit 45 ml Toluol verdünnt wurde. Dann gab man nacheinander 0,18 g (0,25 ml, 0,0018 Mol) Triethylamin, 21 mg (170 µMol, 0,1 Aeq.) 4-Dimethylaminopyridin und schliesslich tropfenweise 0,16 ml (0,0018 Mol) Chlorameisensäuredimethylamid zu. Das Gemisch wurde 5 Stunden unter Rückfluss gekocht, dann abgekühlt, auf Wasser gegossen und mit Toluol ausgeschüttelt. Man wuscht die organische Phase mit Wasser, trocknete sie mit $Na_2SO_4$, filtrierte, engte das Filtrat ein und chromatographierte den Rückstand über 220 g Kieselgel mit Methanol/Essigester 9:1. Das erhaltene Oel (0,49 g) wurde in 10 ml Ethanol gelöst. Man tropfte 0,3 ml (0,0015 Mol) 4,93N ethanolische HCl-Lösung zu und engte ein. Das rac-cis-3-Cyclopentyl-2,3,3a,4,5,9b-hexahydro-1H-benzo[e]indol-6-yl-dimethylcarbamat-hydrochlorid kristallisierte aus Ethanol/Ether aus. Ausbeute: 0,47 g (84%), weisse Kristalle mit Smp. 215-217°.

Beispiel 50

a) Eine Suspension von 150 g (0,7 Mol) 1,3,4,5-Tetrahydro-6-methoxy-2H-benzo[e]indol-2-on in 1,5 l Methylenchlorid wurde mit 333 ml (2,1 Mol) Triethylsilan und dann bei 0° tropfenweise langsam und unter Rühren mit 240 ml Trifluoressigsäure versetzt. Nach dreistündigem Rühren wurden unter Kühlen langsam 400 ml 20%ige Natronlauge zugegeben, bis ein pH von 14 erreicht war. Anschliessend goss man das Gemisch in Wasser und extrahierte mit Methylenchlorid. Die organische Phase wurde mit Kochsalzlösung gewaschen, mit Natriumsulfat getrocknet, filtriert und eingedampft. Der Rückstand wurde mit 500 ml Ether versetzt, worauf der Festkörper abfiltriert und bei 50°/20 Torr getrocknet wurde. Man erhielt 123 g (81%) rac-cis-1,3,3a,4,5,9b-Hexahydro-6-methoxy-2H-benzo[e]indol-2-on mit Smp. 192-194°. Im Dünnschichtchromatogramm (Kieselgel, Essigester) zeigte das Material einen rf-Wert von 0,14.

Aus 1 g des Materials konnten durch Chromatographie an Kieselgel mit Essigester/Hexan 1:2 15 mg rac-trans-1,3,3a,4,5,9b-Hexahydro-6-methoxy-2H-benzo[e]indol-2-on mit Smp. 265-270° isoliert werden, welches im Dünnschichtchromatogramm (Kieselgel, Essigester) einen rf-Wert von 0,27 zeigte.

b) Aus 64,1 g des obigen Materials wurden analog wie in Beispiel 1c) beschrieben mittels 22,4 g Lithiumaluminiumhydrid 58,8 g (98%) eines grauen Oels erhalten, welches im Kühlschrank kristallisierte; Smp. des Hydrochlorids 226-231°.

c) Aus insgesamt 337 g des obigen Materials und insgesamt 394 g 2-Cyclohexylethylbromid wurden analog wie in Beispiel 1d) beschrieben in zwei Ansätzen insgesamt 626 g eines braunen, feste Partikel enthaltenden Oels erhalten, aus welchem durch Behandlung mit ethanolischem HCl insgesamt 413 g (71%) farblose Kristalle des entsprechenden Hydrochlorids erhalten wurden.

d) Aus 155 g des obigen Materials wurden analog wie in Beispiel 1e) beschrieben 144 g eines braunen Oels erhalten.

e) Eine Lösung von 234 g (-)-2,2'-(1,1-Binaphthyl)phosphorsäure in 2,35 l Ethanol und 1,55 l Chloroform wurde erwärmt und mit einer Lösung von 336 g des gemäss Beispiel 50d) erhaltenen Materials in 500 ml Ethanol versetzt. Dann wurden 2,4 l Lösungsmittel, hauptsächlich Chloroform, abdestilliert, worauf die Lösung abgekühlt wurde und Kristallisation einsetzte. Die Suspension wurde über das Wochenenden im Kühlschrank aufbewahrt und dann filtriert. Der Filterrückstand wurde mit Essigester und Ether gewaschen und bei 50°/20 Torr getrocknet, wobei 463 g farblose Kristalle erhalten wurden, deren Enantiomerenverhältnis (gemäss Gaschromatographie nach Extraktion einer Probe von 300 mg mit wässrigem Ammoniak/Ether) 63% des (+)- und 37% des (-)-Enantiomeren betrug.

Das erhaltene Material wurde unter Erwärmen in 3 l Ethanol und 4,2 l Chloroform gelöst. Dann wurden 1,3 l Chloroform abdestilliert, worauf beim Rühren über Nacht bei Raumtemperatur Kristallisation eintrat. Die Suspension wurde auf 0° abgekühlt und dann filtriert. Der Filterrückstand wurde mit Essigester und Ether gewaschen und bei 50°/20 Torr getrocknet. Man erhielt 253 g farblose Kristalle; Enantiomerenverhältnis 89:11. Die Mutterlauge wurde auf ein Volumen von etwa 2 l eingeengt, und man erhielt weitere 200 g farbloser Kristalle. Die beiden Kristallfraktionen wurden vereinigt, und es zeigte sich nachträglich, dass das Enantiomerenverhältnis dieser zweiten Kristallisation 48:52 betrug.

Eine dritte Kristallisation (2,5 l Ethanol und 3,5 l Chloroform) lieferte 288 g Kristalle; Enantiomerenverhältnis 89:11.

Eine vierte Kristallisation (2,3 l Ethanol und 3,5 l Chloroform) lieferte 239 g Kristalle; Enantiomerenverhältnis 97:3.

Eine fünfte Kristallisation (2 l Ethanol und 4 l Chloroform) lieferte 229 g Kristalle; Enantiomerenverhältnis 98,8:1,2.

Eine sechste Kristallisation (1,8 l Ethanol und 4,5 l Chloroform) lieferte 222 g Kristalle; Enantiomerenverhältnis 99,4:0,6.

Eine siebte Kristallisation (1,7 l Ethanol und 4,5 l Chloroform) lieferte 217 g Kristalle; Enantiomerenverhältnis 99,6:0,4.

Eine achte Kristallisation (1,7 l Ethanol und 4,5 l Chloroform) lieferte 212 g Kristalle; Enantiomerenverhältnis 99,85:9,15.

Das erhaltene Material wurde in etwa 5 l Essigester suspendiert und mit einer Mischung aus etwa 4 l Wasser und 4 l 25%iger Ammoniumhydroxidlösung extrahiert. Die organische Phase wurde sechsmal mit 2,5%iger Ammoniumhydroxidlösung extrahiert, und sämtliche wässerigen Phasen wurden dreimal mit Essigester extrahiert. Die vereinigten organischen Phasen wurden mit Natriumsulfat getrocknet und eingedampft, und man erhielt 86,4 g (51%) kristallines Material.

f) Eine Suspension von 70 g (0,23 Mol) des erhaltenen Materials in 2 l Toluol wurde mit 39 ml (0,28 Mol) Triethylamin und 34,3 g (0,28 Mol) 4-Dimethylaminopyridin versetzt. Zur entstandenen homogenen Mischung wurden 25,3 ml (0,28 Mol) Dimethylcarbamoylchlorid gegeben. Dann wurde während 4 Stunden am Rückfluss erhitzt, worauf man das Lösungsmittel abdampfte, den Rückstand in Essigester aufnahm und die Lösung zunächst zweimal mit Wasser und dann mit Kochsalzlösung extrahierte. Die wässerigen Phasen wurden dreimal mit Essigester extrahiert, worauf die organischen Phasen vereinigt, mit Natriumsulfat getrocknet, filtriert und eingedampft wurden. Das als Rückstand verbleibende Oel (103 g) wurde an 3 kg Kieselgel mit Essigester/n-Hexan 1:1 chromatographiert.

Dabei erhielt man als erstes Produkt 80,8 g (85%) eines farbloses Oels, welches in 200 ml Ethanol aufgenommen und unter Kühlung im Eisbad mit 30 ml 8,6M ethanolischer HCl versetzt wurde. Die leicht rote Lösung wurde eingedampft, und der Rückstand wurde erneut in 600 ml Ethanol aufgenommen, worauf die Lösung nach Behandlung mit 8 g Aktivkohle und Filtration eingedampft wurde. Der Rückstand wurde in 600 ml Essigester aufgenommen, worauf der Festkörper abfiltriert und bei 50°/20 Torr getrocknet wurde. Man erhielt 75 g (79%) (+)-cis-3-(2-Cyclohexylethyl)-2,3,3a,4,5,9b-hexahydro-1H-benzo[e]indol-6-yl-dimethylcarbamat-hydrochlorid in Form farbloser Kristalle mit Smp. 205-207°; $[\alpha]_D^{20}$ = +28,97° (c=0,7% in Methanol).

Als zweites Produkt erhielt man 5,5 g (+)-trans-3-(2-Cyclohexylethyl)-2,3,3a,4,5,9b-hexahydro-1H-benzo[e]indol-6-yl-dimethylcarbamat mit Smp. 110-112°; $[\alpha]_D^{20}$ = +7,6° (c=0,8% in Methanol).

Beispiel A

Erfindungsgemässe Verbindungen und/oder pharmazeutisch verwendbare Säureadditionssalze davon können wie nachstehend beschrieben als Wirkstoffe zur Herstellung galenischer Darreichungsformen verwendet werden.

a) Weichgelatinekapseln à 5 mg:

| Zusammensetzung: | Pro Kapsel |
|---|---|
| Wirkstoff | 5.0 mg |
| Gelbes Wachs | 8.0 mg |
| Hydriertes Sojabohnenöl | 8.0 mg |
| Partiell hydrierte Pflanzenöle | 34.0 mg |
| Sojabohnenöl | 110.0 mg |
| Gewicht Kapselinhalt | 165.0 mg |

Der Wirkstoff wird unter geeigneten Vorsichtsmassnahmen in einer warmen Schmelze der restlichen Stoffe gelöst. Die Lösung wird maschinell in Weichgelaltinekapseln geeigneter Grösse abgefüllt.

b) Hartgelatinekapseln à 20 mg:

| Zusammensetzung: | Pro Kapsel |
|---|---|
| Wirkstoff | 20.0 mg |
| Milchzucker krist. | 37.0 mg |
| Mikrokristalline Cellulose | 20.0 mg |
| Crospovidone | 8.5 mg |
| Modifizierte Stärke | 8.5 mg |
| Talk | 4.5 mg |
| Magnesiumstearat | 1.5 mg |
| Gewicht Kapselinhalt | 100.0 mg |

Die gesiebten und vermischten Bestandteile werden in Kapseln geeigneter Grösse abgefüllt.

c) Lacktabletten à 200 mg:

| Zusammensetzung: | Pro Tablette |
|---|---|
| Tablettenkern: | |
| Wirkstoff | 200.0 mg |
| Mikrokristalline Cellulose | 23.5 mg |
| Polyvinylpyrrolidon | 12.5 mg |
| Modifizierte Stärke | 12.5 mg |
| Magnesiumstearat | 1.5 mg |
| Kerngewicht | 250.0 mg |
| Lackschicht: | |
| Hydroxipropylmethylcellulose | 3.5 mg |
| Polyethylenglycol 6000 | 0.8 mg |
| Talk | 1.3 mg |
| Eisenoxid gelb | 0.8 mg |
| Titandioxid | 0.8 mg |
| Gewicht der Lackschicht | 7.4 mg |

Der gesiebte Wirkstoff wird mit mikrokristalliner Cellulose vermischt. Die Mischung wird mit einer Lösung von Polyvinylpyrrolidon in Wasser granuliert. Das Granulat wird mit modifizierter Stärke und Magnesiumstearat vermischt. Das Gemisch wird zu Kernen von 250 mg Gewicht verpresst. Die Kerne werden mit einer Lösung/Suspension der obenerwähnten Zusammensetzung lackiert.

d) Sachets à 50 mg:

| Zusammensetzung: | Pro Sachet |
|---|---|
| Wirkstoff | 50.0 mg |
| Laktose, feinpulverig | 1015.0 mg |
| Mikrokristalline Cellulose | 1400.0 mg |
| Natrium Carboxymethylcellulose | 14.0 mg |
| Polyvinylpyrrolidon | 10.0 mg |
| Magnesiumstearat | 10.0 mg |
| Aromastoffe | 1.0 mg |
| Füllgewicht eines Sachets | 2500.0 mg |

Der Wirkstoff wird mit Laktose, mikrokristalliner Cellulose und Natriumcarboxymethylcellulose vermischt. Die Mischung wird mit einer Lösung von Polyvinylpyrrolidon in Wasser granuliert. Das Granulat wird mit Magnesiumstearat und Aromastoffen vermischt. Die Mischung wird in Sachets geeigneter Grösse abgefüllt.

## Patentansprüche

1. cis-2,3,3a,4,5,9b-Hexahydro-1H-benzo[e]indol-Derivate der allgemeinen Formel

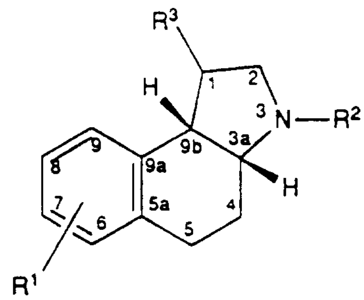

I

worin $R^1$ einen Rest der Formel $-O-CO-NR^4R^5$, $R^2$ niederes Alkyl, niederes Cycloalkyl oder durch niederes Cycloalkyl, Aryl, Aroyl, Aroylamino, Amino, Piperidin-1-yl, Morpholin-4-yl, 1-Oxoisoindolin-2-yl, 1,3-Dioxoisoindolin-2-yl, 1,3-Dioxo-octahydro-cis-isoindolin-2-yl oder 4,4-Dimethyl-2,6-dioxopiperidin-1-yl substituiertes niederes Alkyl, $R^3$ Wasserstoff oder niederes Alkyl, $R^4$ niederes Alkyl und $R^5$ Wasserstoff oder niederes Alkyl bedeuten, wobei die als "Aryl" und "Aroyl" bezeichneten Reste Phenyl- bzw. Benzoylreste sind, welche durch 1 bis 3 niedere Alkoxygruppen, niedere Alkylsulfonylgruppen oder Halogenatome substituiert sein können, und wobei die als "nieder" bezeichneten Reste höchstens 7 Kohlenstoffatome umfassen,
entsprechende trans-Isomere oder cis-trans-Isomerengemische und pharmazeutisch anwendbare Säureadditionssalze davon.

2. cis-2,3,3a,4,5,9b-Hexahydro-1H-benzo[e]indol-Derivate nach Anspruch 1, worin R2 niederes Alkyl oder durch niederes Cycloalkyl, Aryl, Aroyl, Aroylamino, Amino, Piperidin-1-yl, Morpholin-4-yl, 1-Oxoisoindolin-2-yl, 1,3-Dioxoisoindolin-2-yl, 1,3-Dioxo-octahydro-cis-isoindolin-2-yl oder 4,4-Dimethyl-2,6-dioxopiperidin-1-yl substituiertes niederes Alkyl bedeutet, und pharmazeutisch anwendbare Säureadditionssalze davon.

3. Verbindungen nach Anspruch 1 oder 2, worin $R^1$ in 6- oder 9-Stellung sitzt.

4. Verbindungen nach einem der Ansprüche 1 bis 3, worin $R^4$ Methyl, Ethyl, Propyl oder Butyl und $R^5$ Wasserstoff oder $R^4$ Methyl und $R^5$ Ethyl oder $R^4$ und $R^5$ beide Methyl bedeuten.

5. Verbindungen nach einem der Ansprüche 1 bis 4, worin $R^2$ n-Propyl, 3-Methylbutyl, Cyclopentyl, Cyclohexylmethyl, 2-Cyclohexylethyl, 3-Cyclohexylpropyl, 4-Cyclohexylbutyl, 2-Cyclopentylethyl, Benzyl, 2-Phenylethyl, 3-Phenylpropyl, 3-Benzoylpropyl, 4-Benzoylbutyl, 3-(4-Methoxybenzoyl)propyl, 4-(3,4-Dimethoxybenzoyl)butyl, 5-(3,4-Dimethoxybenzoyl)pentyl, 3-(4-Fluorbenzoyl)propyl, 4-Benzoylaminobutyl, 5-Benzoylaminopentyl, 6-Benzoylaminohexyl, 4-(4-Methansulfonylbenzoylamino)butyl, 5-(4-Methansulfonylbenzoylamino)pentyl, 6-(4-Methansulfonylbenzoylamino)hexyl, 4-Aminobutyl, 5-Aminopentyl, 6-Aminohexyl, 2-Morpholin-4-yl-ethyl, 3-Morpholin-4-ylpropyl, 2-Piperidin-1-ylethyl, 3-Piperidin-1-ylpropyl, 4-(1,3-Dioxoisoindolin-1-yl)butyl, 5-(1,3-Dioxoisoindolin-1-yl)pentyl, 6-(1,3-Dioxoisoindolin-1-yl)-hexyl, 5-(1,3-Dioxo-octahydro-cis-isoindolin-2-yl)pentyl, 4-(4,4-Dimethyl-2,6-dioxopiperidin-1-yl)butyl, 5-(4,4-Dimethyl-2,6-dioxopiperidin-1-yl)pentyl, 6-(4,4-Dimethyl-2,6-dioxopiperidin-1-yl)hexyl, 4-(1-Oxoisoindolin-2-yl)butyl, 5-(1-Oxoisoindolin-2-yl)pentyl oder 6-(1-Oxoisoindolin-2-yl)pentyl bedeuten.

6. Verbindungen nach einem der Ansprüche 1 bis 5, worin $R^3$ Wasserstoff oder Methyl bedeutet.

7. (+)-cis-3-(2-Cyclohexylethyl)-2,3,3a,4,5,9b-hexahydro-1H-benzo[e]indol-6-yl dimethylcarbamat.

8. (-)-cis-3-(2-Cyclohexylethyl)-2,3,3a,4,5,9b-hexahydro-1H-benzo[e]indol-6-yl dimethylcarbamat.

**9.** rac-cis-3-(2-Cyclohexylethyl)-2,3,3a,4,5,9b-hexahydro-1H-benzo[e]indol-6-yl dimethylcarbamat.

**10.** rac-cis-2,3,3a,4,5,9b-Hexahydro-3-(4-phthalimidobutyl)-1H-benzo[e]indol-6-yl dimethylcarbamat.

**11.** rac-cis-2,3,3a,4,5,9b-Hexahydro-3-(6-phthalimidohexyl)-1H-benzo[e]indol-6-yl dimethylcarbamat.

**12.** rac-cis-2,3,3a,4,5,9b-Hexahydro-3-(2-morpholinoethyl)-1H-benzo[e]indol-6-yl dimethylcarbamat.

**13.** rac-2,3,3aα,4,5,9bα-Hexahydro-1α-methyl-3-propyl-1H-benzo[e]indol-6-yl dimethylcarbamat.

**14.** rac-cis-2,3,3a,4,5,9b-Hexahydro-3-propyl-1H-benzo[e]indol-9-yl dimethylcarbamat,
(+)-3-(2-Cyclohexylethyl)-cis-2,3,3a,4,5,9b-hexahydro-1H-benzo[e]indol-6-yl-methylcarbamat,
(+)-3-(2-Cyclohexylethyl)-cis-2,3,3a,4,5,9b-hexahydro-1H-benzo[e]indol-6-yl-propylcarbamat,
(+)-3-(2-Cyclohexylethyl)-cis-2,3,3a,4,5,9b-hexahydro-1H-benzo[e]indol-6-6-yl-ethylmethylcarbamat,
rac-3-Cyclohexylmethyl-cis-2,3,3a,4,5,9b-hexahydro-1H-benzo[e]indol-6-yl-dimethylcarbamat,
rac-3-(3-Cyclohexylpropyl)-cis-2,3,3a,4,5,9b-hexahydro-1H-benzo[e]indol-6-yl-dimethylcarbamat,
rac-3-(4-Cyclohexylbutyl)-cis-2,3,3a,4,5,9b-hexahydro-1H-benzo[e]indol-6-yl-dimethylcarbamat und
rac-3-(2-Cyclopentylethyl)-cis-2,3,3a,4,5,9b-hexahydro-1H-benzo[e]indol-6-yl-dimethylcarbamat.

**15.** Verbindungen nach einem der Ansprüche 1 bis 14 zur Anwendung als therapeutische Wirkstoffe.

**16.** Verbindungen nach einem der Ansprüche 1 bis 14 zur Anwendung als therapeutische Wirkstoffe zur Behandlung oder Prophylaxe kognitiver Störungen und seniler Demenz (speziell Alzheimer-Krankheit) und zur Verbesserung der Gedächtnisleistung.

**17.** Verfahren zur Herstellung von Verbindungen nach einem der Ansprüche 1 bis 14 und von pharmazeutisch anwendbaren Säureadditionssalzen davon, dadurch gekennzeichnet, dass man

a) eine Verbindung der allgemeinen Formel

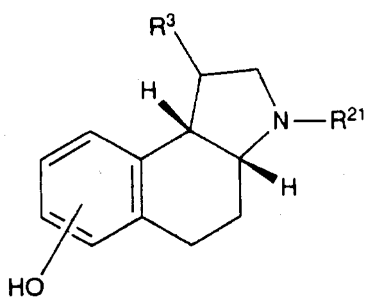

II

worin $R^3$ die in Anspruch 1 angegebene Bedeutung besitzt und $R^{21}$ im Prinzip die in Anspruch 1 für $R^2$ angegebene Bedeutung besitzt aber nicht durch Amino substituiertes niederes Alkyl bedeuten kann,
oder das entsprechende trans-Isomere oder cis-trans-Isomerengemisch mit einem einen Rest der Formel -CO-$NR^4R^5$ liefernden Mittel acyliert, wobei $R^4$ und $R^5$ die in Anspruch 1 angegebene Bedeutung besitzen,
oder

b) eine Verbindung der allgemeinen Formel

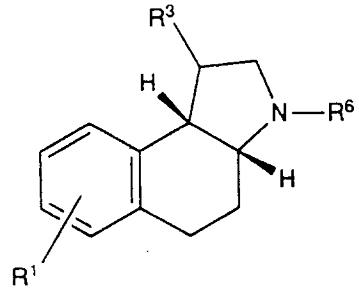

III

worin $R^1$ und $R^3$ die in Anspruch 1 angegebene Bedeutung besitzen und $R^6$ durch einen in eine Aminogruppe überführbaren Rest substituiertes niederes Alkyl bedeutet,
oder das entsprechende trans-Isomere oder cis-trans-Isomerengemisch in eine entsprechende Verbindung der Formel I oder das entsprechende trans-Isomere oder cis-trans-Isomerengemisch überführt, worin $R^2$ durch Amino substituiertes niederes Alkyl bedeutet; oder
c) in einer Verbindung der Formel I, worin $R^2$ durch Amino substituiertes niederes Alkyl bedeutet, oder in einem entsprechenden trans-Isomeren oder cis-trans-Isomerengemisch die Aminogruppe entsprechend acyliert;
d) erwünschtenfalls ein erhaltenes cis-trans-Isomerengemisch und/oder Diastereomerengemisch auftrennt; und/oder
e) erwünschtenfalls ein erhaltenes Racemat aufspaltet; und/oder
f) erwünschtenfalls eine erhaltene Verbindung der Formel I oder ein entsprechendes trans-Isomeres oder cis-trans-Isomerengemisch in ein pharmazeutisch anwendbares Säureadditionssalz überführt.

**18.** Arzneimittel, enthaltend eine Verbindung nach einem der Ansprüche 1 bis 14 und einen inerten Träger.

**19.** Arzneimittel nach Anspruch 18 zur Behandlung oder Prophylaxe kognitiver Störungen und seniler Demenz (speziell Alzheimer-Krankheit) und zur Verbesserung der Gedächtnisleistung.

**20.** Verwendung von Verbindungen nach einem der Ansprüche 1 bis 14 zur Herstellung von Arzneimitteln zur Behandlung oder Prophylaxe kognitiver Störungen und seniler Demenz (speziell Alzheimer-Krankheit) und zur Verbesserung der Gedächtnisleistung.

## Claims

**1.** cis-2,3,3a,4,5,9b-Hexahydro-1H-benzo[e]indole derivatives of the general formula

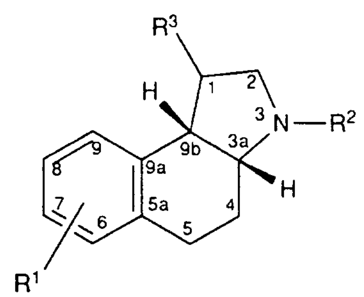

I

wherein $R^1$ signifies a residue of the formula -O-CO-$NR^4R^5$, $R^2$ signifies lower alkyl, lower cycloalkyl or lower alkyl substituted by lower cycloalkyl, aryl, aroyl, aroylamino, amino, piperidin-1-yl, morpholin-4-yl, 1-oxoisoindolin-2-yl, 1,3-dioxoisoindolin-2-yl, 1,3-dioxo-octahydro-cis-isoindolin-2-yl or 4,4-dimethyl-2,6-dioxopiperidin-1-yl, $R^3$ signifies hydrogen or lower alkyl, $R^4$ signifies lower alkyl and $R^5$ signifies hydrogen or lower alkyl, whereby the residues denoted as "aryl" and "aroyl" are phenyl and, respectively, benzoyl residues which can be substituted by 1 to 3 lower alkoxy groups, lower

alkylsulphonyl groups or halogen atoms and whereby the residues denoted as "lower" contain a maximum of 7 carbon atoms,
corresponding trans isomers or cis-trans isomer mixtures and pharmaceutically usable salts thereof.

**2.** cis-2,3,3a,4,5,9b-Hexahydro-1H-benzo[b]indole derivatives according to claim 1, wherein $R^2$ signifies lower alkyl or lower alkyl substituted by lower cycloalkyl, aryl, aroyl, aroylamino, amino, piperidin-1-yl, morpholin-4-yl, 1-oxoisoindolin-2-yl, 1,3-dioxoisoindolin-2-yl, 1,3-dioxo-octahydro-cis-isoindolin-2-yl or 4,4-dimethyl-2,6-dioxopiperidin-1-yl, and pharmaceutically usable acid addition salts thereof.

**3.** Compounds according to claim 1 or 2, wherein $R^1$ is present in the 6- or 9-position.

**4.** Compounds according to any one of claims 1 to 3, wherein $R^4$ signifies methyl, ethyl, propyl or butyl and $R^5$ signifies hydrogen or $R^4$ signifies methyl and $R^5$ signifies ethyl or $R^4$ and $R^5$ both signify methyl.

**5.** Compounds according to any one of claims 1 to 4, wherein $R^2$ signifies n-propyl, 3-methylbutyl, cyclopentyl, cyclohexylmethyl, 2-cyclohexylethyl, 3-cyclohexylpropyl, 4-cyclohexylbutyl, 2-cyclopentylethyl, benzyl, 2-phenylethyl, 3-phenylpropyl, 3-benzoylpropyl, 4-benzoylbutyl, 3-(4-methoxybenzoyl)-propyl, 4-(3,4-dimethoxybenzoyl)butyl, 5-(3,4-dimethoxybenzoyl)pentyl, 3-(4-fluorobenzoyl)propyl, 4-benzoylaminobutyl, 5-benzoylaminopentyl, 6-benzoylaminohexyl, 4-(4-methanesulphonylbenzoylamino)-butyl, 5-(4-methanesulphonylbenzoylamino)pentyl, 6-(4-methanesulphonylbenzoylamino)hexyl, 4-aminobutyl, 5-aminopentyl, 6-aminohexyl, 2-morpholin-4-yl-ethyl, 3-morpholin-4-ylpropyl, 2-piperidin-1-ylethyl, 3-piperidin-1-ylpropyl, 4-(1,3-dioxoisoindolin-1-yl)butyl, 5-(1,3-dioxoisoindolin-1-yl)pentyl, 6-(1,3-dioxoisoindolin-1-yl)hexyl, 5-(1,3-dioxo-octahydro-cis-isoindolin-2-yl)pentyl, 4-(4,4-dimethyl-2,6-dioxopiperidin-1-yl)butyl, 5-(4,4-dimethyl-2,6-dioxopiperidin-1-yl)pentyl, 6-(4,4-dimethyl-2,6-dioxopiperidin-1-yl)hexyl, 4-(1-oxoisoindolin-2-yl)butyl, 5-(1-oxoisoindolin-2-yl)pentyl or 6-(1-oxoisoindolin-2-yl)pentyl.

**6.** Compounds according to any one of claims 1 to 5, wherein $R^3$ signifies hydrogen or methyl.

**7.** (+)-cis-3-(2-Cyclohexylethyl)-2,3,3a,4,5,9b-hexahydro-1H-benzo[e]indol-6-yl dimethylcarbamate.

**8.** (-)-cis-3-(2-Cyclohexylethyl)-2,3,3a,4,5,9b-hexahydro-1H-benzo[e]indol-6-yl dimethylcarbamate.

**9.** rac-cis-3-(2-Cyclohexylethyl)-2,3,3a,4,5,9b-hexahydro-1H-benzo[e]indol-6-yl dimethylcarbamate.

**10.** rac-cis-2,3,3a,4,5,9b-Hexahydro-3-(4-phthalimidobutyl)-1H-benzo[e]indol-6-yl dimethylcarbamate.

**11.** rac-cis-2,3,3a,4,5,9b-Hexahydro-3-(6-phthalimidohexyl)-1H-benzo[e]indole-6-yl dimethylcarbamate

**12.** rac-cis-2,3,3a,4,5,9b-Hexahydro-3-(2-morpholinoethyl)-1H-benzo[e]indol-6-yl dimethylcarbamate.

**13.** rac-2,3,3aα,4,5,9bα-Hexahydro-1α-methyl-3-propyl-1H-benzo[e]indol-6-yl dimethylcarbamate.

**14.** rac-cis-2,3,3a,4,5,9b-Hexahydro-3-propyl-1H-benzo[e]indol-9-yl dimethylcarbamate,
(+)-3-(2-cyclohexylethyl)-cis-2,3,3a,4,5,9b-hexahydro-1H-benzo[e]indol-6-yl methylcarbamate,
(+)-3-(2-cyclohexylethyl)-cis-2,3,3a,4,5,9b-hexahydro-1H-benzo[e]indol-6-yl propylcarbamate,
(+)-3-(2-cyclohexylethyl)-cis-2,3,3a,4,5,9b-hexahydro-1H-benzo[e]indol-6-yl ethylmethylcarbamate,
rac-3-cyclohexylmethyl-cis-2,3,3a,4,5,9b-hexahydro-1H-benzo[e]indol-6-yl dimethylcarbamate,
rac-3-(3-cyclohexylpropyl)-cis-2,3,3a,4,5,9b-hexahydro-1H-benzo[e]indol-6-yl dimethylcarbamate,
rac-3-(4-cyclohexylbutyl)-cis-2,3,3a,4,5,9b-hexahydro-1H-benzo[e]indol-6-yl dimethylcarbamate and
rac-3-(2-cyclopentylethyl)-cis-2,3,3a,4,5,9b-hexahydro-1H-benzo[e]indol-6-yl dimethylcarbamate.

**15.** Compounds according to any one of claims 1 to 14 for use as therapeutically active substances.

**16.** Compounds according to any one of claims 1 to 14 for use as therapeutically active substances for the treatment or prophylaxis of cognitive disorders and senile dementia (especially Alzheimer's disease) and for the improvement of memory capacity.

**17.** A process for the manufacture of compounds according to any one of claims 1 to 14 and of pharmaceutically usable acid addition salts thereof, characterized by

a) acylating a compound of the general formula

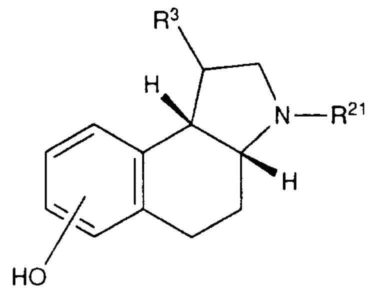

II

wherein $R^3$ has the significance given in claim 1 and $R^{21}$ has in principle the significance given in claim 1 for $R^2$, but can not signify lower alkyl substituted by amino,

or the corresponding trans isomer or cis-trans isomer mixture with an agent yielding a residue of the formula $-CO-NR^4R^5$, wherein $R^4$ and $R^5$ have the significance given in claim 1,

or

b) converting a compound of the general formula

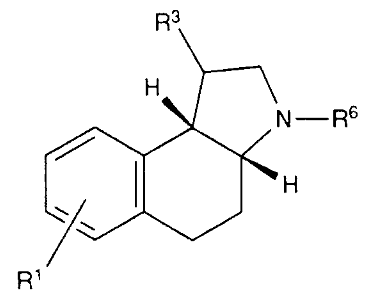

III

wherein $R^1$ and $R^3$ have the significance given in claim 1 and $R^6$ signifies lower alkyl substituted by a residue convertible into an amino group,

or the corresponding trans isomer or cis-trans isomer mixture into a corresponding compound of formula I or the corresponding trans isomer or cis-trans isomer mixture in which $R^2$ signifies lower alkyl substituted by amino; or

c) appropriately acylating the amino group in a compound of formula I in which $R^1$ signifies lower alkyl substituted by amino or in a corresponding trans isomer or cis-trans isomer mixture;

d) if desired, separating a cis-trans isomer mixture and/or a mixture of diastereomers obtained; and/or

e) if desired, resolving a racemate obtained; and/or

f) if desired, converting a compound of formula I or a corresponding trans isomer or cis-trans isomer mixture obtained into a pharmaceutically usable acid addition salt.

**18.** A medicament containing a compound according to any one of claims 1 to 14 and an inert carrier.

**19.** A medicament according to claim 18 for the treatment or prophylaxis of cognitive disorders and senile dementia (especially Alzheimer's disease) and for the improvement of memory capacity.

**20.** The use of compounds according to any one claims 1 to 14 for the manufacture of medicaments for the treatment or prophylaxis of cognitive disorders and senile dementia (especially Alzheimer's disease) and for the improvement of memory capacity.

## Revendications

1. Dérivés de *cis*-2,3,3a,4,5,9b-hexahydro-1H-benzo[e]indole de formule générale

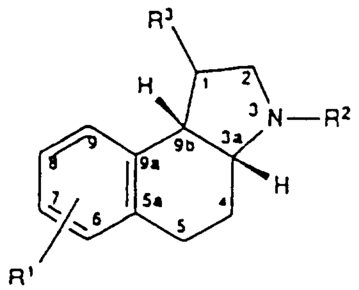

I

dans laquelle :

- $R^1$ représente un radical de formule -O-CO-$NR^4R^5$,
- $R^2$ représente un radical alkyle inférieur, cycloalkyle inférieur ou un radical alkyle inférieur substitué par un groupe cycloalkyle inférieur, aryle, aroyle, aroylamino, amino, pipéridine-1-yle, morpholine-4-yle, 1-oxoisoindoline-2-yle, 1,3-dioxoisoindoline-2-yle, 1,3-dioxooctahydro-cis-isoindoline-2-yle ou 4,4-diméthyl-2,6-dioxopipéridine-1-yle,
- $R^3$ représente un atome d'hydrogène ou un radical alkyle inférieur,
- $R^4$ représente un radical alkyle inférieur et
- $R^5$ représente un atome d'hydrogène ou un radical alkyle inférieur,
- les radicaux désignés par "aryle" et "aroyle" étant des radicaux phényle ou benzoyle, respectivement qui peuvent être substitués par 1 à 3 groupes alcoxy inférieurs, groupes alkylsulfonyle inférieurs ou atomes d'halogène, et
- les radicaux désignés comme "inférieurs" comprenant au maximum 7 atomes de carbone, isomères trans correspondants ou mélanges d'isomères *cis* et *trans,* et sels d'addition d'acides pharmaceutiquement acceptables de ces dérivés.

2. Dérivés de *cis*-2,3,3a,4,5,9b-hexahydro-1H-benzo[e]indole selon la revendication 1, dans lesquels $R^2$ représente un radical alkyle inférieur ou un radical alkyle inférieur substitué par un groupe cycloalkyle inférieur, aryle, aroyle, aroylamino, amino, pipéridine-1-yle, morpholine-4-yle, 1-oxo-isoindoline-2-yle, 1,3-dioxoisoindoline-2-yle, 1,3-dioxo-octahydro-cis-isoindoline-2-yle ou 4,4-diméthyl-2,6-dioxopipéridine-1-yle, et sels d'addition d'acides pharmaceutiquement acceptables de ces dérivés.

3. Composés selon la revendication 1 ou 2, dans lesquels $R^1$ est en position 6 ou 9.

4. Composés selon l'une des revendications 1 à 3, dans lesquels $R^4$ représente le groupe méthyle, éthyle, propyle ou butyle et $R^5$ représente l'hydrogène, ou $R^4$ représente le groupe méthyle et $R^5$ représente le groupe éthyle, ou $R^4$ et $R^5$ représentent chacun le groupe méthyle.

5. Composés selon l'une des revendications 1 à 4, dans lesquels $R^2$ représente le groupe n-propyle, 3-méthylbutyle, cyclopentyle, cyclohexylméthyle, 2-cyclohexyléthyle, 3-cyclohexylpropyle, 4-cyclohexylbutyle, 2-cyclopentyléthyle, benzyle, 2-phényléthyle, 3-phénylpropyle, 3-benzoylpropyle, 4-benzoylbutyle, 3-(4-méthoxybenzoyl)-propyle, 4-(3,4-diméthoxybenzoyl)-butyle, 5-(3,4-diméthoxybenzoyl)-pentyle, 3-(4-fluorobenzoyl)-propyle, 4-benzoylaminobutyle, 5-benzoylaminopentyle, 6-benzoylaminohexyle, 4-(4-méthanesulfonylbenzoylamino)-butyle, 5-(4-méthanesulfonylbenzoylamino)-pentyle, 6-(4-méthanesulfonylbenzoylamino)-hexyle, 4-aminobutyle, 5-aminopentyle, 6-aminohexyle, 2-morpholine-4-yléthyle, 3-morpholine-4-ylpropyle, 2-pipéridine-1-yléthyle, 3-pipéridine-1-ylpropyle, 4-(1,3-dioxo-isoindoline-1-yl)-butyle, 5-(1,3-dioxo-isoindoline-1-yl)-pentyle, 6-(1,3-dioxoisoindoline-1-yl)-hexyle, 5-(1,3-dioxo-octahydro-cis-isoindoline-2-yl)-pentyle, 4-(4,4-diméthyl-2,6-dioxopipéridine-1-yl)-butyle, 5-(4,4-diméthyl-2,6-dioxo-pipéridine-1-yl)-pentyle, 6-(4,4-diméthyl-2,6-dioxopipéridine-1-yl)-hexyle 4-(1-oxoisoindoline-2-yl)-butyle, 5-(1-oxo-isoindoline-2-yl)-pentyle ou 6-(1-oxo-isoindoline-2-yl)-pentyle.

6. Composés selon l'une des revendications 1 à 5, dans lesquels $R^3$ représente l'hydrogène ou le groupe méthyle.

**7.** Diméthylcarbamate de (+)-*cis*-3(2-cyclohexyléthyl)-2,3,3a,4,5,9b-hexahydro-1H-benzo[e]indol-6-yle.

**8.** Diméthylcarbamate de (-)-*cis*-3-(2-cyclohexyléthyl)-2,3,3a,4,5,9b-hexahydro-1H-benzo[e]indol-6-yle.

**9.** Diméthylcarbamate de rac-*cis*-3(2-cyclohexyléthyl)-2,3,3a,4,5,9b-hexahydro-1H-benzo[e]indol-6-yle.

**10.** Diméthylcarbamate de rac-*cis*-2,3,3a,4,5,9b-hexahydro-3-(4-phtalimidobutyl)-1H-benzo[e]-indol-6-yle.

**11.** Diméthylcarbamate de rac-*cis*-2,3,3a,4,5,9b-hexahydro-3-(6-phtalimidohexyl)-1H-benzo[e]indol-6-yle.

**12.** Diméthylcarbamate de rac-*cis*-2,3,3a,4,5,9b-hexahydro-3-(2-morpholinoéthyl)-1H-benzo[e]indol-6-yle.

**13.** Diméthylcarbamate de rac-2,3,3aα,4,5,9bα-hexahydro-1α-méthyl-3-propyl-1H-benzo[e]indol-6-yle.

**14.** Diméthylcarbamate de rac-*cis*-2,3,3a,4,5,9b-hexahydro-3-propyl-1H-benzo[e] indol-9-yle, méthylcarbamate de (+)-3-(2-cyclohexyléthyl)-*cis*-2,3,3a,4,5,9b-hexahydro-1H-benzo[e]indol-6-yle, propylcarbamate de (+)-3-(2-cyclohexyléthyl)-*cis*-2,3,3a, 4,5,9b-hexahydro-1H-benzo[e]indol-6-yle, éthylméthylcarbamate de (+)-3-(2-cyclohexyléthyl)-*cis*-2,3,3a,4,5,9b-hexahydro-1H-benzo[e]indol-6-yle, diméthylcarbamate de rac-3-cyclohexylméthyl-*cis*-2,3,3a,4,5,9b-hexahydro-1H-benzo[e]indol-6-yle, diméthylcarbamate de rac-3-(3-cyclohexylpropyl)-*cis*-2,3,3a,4,5,9b-hexahydro-1H-benzo[e]indol-6-yle, diméthylcarbamate de rac-3-(4-cyclo-hexylbutyl)-*cis*-2,3, 3a,4,5,9b-hexahydro-1H-benzo[e]indol-6-yle et diméthyl-carbamate de rac-3-(2-cyclopentyléthyl)-*cis*-2,3,3a,4,5,9b-hexahydro-1H-benzo[e] indol-6-yle.

**15.** Composés selon l'une des revendications 1 à 14, pour utilisation en tant que substances actives thérapeutiques.

**16.** Composés selon l'une des revendications 1 à 14, pour utilisation en tant que substances actives thérapeutiques pour le traitement ou la prophylaxie de troubles cognitifs et de la démence sénile (en particulier la maladie d'Alzheimer) et pour l'amélioration de la mémoire.

**17.** Procédé pour la préparation de composés selon l'une des revendications 1 à 14 et de leurs sels d'addition avec des acides pharmaceutiquement acceptables, caractérisé en ce que
a) on soumet un composé de formule générale

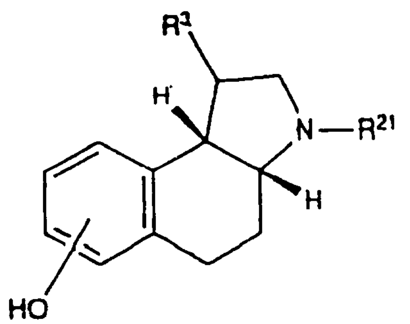

II

dans laquelle $R^3$ a la signification donnée dans la revendication 1 et $R^{21}$ a en principe la signification donnée pour $R^2$ dans la revendication 1, mais ne peut pas représenter un radical alkyle inférieur substitué par le groupe amino,
ou l'isomère *trans* correspondant ou bien un mélange d'isomères *cis* et *trans,* avec un agent fournissant un radical de formule -CO-$NR^4R^5$, $R^4$ et $R^5$ ayant les significations données dans la revendication 1,
ou

b) on convertit un composé de formule générale

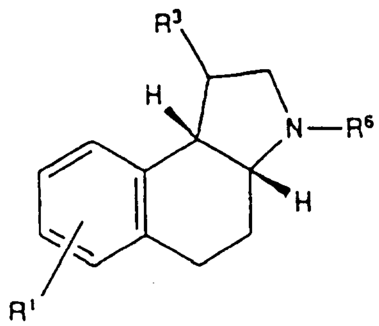

III

dans laquelle $R^1$ et $R^3$ ont les significations données dans la revendication 1, et $R^6$ représente un radical alkyle inférieur substitué par un groupe pouvant être converti en un groupe amino, ou l'isomère *trans* correspondant ou bien un mélange d'isomères *cis* et *trans,* en un composé correspondant de formule I ou l'isomère *trans* correspondant ou bien un mélange des isomères *cis* et *trans,* dans lequel $R^2$ représente un radical alkyle inférieur substitué par le groupe amino ;
ou
c) dans un composé de formule I dans lequel $R^2$ représente un radical alkyle inférieur substitué par le groupe amino, ou dans un isomère *trans* correspondant ou mélange d'isomères *cis* et *trans,* on acyle convenablement le groupe amino ;
d) si on le désire on sépare un mélange d'isomères *cis* et *trans* et/ou un mélange de diastéréoisomères; et/ou
e) si on le désire on dédouble un racémique obtenu ; et/ou
f) si on le désire on convertit un composé de formule I ou un isomère *trans* correspondant ou mélange d'isomères *cis* et *trans* obtenu en un sel d'addition d'acide pharmaceutiquement acceptable.

**18.** Médicament contenant un composé selon l'une des revendications 1 à 14 et un support inerte.

**19.** Médicament selon la revendication 18, pour le traitement ou la prophylaxie de troubles cognitifs et de la démence sénile (en particulier la maladie d'Alzheimer) et pour l'amélioration de la mémoire.

**20.** Utilisation de composés selon l'une des revendications 1 à 14, pour la fabrication de médicaments destinés au traitement ou à la prophylaxie de troubles cognitifs et de la démence sénile (en particulier la maladie d'Alzheimer) et à l'amélioration de la mémoire.